# EUROPEAN PATENT APPLICATION

(11) **EP 4 265 609 A1**
(43) Date of publication of application: **25.10.2023**
(21) Application number: 21905628.0
(22) Date of filing: 10.12.2021
(51) Int. Cl.: C07D 403/10, C07D 403/12, A61K 31/53, A61P 5/16

(54) **THYROID HORMONE RECEPTOR BETA SELECTIVE AGONIST COMPOUND, PHARMACEUTICAL COMPOSITION AND USE THEREOF**

(30) Priority: 15.12.2020 CN 202011478971; 07.12.2021 CN 202111482622
(71) Applicant: Shanghai Institute of Materia Medica, Chinese Academy of Sciences, Pudong, Shanghai 201203 (CN)
(72) Inventor: ZHOU, Bing, Shanghai 201203 (CN); LENG, Ying, Shanghai 201203 (CN); YANG, Yaxi, Shanghai 201203 (CN); NING, Mengmeng, Shanghai 201203 (CN); HU, Liuyu, Shanghai 201203 (CN); FENG, Lei, Shanghai 201203 (CN); LIANG, Ju, Shanghai 201203 (CN)
(74) Representative: Grünecker Patent- und Rechtsanwälte PartG mbB
(86) International application number: PCT/CN2021/136993
(87) International publication number: WO 2022/127699

(57) **Abstract**

The present invention relates to a thyroid hormone receptor β selective agonist compound represented by formula I, a pharmaceutical composition and use thereof. The compound improves the selectivity to THR-α and the druggability of the compound while maintaining good THR-β agonistic activity, and shows a certain activity in *in vivo* pharmacological experiments.

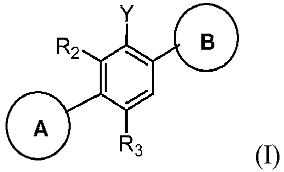

## Description

### Technical field

The present invention relates to thyroid hormone receptor β selective agonists. More specifically, the present invention relates to a compound of formula (I) as a thyroid hormone receptor β subtype agonist, a pharmaceutical composition thereof, and use thereof in preparation of medicines for treating related diseases.

### Background Art

Thyroid hormone (TH) is produced by the thyroid gland and secreted into the circulatory system (hypothalamus/pituitary/thyroid system) in two different forms: 3,5,3',5'-tetraiodo-L-thyronine (T4) and 3,5,3'-triiodo-L-thyronine (T3). Although T4 is the predominant form secreted by the thyroid, T3 is the more physiologically active form. T4 is converted to T3 by tissue-specific deiodinases, which are present in all tissues but mainly in the liver and kidney. The biological activity of thyroid hormones is mediated by thyroid hormone receptors (THRs). THRs are encoded by different gene expressions a and β located on human chromosomes 17 and 3, respectively, and different protein isoforms are genenrated through selective splicing of the primary transcripts, with each gene producing two isoforms, namely THRα1, THRα2, THRβ1, THRβ2. THRβ1 and THRβ2 result from differential expression in promoters, and the two isoforms differ only at the amino terminus. THRα1 and THRα2 result from differential splicing of pre-mRNA, differing mainly at the carboxyl terminus. Among them, THRα1, THRβ1 and THRβ2 can bind thyroid hormone. It has been shown that the thyroid hormone receptor isoforms can differ in their contribution to specific physiological responses. THRβ1 plays an important role in regulating the actions of thyrotropin and thyroid hormones in liver. THRβ2 plays a major role in the regulation of thyroid stimulating hormone. Thyroid hormones have the effect of lowering serum low-density lipoprotein (LDL). Hyperthyroidism is associated with low total serum cholesterol, because thyroid hormones promote hepatic LDL receptor expression and stimulate the metabolism of cholesterol to bile acids. Hypothyroidism is associated with hypercholesterolemia, and the thyroid hormone replacement therapy is known to lower total cholesterol. Thyroid hormones also reduce the risk of atherosclerosis and other cardiovascular diseases. The incidence of atherosclerotic vascular disease is directly related to the LDL cholesterol level. Thyroid hormones have beneficial effects in obese patients by reducing body weight and improving obesity-related co-morbidities by increasing metabolic rate, oxygen consumption and heat release, and may also have beneficial effects on glycemic control in obese patients with type 2 diabete.

The development of thyroid analogs that avoid the adverse effects of hyperthyroidism and hypothyroidism while maintaining the beneficial effects of thyroid hormones will open new avenues for the treatment of patients with metabolic diseases such as obesity, hyperlipidemia, hypercholesterolemia, diabetes; and other diseases and conditions such as hepatic steatosis and nonalcoholic steatohepatitis (NASH), atherosclerosis, cardiovascular diseases, hypothyroidism, thyroid cancer, thyroid diseases, and related conditions and diseases.

MGL-3196 is the first-in-class orally administered small molecule selective agonist of the hepatic thyroid hormone receptor beta subtype (THR-β). Preclinical toxicology and Phase 1 data indicated that, as a potential treatment for nonalcoholic steatohepatitis (NASH) and dyslipidemia, MGL-3196 could significantly reduce LDL cholesterol, triglycerides, and lipoproteins (Journal of Hepatology, 2018, vol. 68, S37-S64), making it an ideal drug candidate to lower cardiovascular risk in NASH patients anddyslipidemia patients on moderate statin doses or intolerant to statins (European Heart Journal, Volume 39, Issue suppl_l, August 2018, ehy566.P5387). Phase 2 clinical data showed that adverse events (AEs) were generally mild (85%) and moderate (15%), and 3 severe AEs occurred and were considered unrelated to the treatment (Journal of Hepatology, 2018, vol.68, S37-S64).

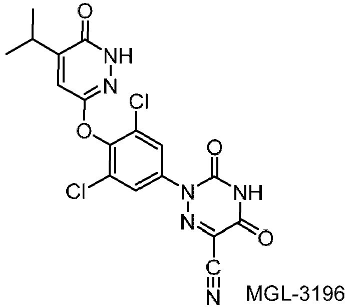

Although MGL-3196 as a THR-β agonist can effectively treat a variety of diseases, it is still a challenging work to find new compounds that have the beneficial effects of thyroid hormones without adverse effects and have good oral bioavailability and druggability. Therefore, there is still a need in the art to develop THR-β selective agonists with better specificity/ pharmacodynamic/ pharmacokinetic properties, and the present invention provides such compounds.

### Summary of the Invention

It is an object of the present invention to provide a compound of formula (I), its pharmaceutically acceptable salts, stereoisomers, enantiomers, diastereomers, atropisomers, racemates, polymorphs, solvates or isotopically labeled compounds (including deuterium substituted compounds).

It is another object of the present invention to provide a method for preparing the compound.

It is another object of the present invention to provide a pharmaceutical composition comprising the compound.

It is another object of the present invention to provide use of the compound in preparation of a medicine.

According to one aspect of the present invention, provided is a compound of formula (I), or a pharmaceutically acceptable salt, stereoisomer, enantiomer, diastereomer, atropisomer, racemate, polymorph, solvate or isotopically labeled compound thereof: 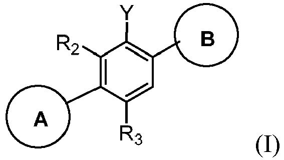 wherein
ring A is selected from the group consisting of 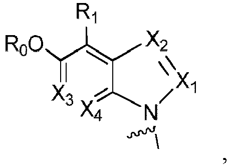 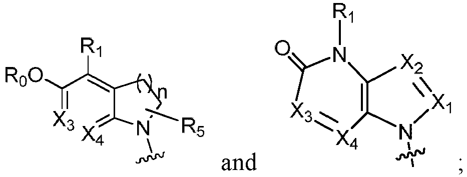
ring B is selected from the group consisting of 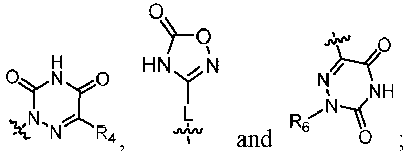
R₀ is selected from the group consisting of hydrogen and C₁₋₁₀ alkyl;
R₁ is selected from the group consisting of hydrogen, substituted or unsubstituted C₁₋₁₀ alkyl, substituted or unsubstituted C₃₋₁₀ cycloalkyl, substituted or unsubstituted 3-10 membered heterocycloalkyl, substituted or unsubstituted C₆₋₁₀ aryl and substituted or unsubstituted 5-10 membered heteroaryl, wherein the substituents for the "substituted" are selected from the group consisting of halogen, hydroxyl, =O, C₁₋₆ alkoxy, C₁₋₆ alkyl, C₃₋₁₀ cycloalkyl, C₆₋₁₀ aryl, halogenated C₆₋₁₀ aryl, C₁₋₁₀ alkyl C₆₋₁₀ aryl, C₁₋₁₀ alkoxy C₆₋₁₀ aryl, 5-10 membered heteroaryl, C₁₋₁₀ alkyl 5-10 membered heteroaryl, halogenated 5-10 membered heteroaryl, 3-10 membered heterocycloalkyl and -NR¹⁰R¹¹;
R₂, R₃ and Y are each independently selected from the group consisting of hydrogen, halogen, substituted or unsubstituted C₁₋₆ alkyl, substituted or unsubstituted C₃₋₆ cycloalkyl, and substituted or unsubstituted C₁₋₆ alkoxy, wherein the substituents for the "substituted" are selected from the group consisting of halogen, hydroxyl, C₁₋₆ alkyl and C₁₋₆ alkoxy;
R₄ is selected from the group consisting of hydrogen, cyano, -NR¹⁰R¹¹, substituted or unsubstituted C₁₋₆ alkyl, substituted or unsubstituted C₃₋₆ cycloalkyl and substituted or unsubstituted C₂₋₈ alkynyl, wherein the substituents for the "substituted" are selected from the group consisting of halogen, hydroxyl, cyano and C₁₋₆ alkoxy;
R₅ is selected from the group consisting of hydrogen, substituted or unsubstituted C₁₋₆ alkyl and substituted or unsubstituted C₃₋₆ cycloalkyl, wherein the substituents for the "substituted" are selected from the group consisting of halogen, hydroxyl and C₁₋₆ alkoxy;
R₆ is selected from the group consisting of hydrogen and substituted or unsubstituted C₁₋₆ alkyl, wherein the substituents for the "substituted" are selected from the group consisting of halogen, hydroxyl and C₁₋₆ alkoxy;
L is absent, or -NR¹⁰C(O)- or -NR¹⁰CR¹¹R¹¹-;
each R¹⁰ is independently selected from the group consisting of hydrogen and substituted or unsubstituted C₁₋₃ alkyl, wherein the substituents for the "substituted" are selected from the group consisting of halogen, hydroxyl and C₁₋₃ alkoxy;
each R¹¹ is independently selected from the group consisting of hydrogen and substituted or unsubstituted C₁₋₆ alkyl, wherein the substituents for the "substituted" are selected from the group consisting of halogen, hydroxyl and C₁₋₆ alkoxy;
X₁ and X₂ are each independently selected from the group consisting of N and CR¹², wherein R¹² is selected from the group consisting of hydrogen, halogen, cyano, -NR^{b}R^{c}, -C(=O)R^{a}, -C(=O)OR^{b}, -C(=O)NR^{b}R^{c}, substituted or unsubstituted C₁₋₄ alkyl, substituted or unsubstituted C₃₋₆ cycloalkyl, substituted or unsubstituted C₆₋₁₀ aryl and substituted or unsubstituted 5-10 membered heteroaryl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, halogen, cyano, hydroxyl, -OR^{a}, -NR^{b}R^{c}, -C(=O)R^{a}, -C(=O)OR^{b}, -C(=O)NR^{b}R^{c}, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
each R^{a} is independently C₁₋₆ alkyl, C₃₋₁₀ cycloalkyl or 3-10 membered heterocycloalkyl, wherein the alkyl, cycloalkyl and heterocycloalkyl are independently and optionally substituted by one or more substituents selected from the group consisting of halogen, hydroxyl, amino and C₁₋₆ alkyl;
each R^{b} and R^{c} is independently hydrogen, C₁₋₆ alkyl, C₃₋₁₀ cycloalkyl or 3-10 membered heterocycloalkyl, wherein the alkyl, cycloalkyl and heterocycloalkyl are independently and optionally substituted by one or more substituents selected from the group consisting of halogen, hydroxyl, amino and C₁₋₆ alkyl;
or R^{b} and R^{c}, together with the nitrogen atom to which they are connected, form a 3-10 membered heterocycloalkyl, wherein the heterocycloalkyl is optionally substituted by one or more substituents selected from the group consisting of halogen, hydroxyl, amino and C₁₋₆ alkyl;
n is independently 1, 2 or 3 at each occurrence.

According to another aspect of the invention, provided is a method for preparing the compound of the present invention, wherein the method is one of the following schemes:

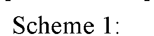

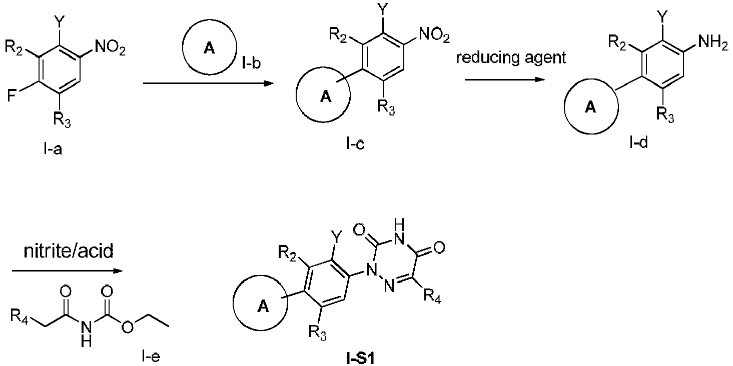

The compound of formula **I-a** is dissolved in a polar solvent, added with a base to react with the compound of formula **I-b** to obtain the compound of formula **I-c.** The base under the condition includes inorganic bases (sodium carbonate, potassium carbonate, cesium carbonate, lithium hydroxide, sodium hydroxide or potassium hydroxide, etc.) and organic bases (triethylamine, N,N-diisopropylethylamine or pyridine, etc.), and is preferably potassium carbonate. The polar solvent under the condition includes N,N-dimethylacetamide, N,N-dimethylformamide, dimethyl sulfoxide or acetonitrile, etc., and is preferably N,N-dimethylformamide.

The compound of formula **I-c** is reacted with a reducing agent to obtain the compound of formula **I-d**. The reducing agent under the condition includes iron powder, sulfide, stannous chloride or zinc powder etc., and is preferably stannous chloride.

The compound of formula **I-d** is reacted with a nitrite in an acidic acetonitrile solution, added with the compound of formula **I-e** to react further and cyclize at a high temperature to obtain the compound of formula **I-S1.** The acid under the condition is an organic acid, including carboxyl or sulfonic acid, etc., and is preferably acetic acid. The nitrite under the condition includes isoamyl nitrite or tert-butyl nitrite, etc., and is preferably tert-butyl nitrite, and ring A, Y, R², R³ and R⁴ are as defined herein. Or

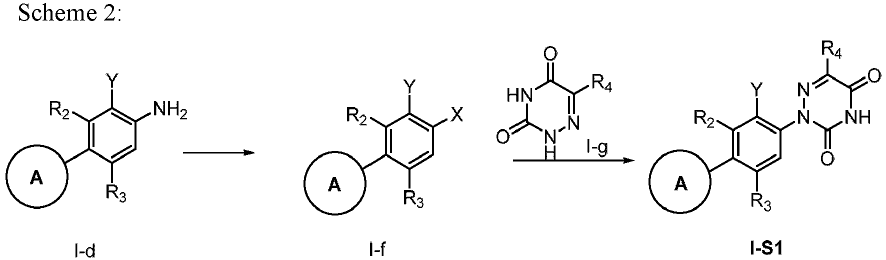

The compound of formula **I-d** is reacted with a nitrite under an acidic condition to generate a diazonium salt, and added with a halide anion to obtain the compound of formula **I-f;** the obtained compound of formula **I-f** is coupled with the intermediate of formula **I-g** under the catalysis of a transition metal compound to obtain the compound of formula **I-S1.** The acid under the condition is an organic acid, including carboxyl or sulfonic acid, etc., and preferably acetic acid. The nitrite under the condition includes isoamyl nitrite or tert-butyl nitrite, etc., and is preferably tert-butyl nitrite, X is a halogen, and ring A, Y, R², R³ and R⁴ are as defined herein. Or

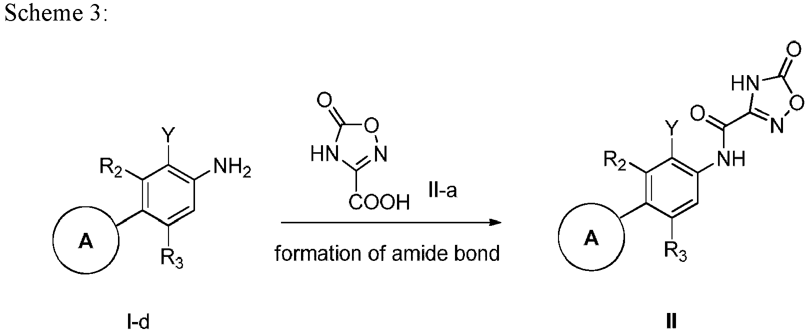

The compound of formula **II-a** is added with a condensing agent under a basic condition, and subjected to a condensation reaction with the compound of formula **I-d** to form an amide bond to obtain the compound of formula **II.** The base under the condition includes organic bases such as triethylamine or N,N-diisopropylethylamine, and the condensing agent under the condition includes carbodiimide-type, phosphonium-type or urea-cation-type condensing agents, etc. Ring A, Y, R² and R³ are as defined herein. or

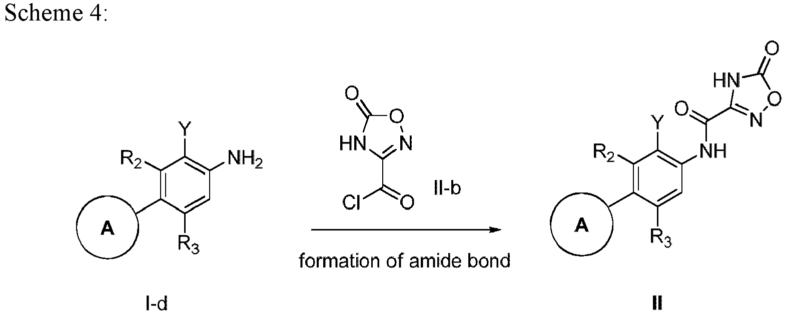

The compound of formula **I-d** is reacted with the acyl chloride of formula **II-b** under a basic condition to form an amide bond to obtain the compound of formula **II.** The base under the condition includes organic bases such as triethylamine or N,N-diisopropylethylamine, and is preferably triethylamine. Ring A, Y, R² and R³ are as defined herein. or

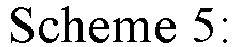

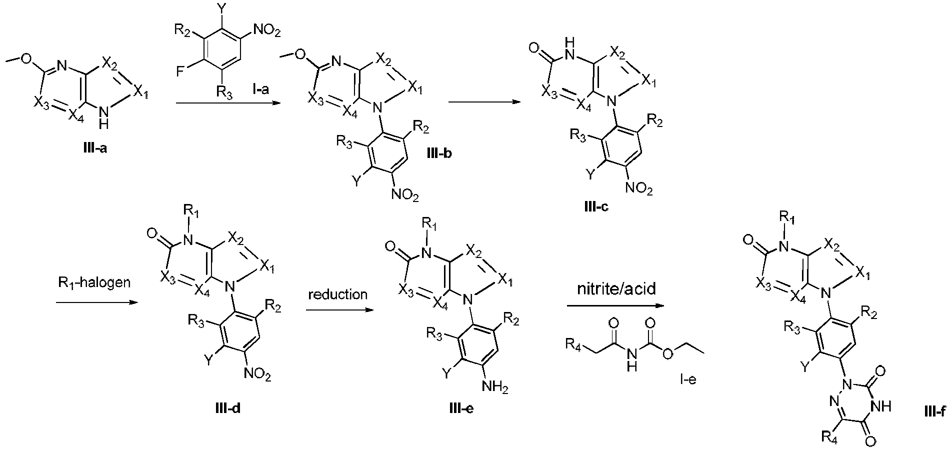

The compound of formula **III-a** is subjected to a substitution reaction with the compound of formula **I-a** under a basic condition to obtain the compound of formula **III-b,** which is then deprotected to remove the methyl ether on the oxygen according to conventional conditions, and subjected further to a substitution reaction with a halogenated hydrocarbon substituted by a R₁ group under a basic condition to obtain the intermediate of formula **III-d.** The base under the condition includes inorganic bases such as potassium carbonate, cesium carbonate, and sodium hydroxide, and organic bases such as triethylamine or N,N-diisopropylethylamine, the reaction temperature is 50-150°C, and the reaction can be carried out under heating or microwave conditions. The organic solvent includes but not limited to dioxane, DMF, DMSO, tetrahydrofuran, NMP, etc. R₁, R₂, R₃, X₁, X₂, X₃ and X₄ are as defined herein.

Then, according to the method of Scheme 1, **III-d** is reduced to obtain the key intermediate of formula **III-e,** which is then reacted with a nitrite, and further reacted with the compound of formula **I-e** and cyclized to obtain the compound of formula **III-f.**

According to another aspect of the present invention, provided is a pharmaceutical composition comprising a therapeutically effective amount of one or more selected from the group consisting of the above compound and the pharmaceutically acceptable salt, stereoisomer, enantiomer, diastereoisomer, atropisomer, racemate, polymorph, solvate and isotopically labeled compound (including deuterium substituted compound) thereof, and optionally a pharmaceutically acceptable excipient.

According to another aspect of the present invention, provided is use of the compound, or the pharmaceutically acceptable salt, stereoisomer, enantiomer, diastereoisomer, atropisomer, racemate, polymorph, solvate or isotopically labeled compound (including deuterium substituted compound) thereof, or the composition in preparation of a medicine for treatment of a metabolic-related disease.

According to another aspect of the present invention, provided is a method for treating a metabolic-related disease, comprising administering to a subject an effective amount of one or more selected from the group consisting of the above compound and the pharmaceutically acceptable salt, stereoisomer, enantiomer, diastereomer, atropisomer, racemate, polymorph, solvate and isotopically labeled compound (including deuterium substituted compound) thereof, or a pharmaceutical composition comprising one or more selected from the group consisting of the above compounds and the pharmaceutically acceptable salt, stereoisomer, enantiomer, diastereomer, atropisomer, racemate, polymorph, solvate and isotopically labeled compound (including deuterium substituted compound) thereof as an active ingredient.

### Beneficial effect

The present disclosure effectively develops a THR-β selective agonist with better specificity and pharmacodynamics and pharmacokinetic properties, which shows the potential to treat various diseases.

### Detailed Embodiments

In order to enable one skilled in the art to understand the characteristics and effects of the present invention, the terms and words mentioned in the description and claims are generally described and defined below. Unless otherwise specified, all technical and scientific terms used herein have the usual meanings understood by those skilled in the art for the present invention. In case of conflict, the definitions in this specification shall prevail.

As used herein, the terms "comprises", "includes", "has", "contains" or any other similar terms are open-ended transitional phrases intended to cover non-exclusive inclusions. For example, a composition or article containing a plurality of elements is not limited to only those elements listed herein, but may also include other elements not specifically listed but which are generally inherent in the composition or article. Otherwise, unless expressly stated to the contrary, the word "or" means an inclusive "or" and not an exclusive "or". For example, the condition "A or B" is satisfied by any of the following: A is true (or exists) and B is false (or does not exist), A is false (or does not exist) and B is true (or exists), both A and B are true (or exist). In addition, the interpretation of the terms "comprises", "includes", "has", "contains" herein should be deemed to have been specifically disclosed and also cover closed or semi-closed transitional phrases such as "consists of" and "essentially consists of".

Herein, all features or conditions defined in the form of numerical ranges or percentage ranges are for the sake of brevity and convenience only. Accordingly, the description of numerical ranges or percentage ranges should be considered to encompass and specifically disclose all possible subranges and individual numerical values, especially integer values, within such ranges. For example, a range of "1 to 8" should be deemed to have specifically disclosed all subranges such as 1 to 7, 2 to 8, 2 to 6, 3 to 6, 4 to 8, 3 to 8, etc., specifically subranges defined by all integer values, and should be deemed to have specifically disclosed individual values such as 1, 2, 3, 4, 5, 6, 7, 8 etc. within the specifically disclosed ranges. Unless otherwise indicated, the foregoing method of interpretation is applicable to all content throughout the present invention, whether broad or not.

Where a quantity or other value or parameter is expressed in the form of a range, a preferred range, or a series of upper and lower limits, it is to be understood that all ranges consisting of any upper or preferred value of that range and lower limit or preferred value of that range have been specifically disclosed, whether or not those ranges are separately disclosed. Further, whenever a numerical range is referred to herein, unless otherwise indicated, such range shall include its endpoints and all integers and fractions within the range.

Herein, under the premise that the object of the invention can be achieved, the numerical value should be understood as having the precision of the effective digit of the numerical value. For example, the number 40.0 should be understood to cover the range from 39.50 to 40.49.

Herein, for the condition of using Markush group or optional terms to describe the features or examples of the present invention, one skilled in the art should understand that the subgroups of all elements or any individual elements in the Markush group or option list may also be used to describe the invention. For example, if X is described as "selected from the group consisting of X₁, X₂ and X₃", it also means that claim of X being X₁ and claim of X being X₁ and/or X₂ has been fully described. Furthermore, for the condition of using Markush group or optional terms to describe the features or examples of the present invention, one skilled in the art should understand that any combinations of the subgroups of all elements or any individual elements in the Markush group or option list may also be used to describe the invention. Accordingly, for example, if X is described as "selected from the group consisting of X₁, X₂ and X₃", and Y is described as "selected from the group consisting of Y₁, Y₂ and Y₃", it also means that claim of X being X₁ or X₂ or X₃ and Y being Y₁ or Y₂ or Y₃ has been fully described.

The following detailed embodiments are merely exemplary in nature and not intended to limit the invention and its uses. Furthermore, the present disclosure is not bound by any theory presented in the above prior art or summary, or the following detailed embodiments or examples.

According to one embodiment of the present invention, provided is a compound represented by formula (I), or a pharmaceutically acceptable salt, stereoisomer, enantiomer, diastereomer, atropisomer, racemate, polymorph, solvate or isotopically labeled compound thereof: 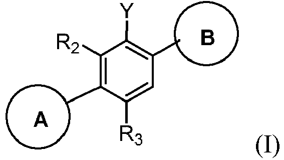 wherein
ring A is selected from the group consisting of 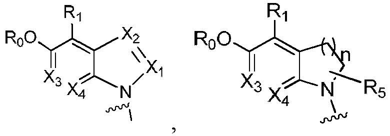 and 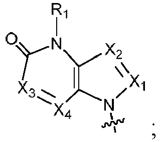
ring B is selected from the group consisting of 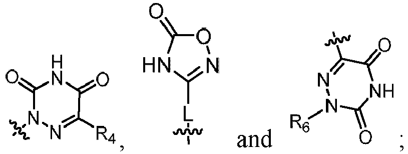
R₀ is selected from the group consisting of hydrogen, and C₁₋₁₀ alkyl;
R₁ is selected from the group consisting of hydrogen, substituted or unsubstituted C₁₋₁₀ alkyl, substituted or unsubstituted C₃₋₁₀ cycloalkyl, substituted or unsubstituted 3-10 membered heterocycloalkyl, substituted or unsubstituted C₆₋₁₀ aryl and substituted or unsubstituted 5-10 membered heteroaryl, wherein the substituents for the "substituted" are selected from the group consisting of halogen, hydroxyl, =O, C₁₋₆ alkoxy, C₁₋₆ alkyl, C₃₋₁₀ cycloalkyl, C₆₋₁₀ aryl, halogenated C₆₋₁₀ aryl, C₁₋₁₀ alkyl C₆₋₁₀ aryl, C₁₋₁₀ alkoxy C₆₋₁₀ aryl, 5-10 membered heteroaryl, C₁₋₁₀ alkyl 5-10 membered heteroaryl, halogenated 5-10 membered heteroaryl, 3-10 membered heterocycloalkyl and -NR¹⁰R¹¹;
R₂, R₃ and Y are each independently selected from the group consisting of hydrogen, halogen, substituted or unsubstituted C₁₋₆ alkyl, substituted or unsubstituted C₃₋₆ cycloalkyl, substituted or unsubstituted C₁₋₆ alkoxy, wherein the substituents for the "substituted" are selected from the group consisting of halogen, hydroxyl, C₁₋₆ alkyl and C₁₋₆ alkoxy;
R₄ is selected from the group consisting of hydrogen, cyano, -NR¹⁰R¹¹, substituted or unsubstituted C₁₋₆ alkyl, substituted or unsubstituted C₃₋₆ cycloalkyl and substituted or unsubstituted C₂₋₈ alkynyl, wherein the substituents for the "substituted" are selected from the group consisting of halogen, hydroxyl, cyano and C₁₋₆ alkoxy;
R₅ is selected from the group consisting of hydrogen, substituted or unsubstituted C₁₋₆ alkyl and substituted or unsubstituted C₃₋₆ cycloalkyl, wherein the substituents for the "substituted" are selected from the group consisting of halogen, hydroxyl and C₁₋₆ alkoxy;
R₆ is selected from the group consisting of hydrogen and substituted or unsubstituted C₁₋₆ alkyl, wherein the substituents for the "substituted" are selected from the group consisting of halogen, hydroxyl and C₁₋₆ alkoxy;
L is absent, or -NR¹⁰C(O)- or -NR¹⁰CR¹¹R¹¹-;
each R¹⁰ is independently selected from the group consisting of hydrogen and substituted or unsubstituted C₁₋₃ alkyl, wherein the substituents for the "substituted" are selected from the group consisting of halogen, hydroxyl and C₁₋₃ alkoxy;
each R¹¹ is independently selected from the group consisting of hydrogen and substituted or unsubstituted C₁₋₆ alkyl, wherein the substituents for the "substituted" are selected from the group consisting of halogen, hydroxyl and C₁₋₆ alkoxy;
X₁ and X₂ are each independently selected from the group consisting of N and CR¹², wherein R¹² is selected from the group consisting of hydrogen, halogen, cyano, -NR^{b}R^{c}, -C(=O)R^{a}, -C(=O)OR^{b}, -C(=O)NR^{b}R^{c}, substituted or unsubstituted C₁₋₄ alkyl, substituted or unsubstituted C₃₋₆ cycloalkyl, substituted or unsubstituted C₆₋₁₀ aryl and substituted or unsubstituted 5-10 membered heteroaryl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, halogen, cyano, hydroxyl, -OR^{a}, -NR^{b}R^{c}, -C(=O)R^{a}, -C(=O)OR^{b}, -C(=O)NR^{b}R^{c}, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
each R^{a} is independently C₁₋₆ alkyl, C₃₋₁₀ cycloalkyl or 3-10 membered heterocycloalkyl, wherein the alkyl, cycloalkyl and heterocycloalkyl are independently and optionally substituted by one or more substituents selected from the group consisting of halogen, hydroxyl, amino and C₁₋₆ alkyl;
each R^{b} and R^{c} is independently hydrogen, C₁₋₆ alkyl, C₃₋₁₀ cycloalkyl or 3-10 membered heterocycloalkyl, wherein the alkyl, cycloalkyl and heterocycloalkyl are independently and optionally substituted by one or more substituents selected from the group consisting of halogen, hydroxyl, amino and C₁₋₆ alkyl;
or R^{b} and R^{c}, together with the nitrogen atom to which they are connected, form a 3-10 membered heterocycloalkyl, wherein the heterocycloalkyl is optionally substituted by one or more substituents selected from the group consisting of halogen, hydroxyl, amino and C₁₋₆ alkyl;
n is independently 1, 2 or 3 at each occurrence.

According to one embodiment of the present disclosure, the compound of formula (I) is selected from the group consisting of the compounds represented by formula (Ia) or (Ib): 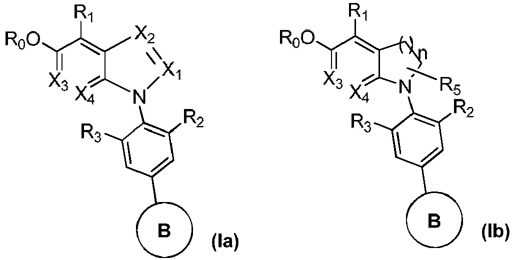 wherein
R₀ is hydrogen;
R₁ is selected from the group consisting of hydrogen, substituted or unsubstituted C₁₋₆ alkyl, substituted or unsubstituted C₃₋₆ cycloalkyl and substituted or unsubstituted C₃₋₈ heterocycloalkyl, wherein the substituents for the "substituted" are selected from the group consisting of halogen, a hydroxyl, =O, and C₁₋₆ alkoxy;
R₂ and R₃ are each independently selected from the group consisting of halogen, substituted or unsubstituted C₁₋₆ alkyl and substituted or unsubstituted C₃₋₆ cycloalkyl, wherein the substituents for the "substituted" are selected from the group consisting of halogen, hydroxyl, C₁₋₄ alkyl and C₁₋₄ alkoxy;
n is independently 1 or 2 at each occurrence;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
R₅ and B ring are as defined above.

According to one embodiment of the present disclosure, the compound of formula (I) is selected from the group consisting of the compounds represented by formula (Ic): 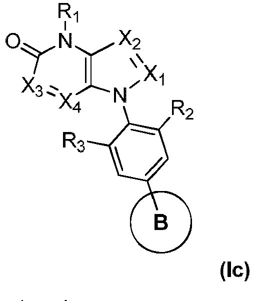 wherein
R₂ and R₃ are each independently selected from the group consisting of halogen, substituted or unsubstituted C₁₋₆ alkyl and substituted or unsubstituted C₃₋₆ cycloalkyl, wherein the substituents for the "substituted" are selected from the group consisting of halogen, hydroxyl, C₁₋₄ alkyl and C₁₋₄ alkoxy;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
R₁ and B ring are as defined above.

According to one embodiment of the present disclosure, the compound of formula (I) is selected from the group consisting of the compounds represented by formula (Id): 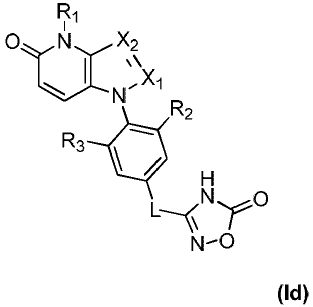 wherein
R₂ and R₃ are each independently selected from the group consisting of halogen;
L is -NHC(O)- or -NHCHR¹¹-; wherein R¹¹ is selected from the group consisting of hydrogen and substituted or unsubstituted C₁₋₆ alkyl, wherein the substituents for the "substituted" are selected from the group consisting of halogen, hydroxyl and C₁₋₆ alkoxy;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ is as defined above.

According to one embodiment of the present disclosure, the compound of formula (I) is selected from the group consisting of the compounds represented by formula (Ie): 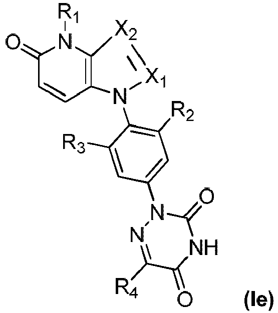 wherein
R₂ and R₃ are each independently selected from the group consisting of halogen;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ and R₄ are as defined above.

According to one embodiment of the present disclosure, the compound of formula (I) is selected from the group consisting of the compounds represented by formula (If): 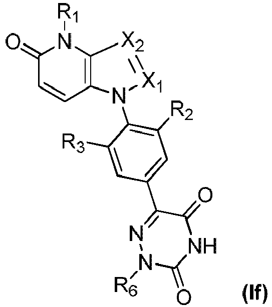 wherein
R₂ and R₃ are each independently selected from the group consisting of halogen;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ and R₆ are as defined above.

In the present invention, the heteroatom in the heterocycloalkyl and the heteroaryl is one or more selected from the group consisting of O, N and S, and the sulfur atom is optionally oxidized to form a sulfoxide group and a sulfone group.

According to one embodiment of the present disclosure, the compound of formula I is selected from the group consisting of:

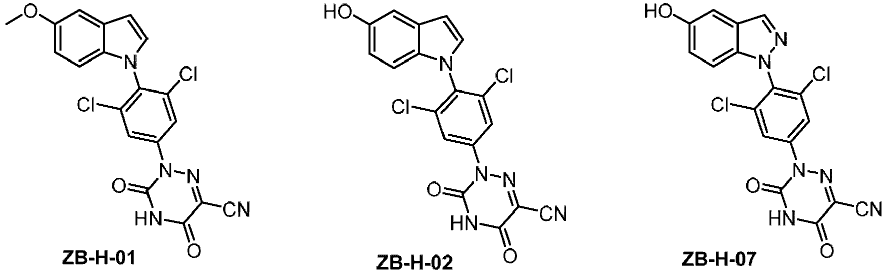

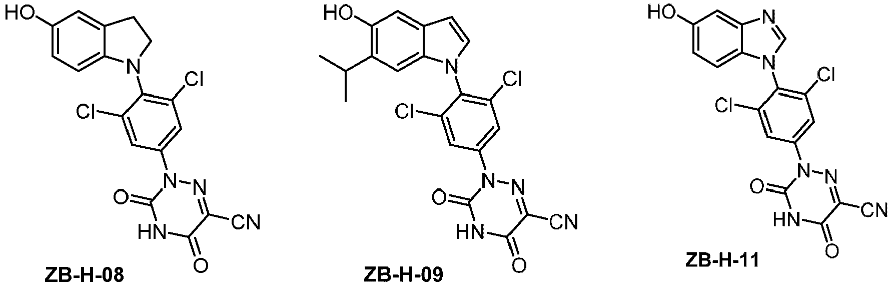

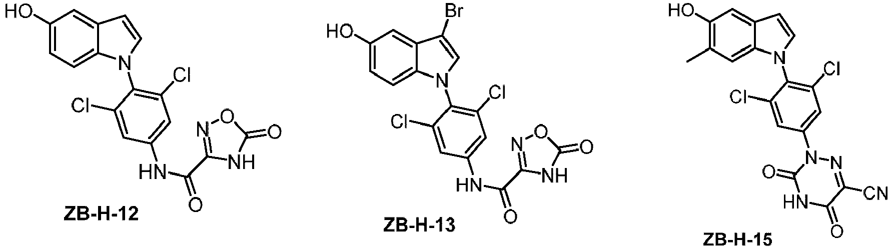

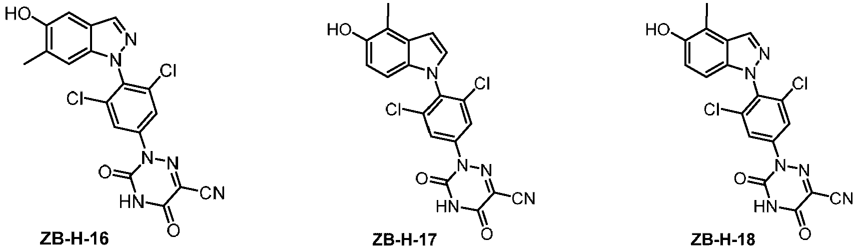

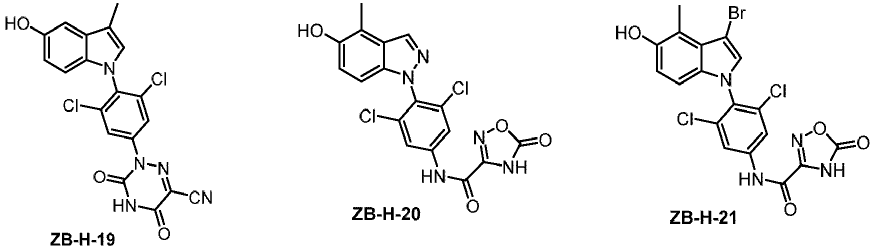

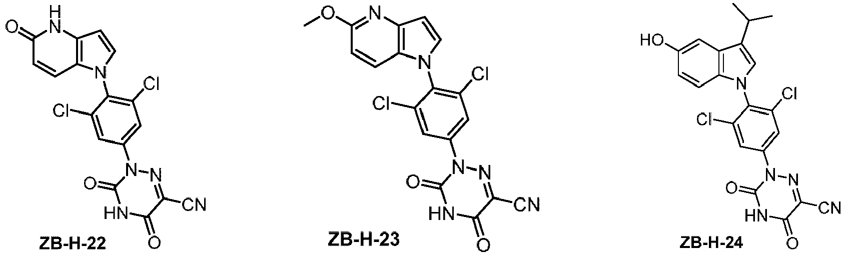

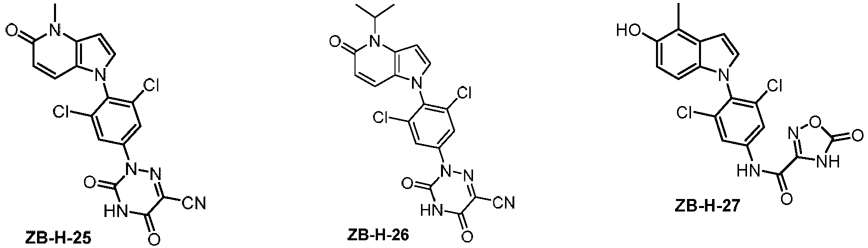

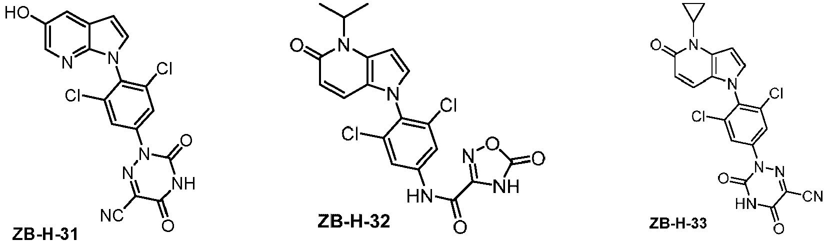

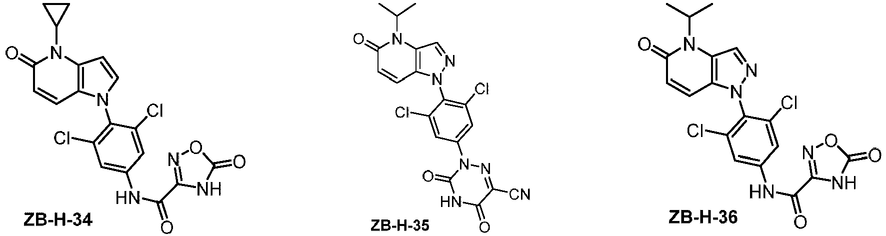

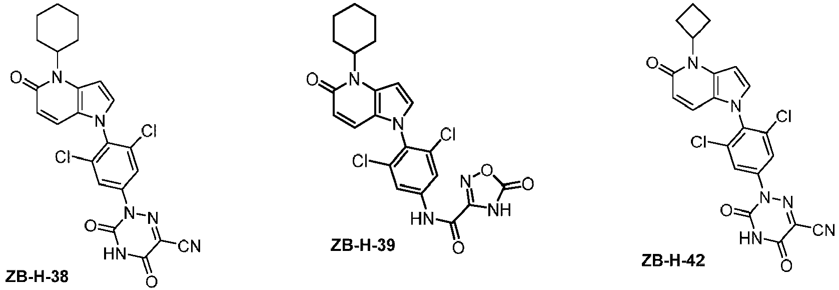

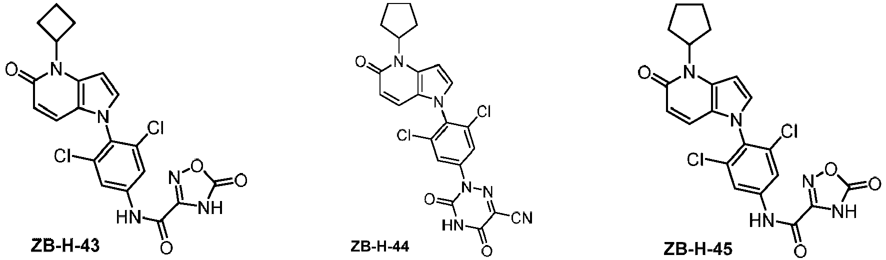

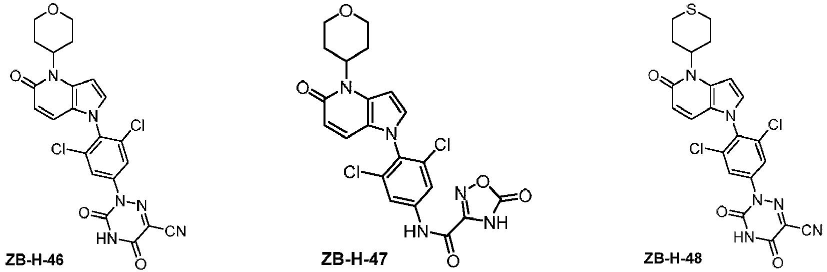

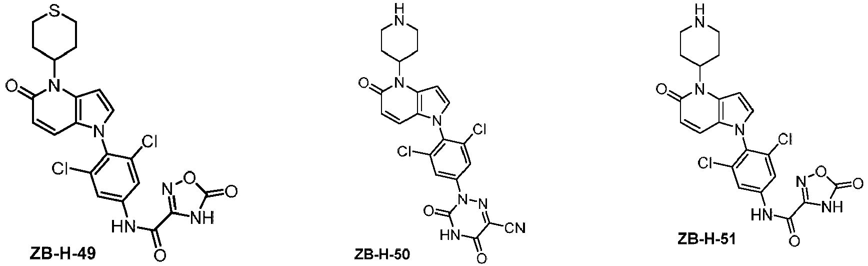

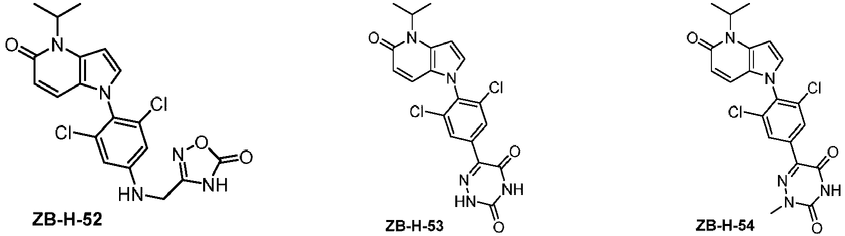

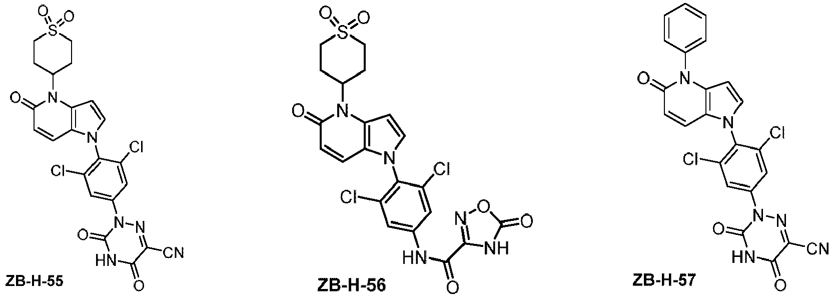

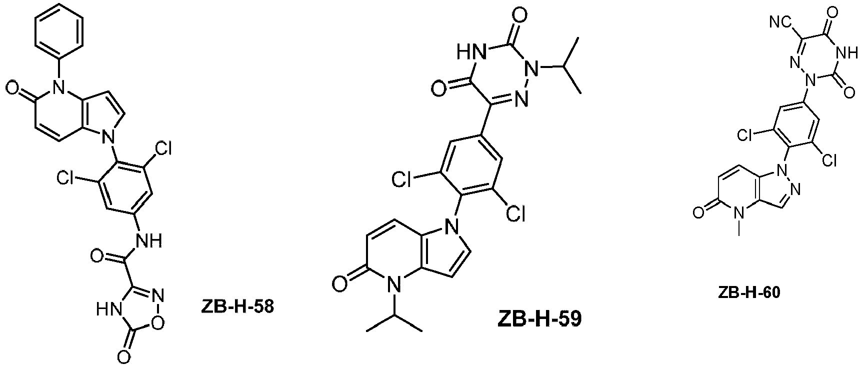

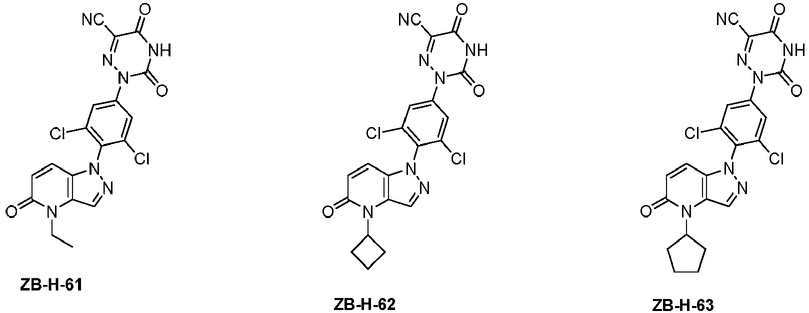

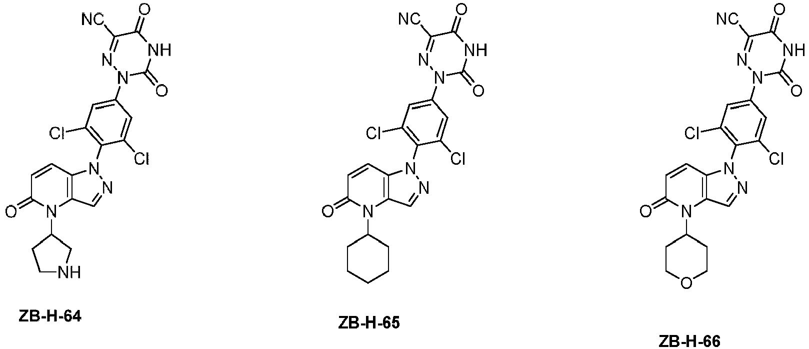

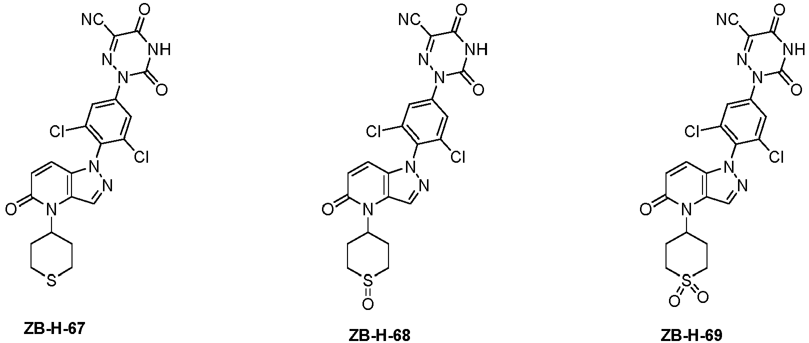

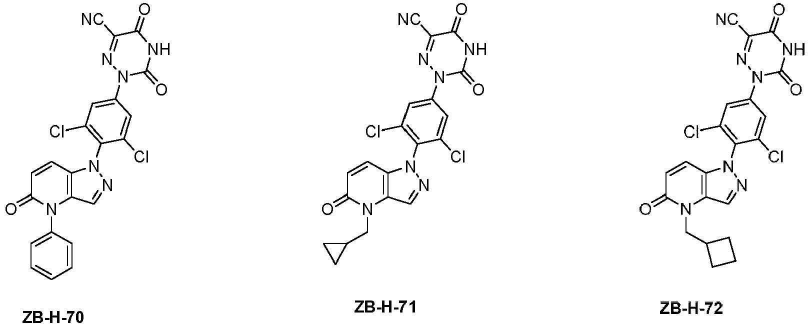

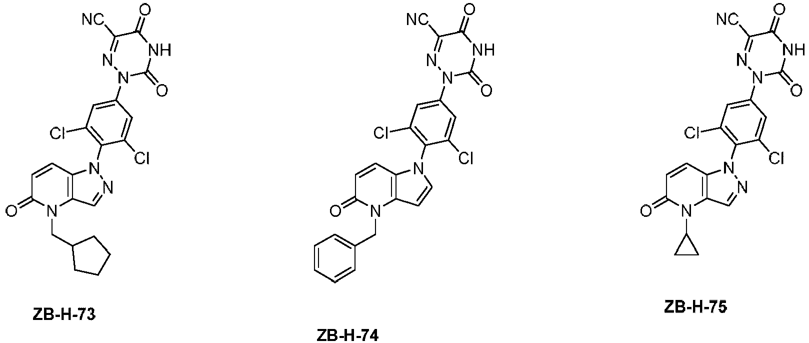

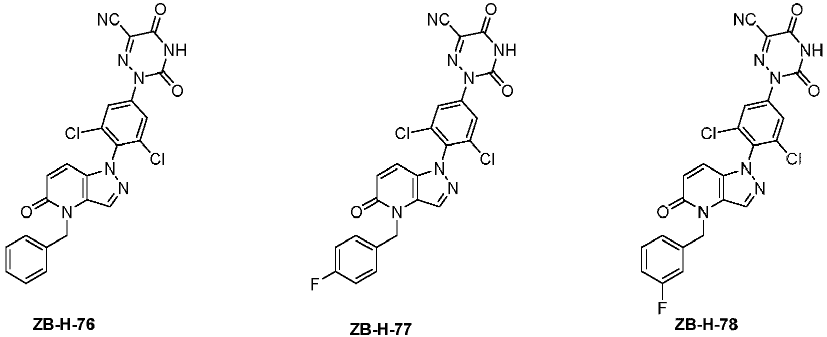

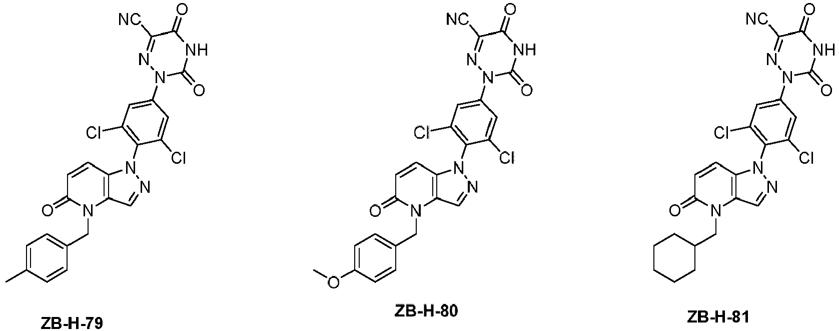

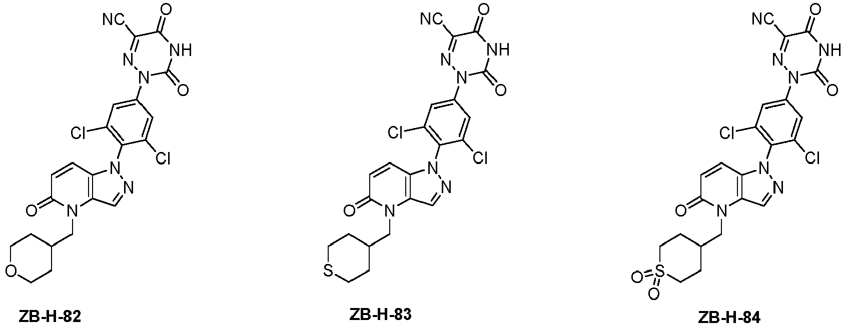

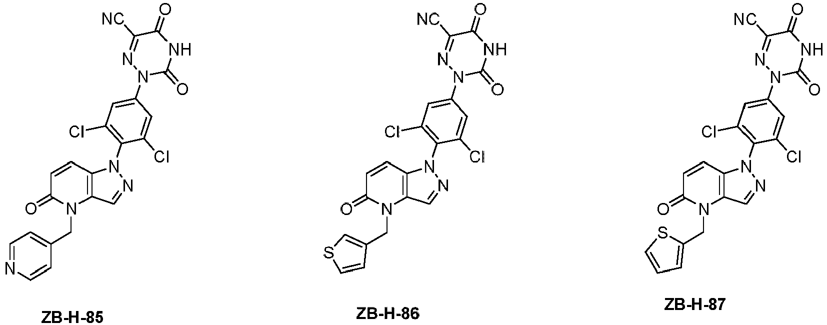

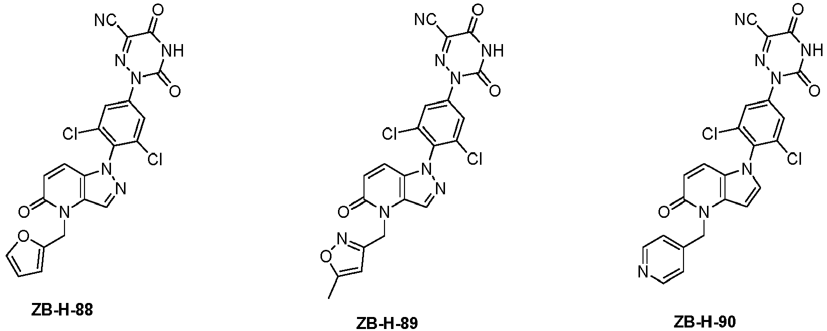

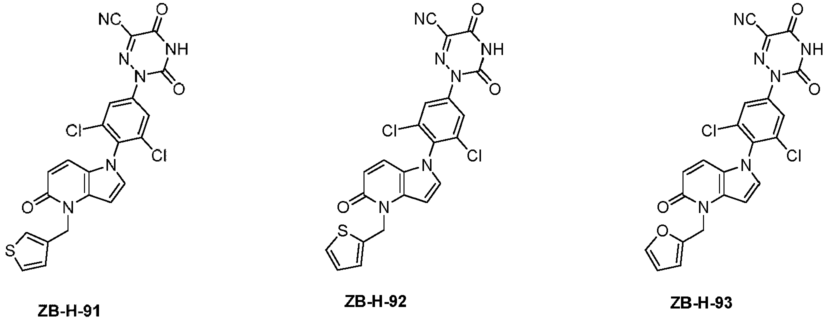

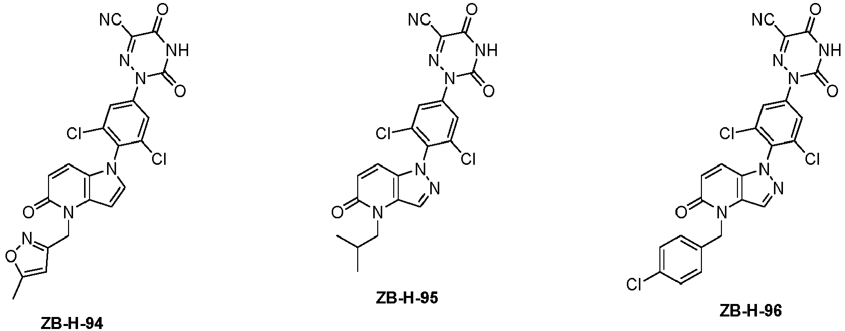

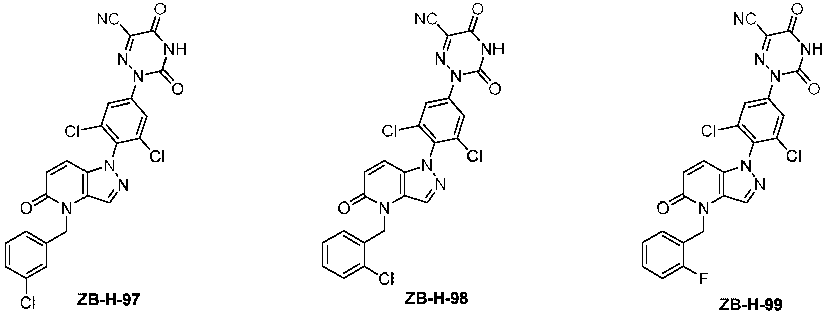

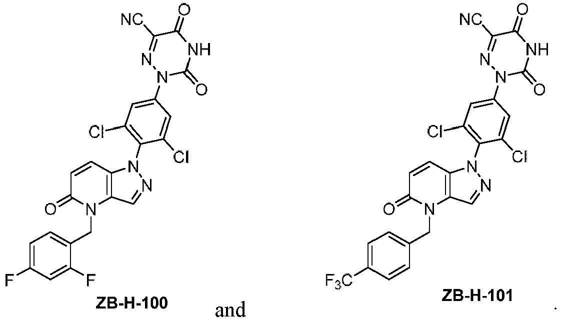

According to one embodiment of the present disclosure, provided is a pharmaceutical composition, comprising one or more selected from the group consisting of the above compound, and the pharmaceutically acceptable salt, stereoisomer, enantiomer, diastereoisomer, atropisomer, racemate, polymorph, solvate and isotopically labeled compound thereof, and optionally a pharmaceutically acceptable excipient.

According to one embodiment of the present disclosure, provided is use of the above compound, or the pharmaceutically acceptable salt, stereoisomer, enantiomer, diastereoisomer, atropisomer, racemate, polymorph, solvate or isotopically labeled compound thereof, or the above composition in preparation of a medicine for treatment of a metabolic-related disease.

According to one embodiment of the present disclosure, provided is a method for treating a metabolic-related disease, comprising administering to a subject an effective amount of one or more selected from the group consisting of the above compound, and the pharmaceutically acceptable salt, stereoisomer, enantiomer, diastereomer, atropisomer, racemate, polymorph, solvate or isotopically labeled compound thereof, or the above pharmaceutical composition.

According to one embodiment of the present disclosure, the metabolic-related disease is selected from the group consisting of obesity, hyperlipidemia, hypercholesterolemia, diabetes, and non-alcoholic steatohepatitis (NASH), hepatic steatosis, atherosclerosis, thyroid function hypothyroidism and thyroid cancer.

According to an embodiment of the present disclosure, the metabolic-related disease is selected from the group consisting of: non-alcoholic steatohepatitis (NASH), hypothyroidism and thyroid cancer.

### Examples

In the following examples, the optimal reaction conditions and reaction time for each individual step can be changed according to the specific reactants used and the substituents present in all reactants. Unless otherwise specified, the solvent, temperature, and other reaction conditions can be easily selected by those skilled in the art. The specific steps are provided in the section of synthetic examples. The reaction can be further processed in a conventional manner, for example, by removing the solvent from the residue and further purifying according to methods generally known in the art such as, but not limited to, crystallization, distillation, extraction, grinding, and chromatography. Unless otherwise specified, starting materials and reactants are commercially available or can be prepared by those skilled in the art from commercially available materials using methods described in chemical literature.

Routine tests, including proper adjustment of the reaction conditions, the reactants and sequence of the synthetic route, the protection of any chemical functional group which may not be compatible with the reaction conditions, and the deprotection at an appropriate point in the reaction sequence of the method, are all included within the scope of the invention. Appropriate protecting groups and methods for protecting and deprotecting different substituents using such appropriate protecting groups are well known to those skilled in the art; examples of which are found in T. Greene and P. Wuts, Protecting Groups in Chemical Synthesis (third edition), John Wiley & Sons, NY (1999), which is incorporated herein by reference in its entirety. The synthesis of the compounds of the present invention can be achieved by methods similar to those described in the synthetic schemes described above and in the specific examples.

If the starting material is not commercially available, it can be prepared by steps selected from the group consisting of standard organic chemistry techniques, techniques similar to the synthesis of known structural analogs, or techniques similar to the steps described in the above scheme or synthesis example section. When an optically active form of the compounds of the present invention are required, they can be obtained by performing one of the steps described herein using optically active starting materials (for example, prepared by asymmetric induction of an appropriate reaction step), or can be obtained by resolving the mixture of stereoisomers of compounds or intermediates by using standard procedures (for example, chromatographic separation, recrystallization or enzymatic resolution).

Similarly, when pure geometric isomers of the compounds of the present invention are required, they can be obtained by performing one of the steps described above using pure geometric isomers as starting materials, or can be obtained by resolving the mixture of geometric isomers of compounds or intermediates by using standard procedures, such as chromatographic separation.

The following examples are for illustrative purposes, and they are only used to explain the technical solutions of the present invention, and it is not intended to limit the present invention to these examples.

### Example 1: preparation of ZB-H-01 and ZB-H-02

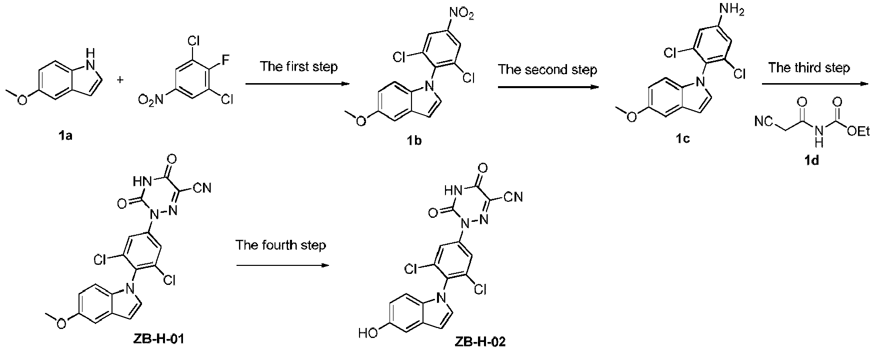

### The first step: preparation of 1b

To a solution of 5-methoxy-1H-indole (1a, 1.47 g, 10 mmol) in N,N-dimethylformamide (20 mL), 1,3-dichloro-2-fluoro-5-nitrobenzene (2.5 g, 12 mmol), potassium carbonate (2 g, 15 mmol) were added sequencely. After the addition, the reaction solution was raised to 100°C and stirred overnight. The reaction was stopped, and the reaction solution was cooled to room temperature naturally, added with saturated saline (100 mL), and extracted three times with ethyl acetate. The organic phases were combined, washed with saturated brine, and concentrated to dryness under reduced pressure to obtain **1b** (3 g), which was directly used in the next reaction. LC-MS [M+H]⁺: 338.

### The second step: preparation of 1c

To a solution of **1b** (1 g, 2.98 mmol) in ethanol (20 mL), stannous chloride dihydrate (3.4 g, 14.9 mmol) was added. After the addition, the reaction solution was raised to 80°C and stirred for 6 hours. After the reaction was stopped, the reaction solution was cooled to room temperature naturally, concentrated to dryness under a reduced pressure, and added with ethyl acetate (200 mL) to dissolve. The organic phase was washed with an aqueous sodium hydroxide solution (2M) three times, concentrated, and then subjected to column chromatography to obtain **1c** (750 mg). ¹H NMR (400 MHz, CDCl₃) δ 7.16 (s, 1H), 7.05 (d, *J* = 3.2 Hz, 1H), 6.90 - 6.82 (m, 2H), 6.74 (s, 2H), 6.62 (d, *J* = 3.2 Hz, 1H), 3.96 (s, 2H), 3.87 (s, 3H); LC-MS [M+H]⁺: 307.

### The third step: preparation of ZB-H-01

Tert-butyl nitrite (202 mg, 1.9 mmol) was dissolved in acetic acid (3 ml). This solution was slowly added to a solution of **1c** (500 mg, 1.63 mmol) in acetic acid (20 ml) and acetonitrile (10 mL) at 0°C, kept at 0°C and stirred for 30 minutes, added dropwise with an acetonitrile solution of N-cyanoacetylurethane (**1d**, 330 mg, 2.1 mmol), and stirred for 3 hours after the dropwise addition. After the reaction was stopped, the reaction solution was added to a saturated aqueous sodium bicarbonate solution (150 ml), and a red solid was filtered out, washed with water and petroleum ether, and dried. N,N-dimethylacetamide (5 ml) and potassium acetate (1.9 mmol) were added to the obtained solid, heated to 120°C and stirred for 6 hours, cooled to room temperature, added with 50 ml of water, and extracted with ethyl acetate three times. The organic phases were combined, washed with saturated brine, concentrated to dryness under reduced pressure, separated and purified by column chromatography to obtain **ZB-H-01** (520 mg). LC-MS [M+H]⁺: 428.

### The fourth step: preparation of ZB-H-02

**ZB-H-01** (30 mg) was dissolved in 20 mL of DCM and purged with argon three times, slowly added with a solution (2M, 0.7 mL) of boron tribromide in dichloromethane at -78°C, and then gradually raised to -10°C. After TLC monitoring showed the raw material point disappearred, 10 mL of a saturated sodium bicarbonate aqueous solution was added, stirred and warmed to room temperature. The reaction solution was allowed to stand for phase separation. The dichloromethane layer was separated, and extracted three times with ethyl acetate. The organic phases were combined, dried, concentrated to dryness under reduced pressure, separated and purified by thin layer chromatography (DCM:MeOH = 8:1) to obtain **ZB-H-02** (18 mg). ¹H NMR (400 MHz, DMSO-*d*₆) δ 8.89 (s, 1H), 7.89 (s, 2H), 7.36 (d, *J* = 3.2 Hz, 1H), 6.96 (s, 1H), 6.75 (d, *J=* 8.7 Hz, 1H), 6.65 (d, *J* = 8.7 Hz, 1H), 6.55 (d, *J=* 3.2 Hz, 1H); LC-MS [M+H]⁺: 414.

### Example 2: preparation of ZB-H-07

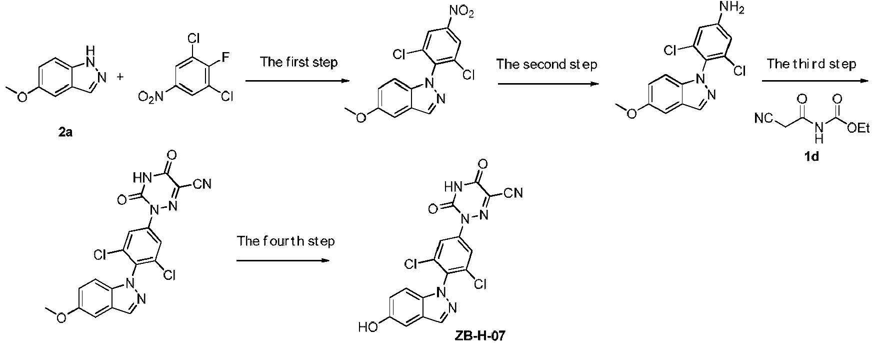

The target product **ZB-H-07** was finally prepared by using the synthetic route of Example 1, wherein the raw material **1a** in the first step was replaced by **2a,** and the remaining steps and conditions were carried out as described in Example 1. ¹H NMR (400 MHz, DMSO-*d*₆) δ 9.41 (s, 1H), 8.25 (s, 1H), 7.92 (s, 2H), 7.20 - 7.05 (m, 2H), 7.03 - 6.93 (m, 1H); LC-MS [M+H]⁺: 415.

### Example 3: preparation of ZB-H-08

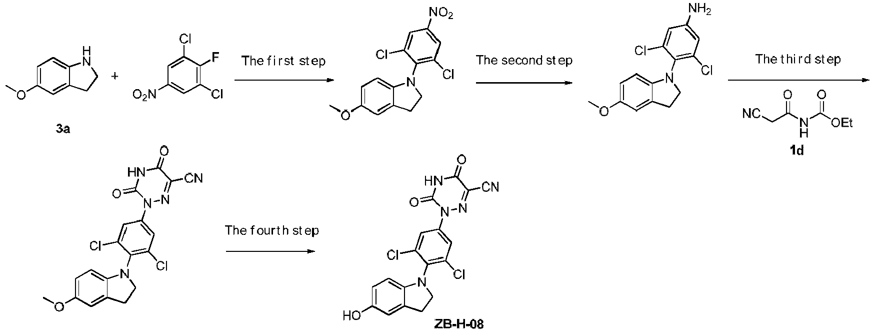

The target product **ZB-H-08** was finally prepared by using the synthetic route of Example 1, wherein the raw material **1a** in the first step was replaced by **3a,** and the remaining steps and conditions were carried out as described in Example 1. ¹H NMR (400 MHz, DMSO-*d*₆) δ 13.30 (s, 1H), 9.30 (s, 1H), 7.79 (s, 2H), 7.45 (d, *J=* 8.6 Hz, 1H), 6.72 (s, 1H), 6.61 (d, *J* = 7.3 Hz, 1H), 4.26 (t, *J* = 8.4 Hz, 2H), 3.18 (t, *J* = 8.4 Hz, 2H); LC-MS [M+H]⁺: 416.

### Example 4: preparation of ZB-H-09

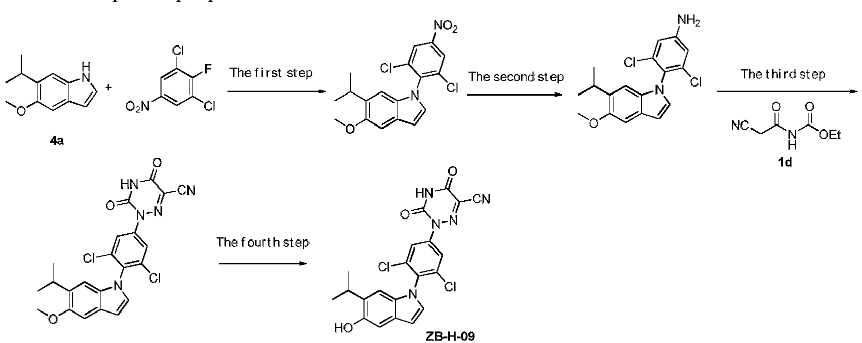

The target product **ZB-H-09** was finally prepared by using the synthetic route of Example 1, wherein the raw material **1a** in the first step was replaced by **4a,** and the remaining steps and conditions were carried out as described in Example 1. ¹H NMR (400 MHz, DMSO-*d*₆) δ 8.92 (s, 1H), 7.89 (s, 2H), 7.27 (d, *J* = 3.2 Hz, 1H), 6.98 (s, 1H), 6.61 (s, 1H), 6.51 (d, *J* = 3.2 Hz, 1H), 3.26 - 3.21 (m, 1H), 1.12 (d, *J* = 6.9 Hz, 6H); LC-MS [M+H]⁺: 456.

### Example 5: preparation of ZB-H-11

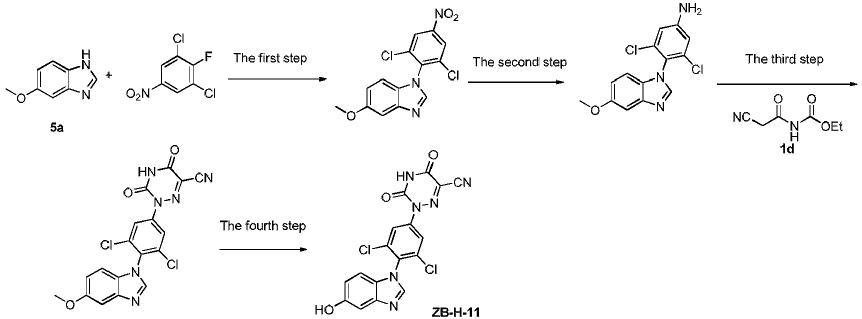

The target product **ZB-H-11** was finally prepared by using the synthetic route of Example 1, wherein the raw material **1a** in the first step was replaced by **5a,** and the remaining steps and conditions were carried out as described in Example 1. ¹H NMR (400 MHz, DMSO-*d*₆) δ 8.56 (s, 1H), 7.98 (s, 2H), 7.63 (d, *J* = 8.7 Hz, 1H), 6.87 (d, *J* = 8.7 Hz, 1H), 6.50 (s, 1H); LC-MS [M+H]⁺: 415.

### Example 6: preparation of ZB-H-15

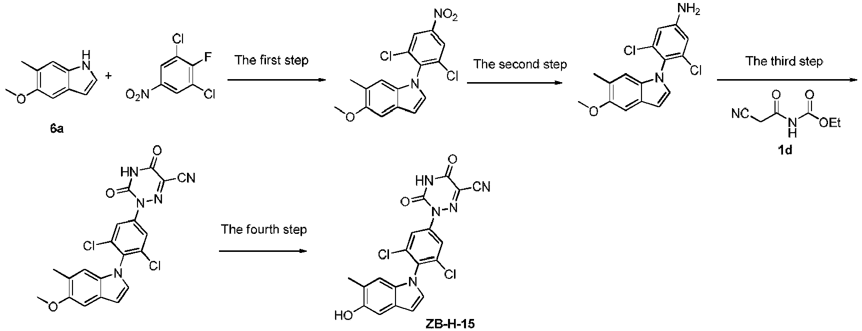

The target product **ZB-H-15** was finally prepared by using the synthetic route of Example 1, wherein the raw material **1a** in the first step was replaced by **6a,** and the remaining steps and conditions were carried out as described in Example 1. ¹H NMR (400 MHz, DMSO-*d*₆) δ 13.34 (s, 1H), 8.89 (s, 1H), 7.87 (s, 2H), 7.27 (d, *J=* 3.2 Hz, 1H), 6.98 (s, 1H), 6.65 (s, 1H), 6.52 (d, *J=* 3.1 Hz, 1H), 2.15 (s, 3H); LC-MS [M+H]⁺: 428.

### Example 7: preparation of ZB-H-16

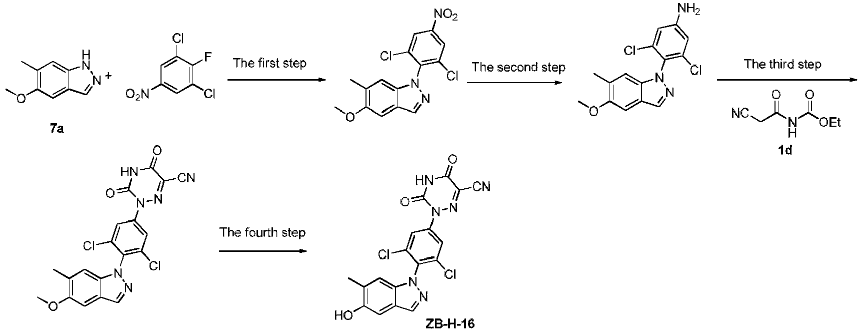

The target product **ZB-H-16** was finally prepared by using the synthetic route of Example 1, wherein the raw material **1a** in the first step was replaced by **7a,** and the remaining steps and conditions were carried out as described in Example 1. ¹H NMR (400 MHz, DMSO-*d*₆) δ 9.44 (s, 1H), 8.18 (s, 1H), 7.92 (s, 2H), 7.11 (s, 1H), 6.98 (s, 1H), 2.21 (s, 3H); LC-MS [M+H]⁺: 429.

### Example 8: preparation of ZB-H-17

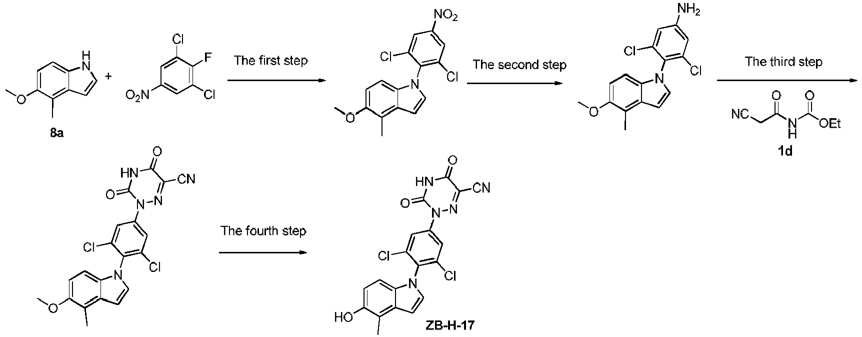

The target product **ZB-H-17** was finally prepared by using the synthetic route of Example 1, wherein the raw material **1a** in the first step was replaced by **8a,** and the remaining steps and conditions were carried out as described in Example 1. ¹H NMR (400 MHz, DMSO-*d*₆) δ 13.28 (s, 1H), 8.66 (s, 1H), 7.36 (d, *J* = 3.3 Hz, 1H), 6.71 (d, *J* = 8.6 Hz, 1H), 6.63 (d, *J=* 3.3 Hz, 1H), 6.58 (d, *J=* 8.6 Hz, 1H), 2.32 (s, 3H); LC-MS: [M+H]⁺: 428.

### Example 9: preparation of ZB-H-18

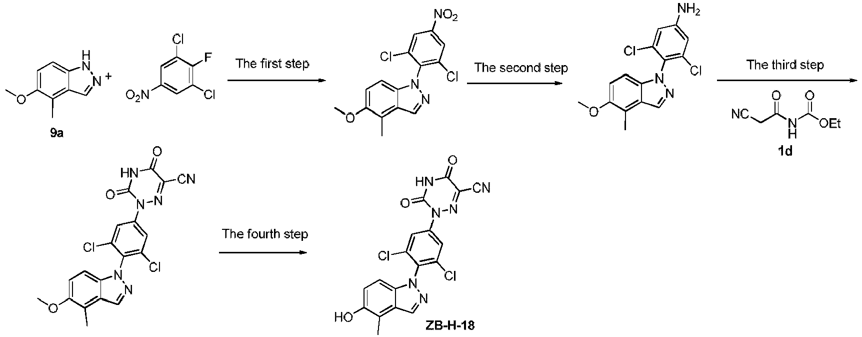

The target product **ZB-H-18** was finally prepared by using the synthetic route of Example 1, wherein the raw material **1a** in the first step was replaced by **9a,** and the remaining steps and conditions were carried out as described in Example 1. ¹H NMR (400 MHz, DMSO-*d*₆) δ 13.37 (s, 1H), 9.12 (s, 1H), 8.36 (s, 1H), 7.92 (s, 2H), 7.02 (d, *J=* 8.8 Hz, 1H), 6.91 (d, *J=* 8.8 Hz, 1H), 2.40 (s, 3H); LC-MS [M+H]⁺: 429.

### Example 10: preparation of ZB-H-19

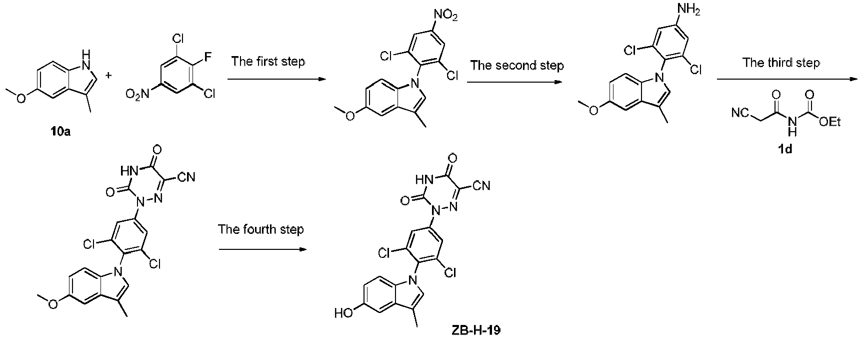

The target product **ZB-H-19** was finally prepared by using the synthetic route of Example 1, wherein the raw material **1a** in the first step was replaced by **10a,** and the remaining steps and conditions were carried out as described in Example 1. ¹H NMR (400 MHz, DMSO-*d*₆) δ 8.92 (s, 1H), 7.86 (s, 2H), 7.14 (s, 1H), 6.88 (s, 1H), 6.71 (d, *J* = 8.7 Hz, 1H), 6.65 (d, *J=* 8.7 Hz, 1H), 2.24 (s, 3H); LC-MS [M+H]⁺: 428.

### Example 11: preparation of ZB-H-22 and ZB-H-23

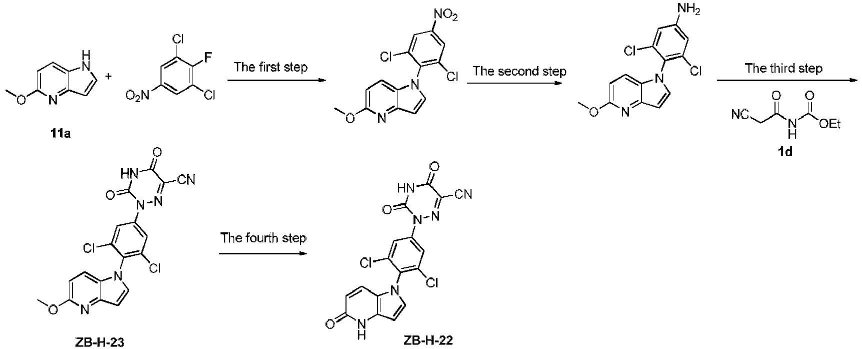

The target product **ZB-H-22** and **ZB-H-23** was finally prepared by using the synthetic route of Example 1, wherein the raw material **1a** in the first step was replaced by **11a,** and the remaining steps and conditions were carried out as described in Example 1.

**ZB-H-22:** ¹H NMR (400 MHz, DMSO-*d*₆) δ 11.71 (s, 1H), 7.89 (s, 2H), 7.41 (d, *J* = 3.1 Hz, 1H), 7.30 (d, *J* = 9.5 Hz, 1H), 6.28 (d, *J* = 3.0 Hz, 1H), 6.06 (d, *J* = 9.4 Hz, 1H); LC-MS [M+H]⁺: 415.

**ZB-H-23:** LC-MS [M+H]⁺: 429.

### Example 12: preparation of ZB-H-24

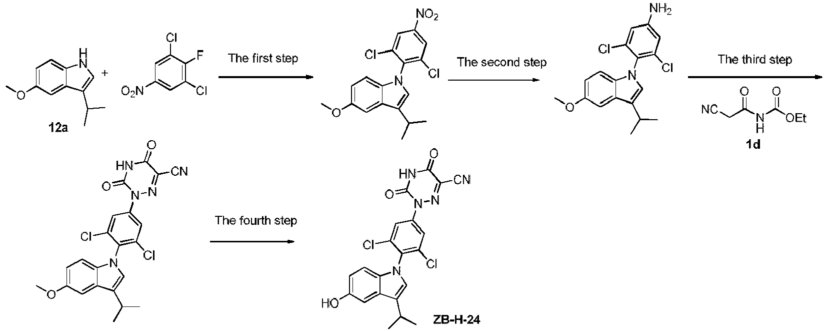

The target product **ZB-H-24** was finally prepared by using the synthetic route of Example 1, wherein the raw material **1a** in the first step was replaced by **12a,** and the remaining steps and conditions were carried out as described in Example 1. ¹H NMR (400 MHz, DMSO-*d*₆) δ 8.90 (s, 1H), 7.86 (s, 2H), 7.11 (s, 1H), 6.96 (d, *J* = 2.1 Hz, 1H), 6.71 (d, *J* = 8.7 Hz, 1H), 6.63 (d, *J =* 8.7 Hz, 1H), 3.15 - 3.02 (m, 1H), 1.31 (d, *J =* 6.8 Hz, 6H); LC-MS [M+H]⁺: 456.

### Example 13: preparation of ZB-H-31

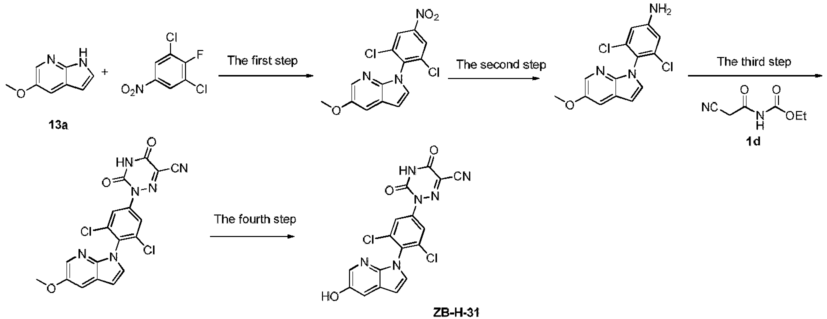

The target product **ZB-H-31** was finally prepared by using the synthetic route of Example 1, wherein the raw material **1a** in the first step was replaced by **13a,** and the remaining steps and conditions were carried out as described in Example 1. ¹H NMR (400 MHz, DMSO-*d*₆) δ 13.35 (s, 1H), 9.38 (s, 1H), 7.86 (s, 2H), 7.81 (s, 1H), 7.58 (d, *J=* 3.6 Hz, 1H), 7.41 (s, 1H), 6.61 (d, *J* = 3.6 Hz, 1H); LC-MS: [M+H]⁺: 415.

### Example 14: preparation of 14e

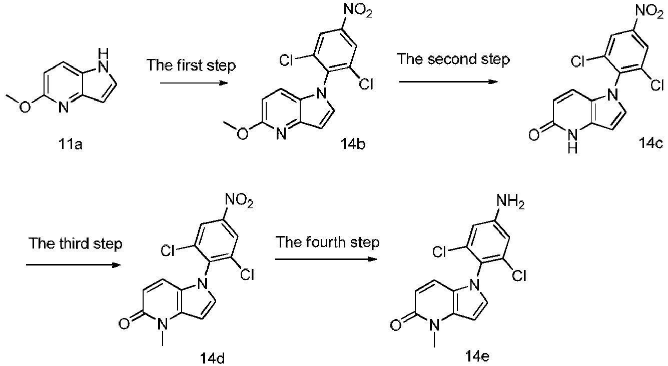

### The first step: preparation of intermediate 14b

The intermediate **14b** was prepared by using the synthetic route of the first step of Example 1, except that the raw material **1a** was replaced by **11a.** LC-MS [M+H]⁺: 339.

### The second step: preparation of intermediate 14c

**14b** (337 mg, 1 mmol) was dissolved in anhydrous acetonitrile (10 mL), stirred under the protection of argon, added with sodium iodide (750 mg, 5 mmol) and trimethylchlorosilane (0.6 mL, 5 mmol) respectively, and refluxed. After TLC monitoring showed the raw material point disappeared, the reaction solution was cooled to room temperature, continued stirring and quenched with methanol (5 mL), concentrated to dryness under reduced pressure, and separated and purified by column chromatography to obtain **14c** (249 mg). ¹H NMR (400 MHz, DMSO-*d*₆) δ 11.70 (s, 1H), 8.61 (s, 2H), 7.40 (d, *J* = 3.2 Hz, 1H), 7.29 (d, *J =* 9.5 Hz, 1H), 6.32 (d, *J =* 3.1 Hz, 1H), 6.09 (d, *J =* 9.5 Hz, 1H); LC-MS [M+H]⁺: 325.

### The third step: preparation of intermediate 14d

**14c** (323 mg, 1 mmol) was dissolved in 1,4-dioxane (5 mL) and put into a microwave reaction tube, added with methyl iodide (3 mmol) and cesium carbonate (3 mmol) respectively, and microwaved (150°C, 12 hours). After the reaction was finished, the reaction solution was concentrated to dryness under reduced pressure, and purified by column chromatography to obtain **14d** (288 mg). LC-MS: [M+H]⁺: 338.

### The fourth step: preparation of 14e

To a solution of **14d** (1 g, 2.98 mmol) in ethanol (20 mL), stannous chloride dihydrate (3.4 g, 14.9 mmol) was added. After the addition, the reaction solution was raised to 80°C and stirred for 6 hours. After the reaction was stopped, the reaction solution was cooled to room temperature naturally, concentrated to dryness under reduced pressure, added with ethyl acetate (200 mL) to dissolve. The organic phase was washed with sodium hydroxide aqueous solution (2M) three times, concentrated, and subjected to column chromatography to obtain **14e.** LC-MS [M+H]⁺: 308.

### Example 15: preparation of 15e

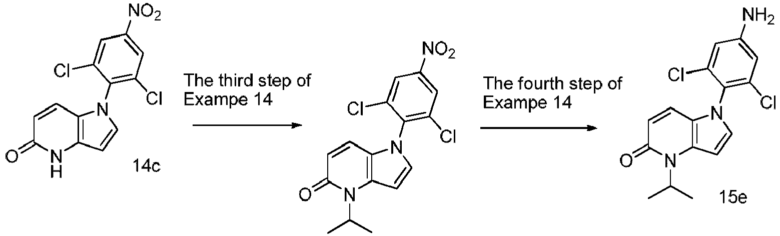

**15e** was prepared by using the synthetic route of Example 14, except that the raw material iodomethane in the third step was replaced by iodoisopropane. LC-MS: [M+H]⁺: 336.

### Example 16: preparation of 16e

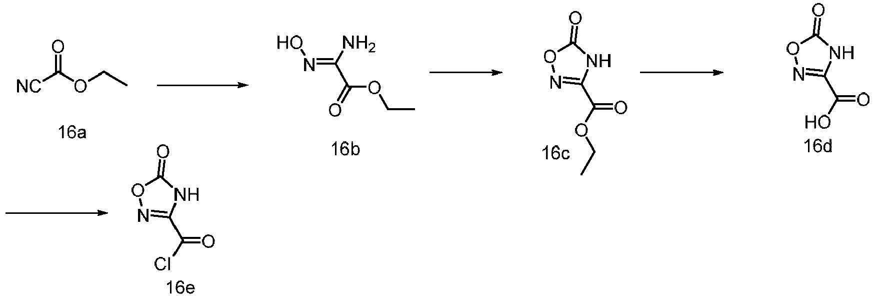

Step 1: To a solution of ethyl cyanoformate (**16a,** 9 g, 106 mmol) in ethanol (100 mL) and water (80 ml), hydroxylamine hydrochloride (11 g, 159 mmol) and sodium carbonate (11 g, 106 mmol) were added. The mixture was stirred at room temperature for 2 hours. The organic solvent was removed in vacuum, and the aqueous layer was extracted with dichloromethane (8 x 100 mL). The combined organic layers were dried over MgSO₄, filtered and concentrated to obtain **16b** (5.7 g) as a white solid. LC-MS [M+H]⁺: 133.

The second step: N,N'-carbonyldiimidazole (9.4 g, 58 mmol) and 1,8-diazabicycloundec-7-ene (8.7 g, 58 mmol) were added to a solution of **16b** (5.7 g, 48 mmol) in 1,4-dioxane (50 mL) and stirred at 80°C for 2 hours. The reaction solution was quenched with HCl, concentrated and extracted with dichloromethane (8 x 100 mL). The combined organic layers were concentrated and purified by column chromatography (DCM:EA=8:1) to give **16c** (3 g) as a yellow oil. ¹H NMR (400 MHz, DMSO-*d*₆) δ 13.41 (s, 1H), 4.37 (q, *J=* 7.1 Hz, 2H), 1.30 (t, *J=* 7.1 Hz, 3H); LC-MS [M-H]⁻: 157.

The third step: Lithium hydroxide (0.5 g, 20.8 mmol) was added to a mixed solution of **2** (3 g, 20.8 mmol) in tetrahydrofuran (30 mL) and water (30 mL), and stirred at room temperature for 3 hours. The mixture was washed with ethyl acetate (2 x 20 mL), acidified to pH = 3 with 1M HCl, concentrated, and extracted with ethyl acetate (8 x 20 mL). The organic phase was dried, and concentrated to dryness under reduced pressure to obtain **16d** (2 g) as a white solid. LC-MS [M-H]⁻: 129.

Step 4: **16d** (2 g, 15.4 mmol) was dissolved in anhydrous tetrahydrofuran, added with a drop of N,N-dimethylformamide, stirred at 0°C, added dropwise with oxalyl chloride (1.3 mL, 15.4 mmol), warmed to room temperature, kept stirring for 30 minutes, and concentrated to dryness under reduced pressure to obtain **16e** (1.8 g).

### Example 17: preparation of ZB-H-25

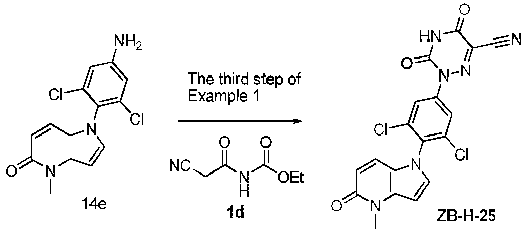

The target **ZB-H-25** was prepared by using the synthetic route of the third step and the fourth step in Example 1, except that **1c** in the third step was replaced by **14e.** ¹H NMR (400 MHz, DMSO-*d*₆) δ 7.90 (s, 2H), 7.51 (d, *J=* 3.2 Hz, 1H), 7.32 (d, *J=* 9.4 Hz, 1H), 6.61 (d, *J* = 3.0 Hz, 1H), 6.15 (d, *J=* 9.4 Hz, 1H), 3.55 (s, 3H); LC-MS: [M+H]⁺: 429.

### Example 18: preparation of ZB-H-26

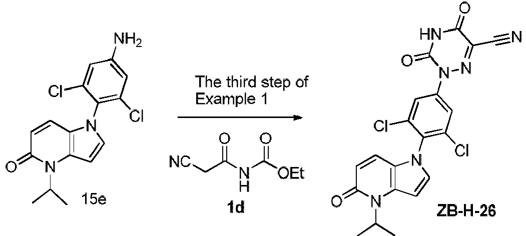

The target **ZB-H-26** was prepared by using the synthetic route of the third step and the fourth step in Example 1, except that **1c** in the third step was replaced by **15e.** ¹H NMR (400 MHz, DMSO-*d*₆) δ 13.35 (s, 1H), 7.89 (s, 2H), 7.50 (d, *J* = 3.2 Hz, 1H), 7.27 (d, *J* = 9.4 Hz, 1H), 6.76 (d, *J* = 3.2 Hz, 1H), 6.12 (d, *J* = 9.4 Hz, 1H), 5.47 - 5.06 (m, 1H), 1.51 (d, *J* = 7.0 Hz, 6H); LC-MS [M+H]⁺: 458.

### Example 19: preparation of ZB-H-32

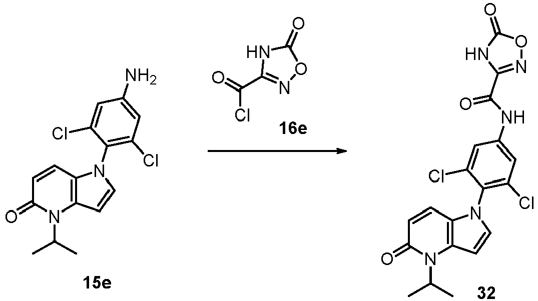

**15e** (63 mg, 0.19 mmol) was dissolved in anhydrous tetrahydrofuran (2 mL), added with triethylamine (0.6 mL, 0.9 mmol), stirred and cooled to 0°C, added dropwise with a solution of the intermediate **16e** (125 mg, 0.9 mmol) in anhydrous tetrahydrofuran (2 mL), warmed to room temperature, stirred for 30 minutes, concentrated to dryness under reduced pressure, separated and purified by column chromatography to obtain **ZB-H-32** (48 mg). ¹H NMR (400 MHz, DMSO-*d*₆) δ 13.49 (s, 1H), 11.52 (s, 1H), 8.12 (s, 2H), 7.41 (d, *J=* 3.2 Hz, 1H), 7.17 (d, *J* = 9.3 Hz, 1H), 6.70 (d, *J* = 3.3 Hz, 1H), 6.10 (d, *J* = 9.4 Hz, 1H), 5.40 - 5.17 (m, 1H), 1.50 (d, *J* = 7.0 Hz, 6H); LC-MS [M+H]⁺: 449.

### Example 20: preparation of 20e

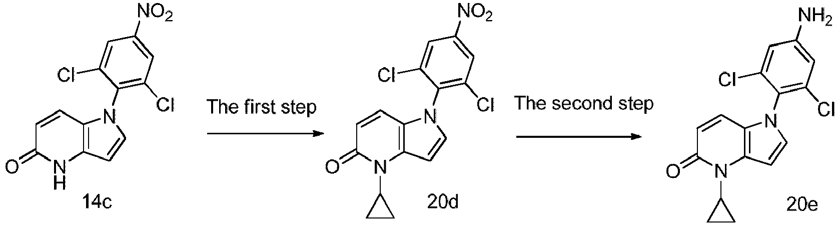

### The first step: preparation of 20d

**14c** (323 mg, 1 mmol) was dissolved in toluene (5 mL) and put into a microwave reaction tube, added with cyclopropylboronic acid (3 mmol), copper acetate (1 mmol), pyridine (5 mmol) and sodium bis(trimethylsilyl)amide (1 mmol) respectively, blown in oxygen, and microwaved (120°C, 3 hours). After the reaction was completed, the reaction solution was concentrated to dryness under reduced pressure, and purified by column chromatography to obtain **20d** (300 mg).

### The second step: preparation of 20e

**20e** was prepared by using the synthetic route of the first step in Example 1, except that the raw material **1b** was replaced by **20d.** LC-MS [M+H]⁺: 334.

### Example 21: preparation of 21e

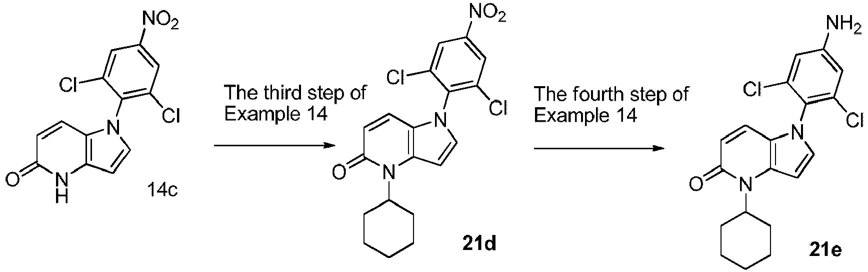

**21e** was prepared by using the synthetic route of Example 14, except that the raw material iodomethane in the third step was replaced by iodocyclohexane. LC-MS [M+H]⁺: 376.

### Example 22: preparation of 22e

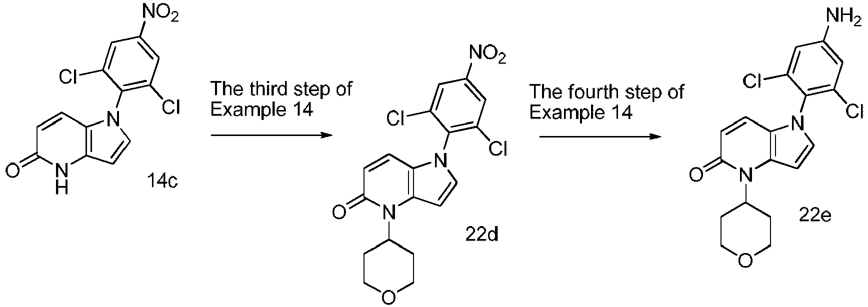

**22e** was prepared by using the synthetic route of Example 14, except that the raw material iodomethane in the third step was replaced by 4-bromotetrahydropyran. LC-MS [M+H]⁺: 378.

### Example 23: preparation of 23e

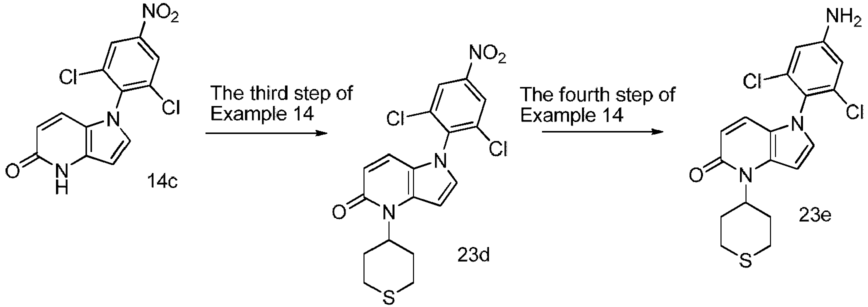

**23e** was prepared by using the synthetic route of Example 14, except that the raw material iodomethane in the third step was replaced by 4-bromotetrahydrothiopyran. LC-MS [M+H]⁺: 394.

### Example 24: preparation of 24e

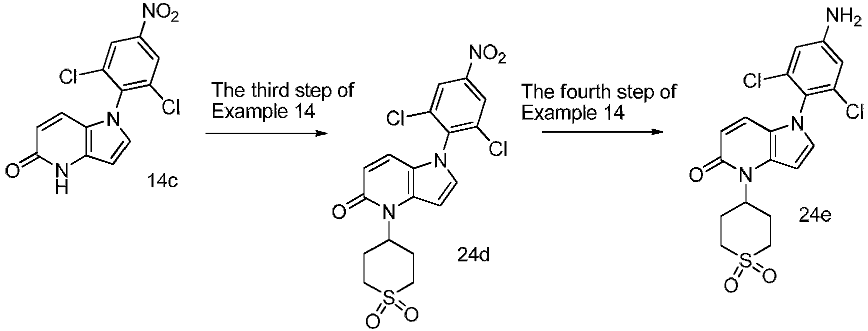

**24e** was prepared by using the synthetic route of Example 14, except that the raw material iodomethane in the third step was replaced by 4-bromotetrahydro-2H-thiopyran-1,1-dioxide. LC-MS: [M+H]⁺: 426.

### Example 25: preparation of 25e

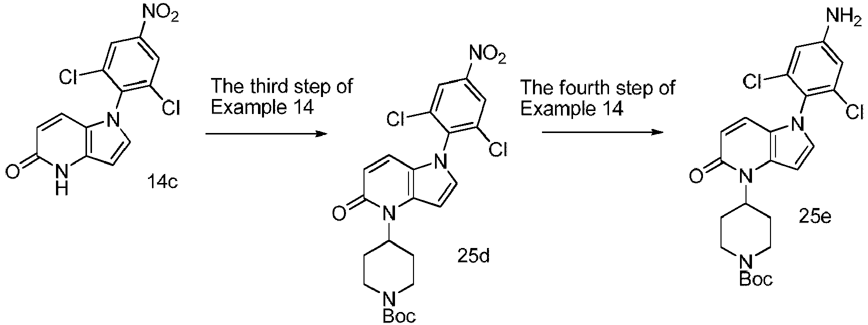

**25e** was prepared by using the synthetic route of Example 14, except that the raw material iodomethane in the third step was replaced by tert-butyl 4-iodopiperidine-1-carboxylate. LC-MS [M+H]⁺: 477.

### Example 26: preparation of 26e

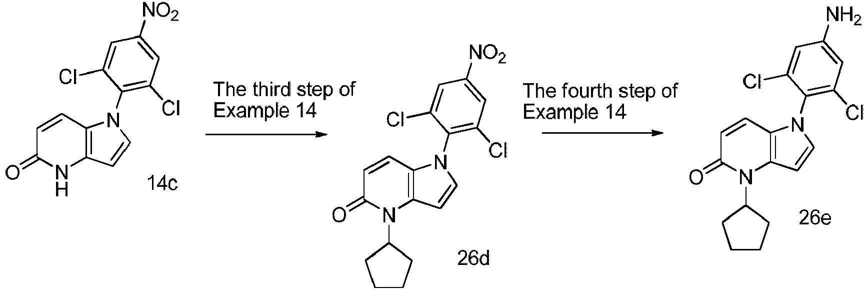

**26e** was prepared by using the synthetic route of Example 14, except that the raw material iodomethane in the third step was replaced by iodocyclopentane. LC-MS [M+H]⁺: 362.

### Example 27: preparation of 27e

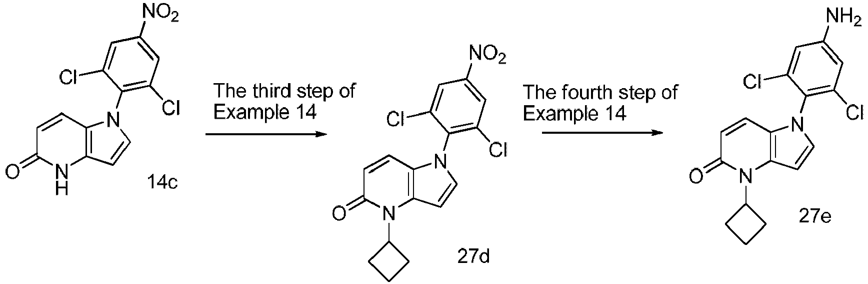

**27e** was prepared by using the synthetic route of Example 14, except that the raw material iodomethane in the third step was replaced by iodocyclobutane. LC-MS [M+H]⁺: 348.

### Example 28: preparation of ZB-H-33

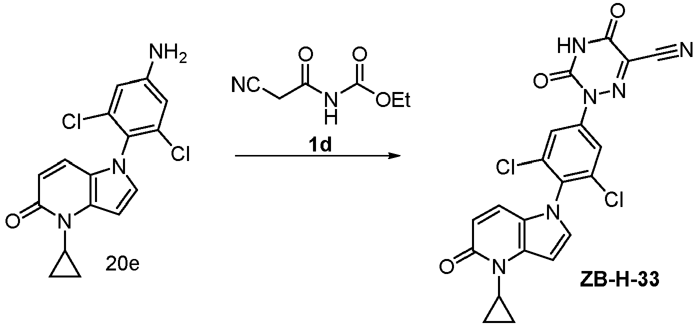

The target **ZB-H-33** was prepared by using the synthetic route of the third step and the fourth step in Example 1, except that the raw material **1c** in the third step was replaced by **20e.** ¹H NMR (400 MHz, DMSO-*d*₆) δ 13.35 (s, 1H), 7.89 (s, 2H), 7.48 (d, *J* = 3.2 Hz, 1H), 7.29 (d, *J* = 9.5 Hz, 1H), 6.61 (d, *J* = 3.2 Hz, 1H), 6.08 (d, *J* = 9.5 Hz, 1H), 3.05 (tt, *J* = 7.2, 4.1 Hz, 1H), 1.21 - 1.11 (m, 2H), 0.91 - 0.82 (m, 2H); LC-MS [M+H]⁺: 456.

### Example 29: preparation of ZB-H-34

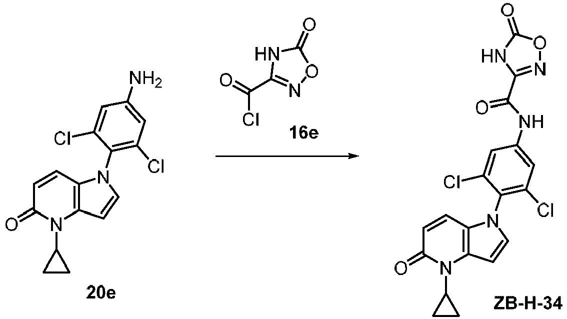

The target **ZB-H-34** was prepared by using the synthetic route of the Example 19, except that **15e** was replaced by **20e.** ¹H NMR (400 MHz, DMSO-*d*₆) δ 13.57 (s, 1H), 11.51 (s, 1H), 8.12 (s, 2H), 7.39 (d, *J=* 3.1 Hz, 1H), 7.19 (d, *J=* 9.5 Hz, 1H), 6.56 (d, *J* = 3.1 Hz, 1H), 6.07 (d, *J=* 9.4 Hz, 1H), 3.03 (tt, *J=* 7.1, 4.0 Hz, 1H), 1.21 - 1.12 (m, 2H), 0.91 - 0.83 (m, 2H); LC-MS [M+H]⁺: 447.

### Example 30: preparation of ZB-H-38

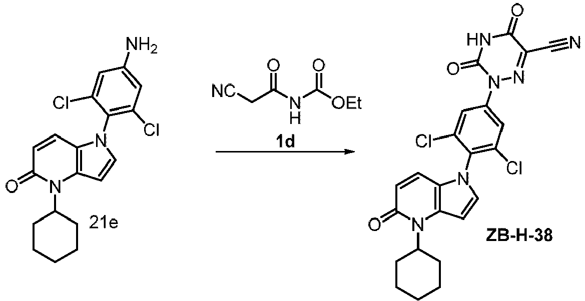

The target **ZB-H-38** was prepared by using the synthetic route of the third step and the fourth step in Example 1, except that the raw material **1c** in the third step was replaced by **21e.** ¹H NMR (400 MHz, DMSO-*d*₆) δ 13.34 (s, 1H), 7.89 (s, 2H), 7.49 (d, *J* = 3.2 Hz, 1H), 7.25 (d, *J=* 9.4 Hz, 1H), 6.80 (d, *J* = 3.0 Hz, 1H), 6.13 (d, *J=* 9.4 Hz, 1H), 2.41 - 2.28 (m, 1H), 1.95 - 1.09 (m, 10H); LC-MS [M+H]⁺: 498.

### Example 31: preparation of ZB-H-44

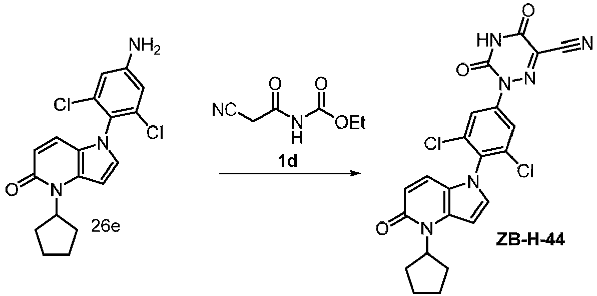

The target **ZB-H-44** was prepared by using the synthetic route of the third step and the fourth step in Example 1, except that the raw material **1c** in the third step was replaced by **26e.** LC-MS [M+H]⁺: 484.

### Example 32: preparation of ZB-H-42

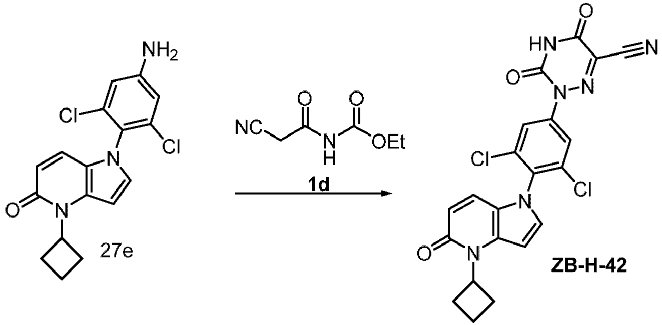

The target **ZB-H-42** was prepared by using the synthetic route of the third step and the fourth step in Example 1, except that the raw material **1c** in the third step was replaced by **27e.** LC-MS [M+H]⁺: 470.

### Example 33: preparation of ZB-H-46

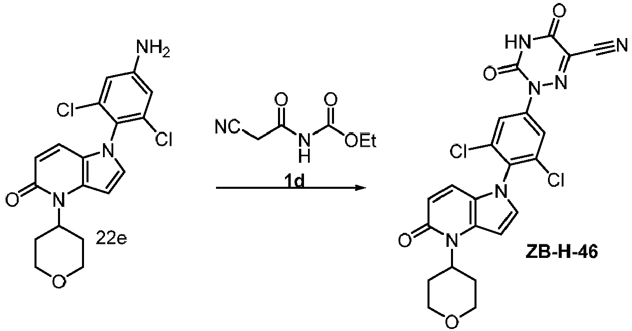

The target **ZB-H-46** was prepared by using the synthetic route of the third step and the fourth step in Example 1, except that the raw material **1c** in the third step was replaced by **22e.** LC-MS [M+H]⁺: 500.

### Example 34: preparation of ZB-H-48

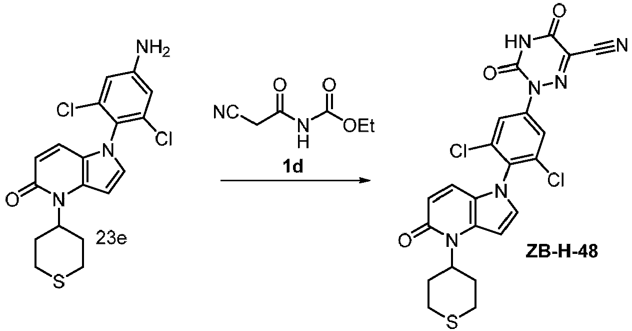

The target **ZB-H-48** was prepared by using the synthetic route of the third step and the fourth step in Example 1, except that the raw material **1c** in the third step was replaced by **23e.** LC-MS [M+H]⁺: 516.

### Example 35: preparation of ZB-H-55

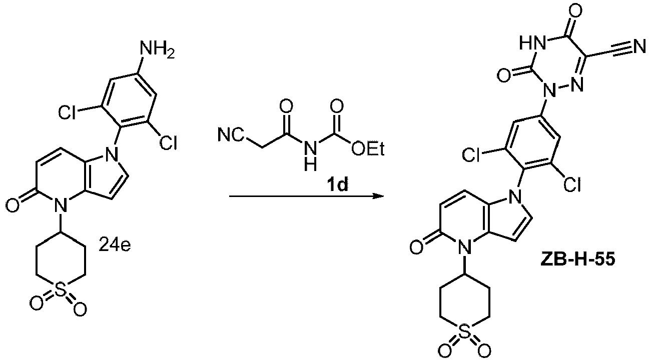

The target **ZB-H-49** was prepared by using the synthetic route of the third step and the fourth step in Example 1, except that the raw material **1c** in the third step was replaced by **24e.** LC-MS [M+H]⁺: 548.

### Example 36: preparation of ZB-H-50

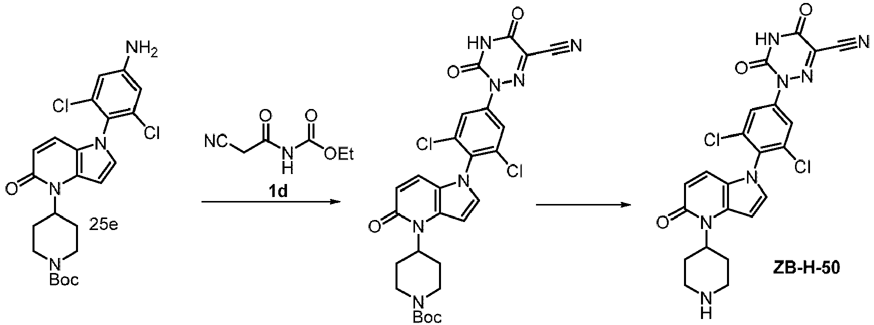

The target **ZB-H-50** was prepared by using the synthetic route of the third step and the fourth step in Example 1, except that the raw material 1c in the third step was replaced by **25e,** and the Boc protecting group was further removed. LC-MS [M+H]⁺: 499.

### Example 37: preparation of ZB-H-52

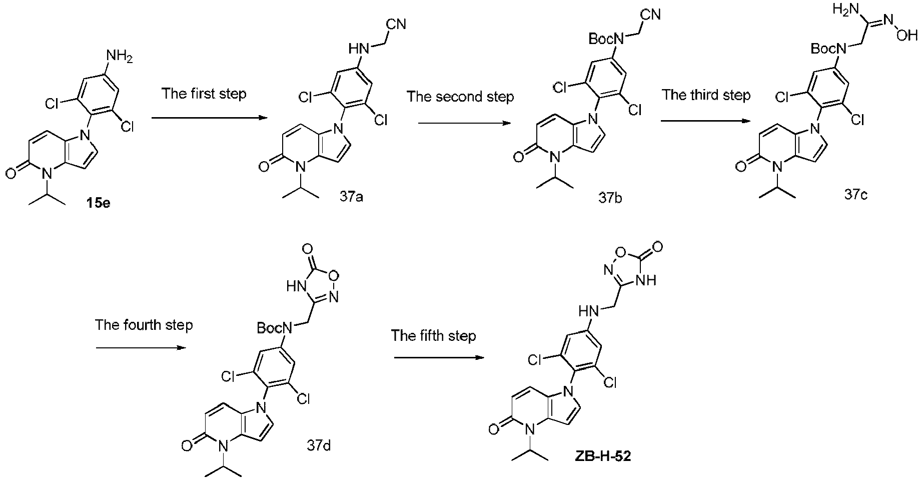

### The first step: preparation of intermediate 37a

To a solution of **15e** (1 mmol) in acetonitrile (4 mL), 2-bromoacetonitrile (3.66 mmol, 243 µL), NaI (219 mg, 1.46 mmol) and K₂CO₃ (202 mg, 1.46 mmol) were added. The mixture was then sealed in a tube and stirred at 100°C for 16 hours. LCMS showed complete consumption of the starting material. The suspension was filtered through a pad of celite, and the filter cake was washed with ethyl acetate. The combined washings were concentrated to dryness, separated and purified by column chromatography to obtain **37a.** LC-MS [M+H]⁺: 374.

### The second step: preparation of intermediate 37b

To a solution of **37a** (571.9 mmol) in tetrahydrofuran (3 mL), di-tert-butyl dicarbonate (374 mg, 1.72 mmol) and DMAP (70 mg, 571.85 mmol) were added, and the mixture was stirred at 40°C for 3 hours. LCMS showed complete consumption of the starting material and the desired MS was detected. The mixture was partitioned between ethyl acetate and water, and the aqueous layer was extracted with ethyl acetate. The combined organic phases were washed with brine, dried over anhydrous sodium sulfate, filtered and concentrated in vacuum to obtain a residue, which was separated and purified by column chromatography to obtain 37b. LC-MS [M+H]⁺: 474.

### The third step: preparation of intermediate 37c

To a solution of 37b (534.9 mmol) in DMF (3 mL), hydroxylamine hydrochloride (297 mg, 4.28 mmol) and NaOAc (351 mg, 4.28 mmol) were added. The mixture was stirred at 80°C for 1 hour. LCMS showed complete consumption of the starting material. The reaction mixture was concentrated under reduced pressure to remove DMF. The residue was partitioned between ethyl acetate and water, and the aqueous layer was extracted with ethyl acetate. The combined organic layers were washed with brine, dried over anhydrous Na₂SO₄, filtered and concentrated in vacuum to obtain **37c,** which was directly used in the next step without further purification. LC-MS [M+H]⁺: 507.

### The fourth step: preparation of intermediate 37d

To a solution of **37c** (520 mmol) in tetrahydrofuran (3 mL), DSC (173 mg) and TEA (105 mg, 1.04 mmol) were added. The mixture was stirred at 60°C for 16 hours. LCMS showed complete consumption of the starting material and the desired MS was detected. The mixture was partitioned between ethyl acetate and water, and the aqueous layer was extracted with ethyl acetate. The combined organic layers were washed with brine, dried over anhydrous sodium sulfate, filtered and concentrated in vacuum to obtain a residue, which was separated and purified by column chromatography to obtain **37d.** LC-MS [M+H]⁺: 533.

### The fifth step: preparation of ZB-H-52

**37d** (323.0 mmol) was put in hydrogen chloride/ethyl acetate (2 mL) and stirred at 25°C for 2 hours. LCMS showed complete consumption of the starting material and the desired MS was detected. The mixture was diluted with water (0.5 mL), and the pH was adjusted to 8 with aqueous NaHCO₃. The mixture was extracted with ethyl acetate. The combined organic layers was washed with brine, dried over anhydrous sodium sulfate, filtered and concentrated in vacuum to give a residue. The crude product was purified by preparative HPLC column to obtain the titled **ZB-H-52.** LC-MS [M+H]⁺: 433.

### Example 38: preparation of ZB-H-53

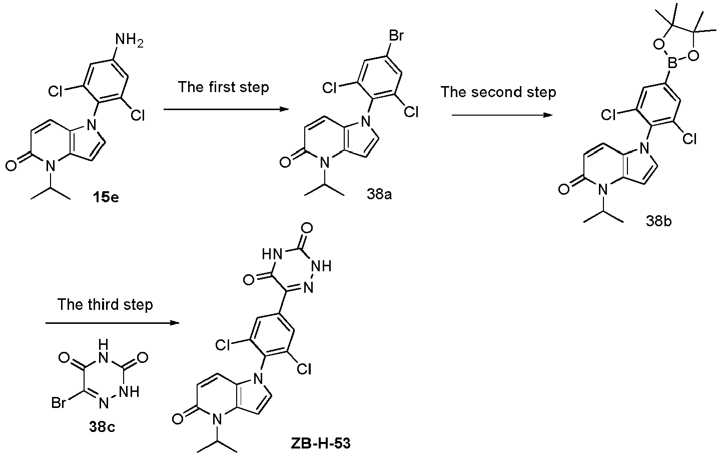

### The first step: preparation of intermediate 38a

**15e** (1 mmol) was dissolved in acetonitrile (5 mmol), added with cuprous iodide (2 mmol), and dropwise added with tert-butyl nitrite (2 mmol) under ice-water bath, stirred overnight at room temperature, rotarily evaporated to remove the solvent, and separated and purified by column chromatography to obtain **38a.** LC-MS: [M+H]⁺: 398.

### The second step: preparation of intermediate 38b

**38a** (1 mmol) was dissolved in dry 1,4-dioxane, added with diboron pinacol ester (2 mmol), potassium acetate (3 mmol) and [1,1'-bis(diphenylphosphino)ferrocene]palladium dichloride (0.05 mmol), reacted overnight at 80°C, rotarily evaporated to remove the solvent, and separated and purified by column chromatography to obtain **38b.** LC-MS: [M+H]⁺: 447.

### The third step: preparation of ZB-H-53

**38b** (1 mmol) and 5-bromo-6-azauracil **(38c,** 1 mmol) were dissolved in 1,4-dioxane (5 mL), added with 2M aqueous sodium carbonate solution (2.5 mL), and added with [1,1'-bis (diphenylphosphino)ferrocene]palladium dichloride (0.1 mmol) under argon protection, refluxed at 110°C for 3 hours under the protection of argon, cooled to room temperature, added with 50 ml of water, and extracted with ethyl acetate three times. The organic phases were combined, washed with saturated brine, concentrated to dryness under reduced pressure, separated and purified by column chromatography to obtain **ZB-H-53.** LC-MS [M+H]⁺: 432; ¹H NMR (400 MHz, DMSO-*d*₆) δ 12.82 (s, 1H), 12.32 (s, 1H), 8.15 (s, 2H), 7.46 (d, *J* = 3.2 Hz, 1H), 7.18 (d, *J* = 9.4 Hz, 1H), 6.73 (d, *J* = 3.2 Hz, 1H), 6.11 (d, *J* = 9.4 Hz, 1H), 5.51 - 5.00 (m, 1H), 1.51 (d, *J* = 7.0 Hz, 6H)

### Example 39: preparation of ZB-H-54

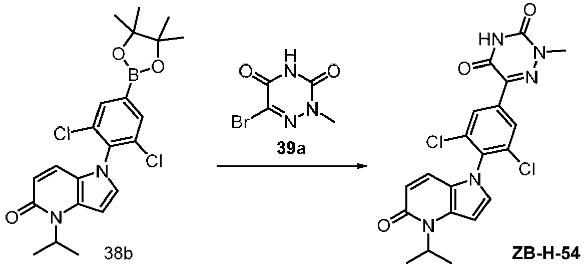

**ZB-H-54** was prepared by using the synthetic route of the third step in Example 38, except that the raw material **38c** in the third step was replaced by **39a.** LC-MS [M+H]⁺: 498. ¹H NMR (400 MHz, DMSO-*d*₆) δ 12.52 (s, 1H), 8.20 (s, 2H), 7.46 (d, *J=* 3.2 Hz, 1H), 7.19 (d, *J=* 9.4 Hz, 1H), 6.74 (d, *J=* 3.1 Hz, 1H), 6.11 (d, *J=* 9.4 Hz, 1H), 5.47 - 5.00 (m, 1H), 3.60 (s, 3H), 1.51 (d, *J=* 7.0 Hz, 6H).

### Example 40: preparation of ZB-H-39

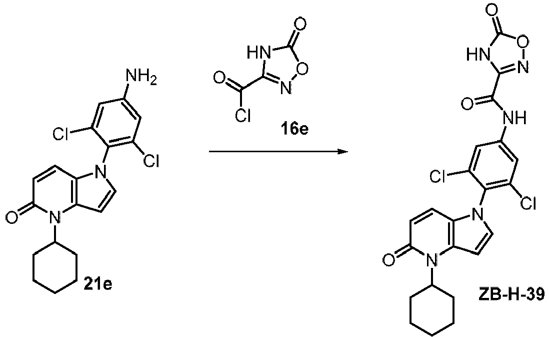

The target **ZB-H-39** was prepared by using the synthetic route of Example 19, except that **15e** was replaced by **21e.** LC-MS [M+H]⁺: 488.

### Example 41: preparation of ZB-H-43

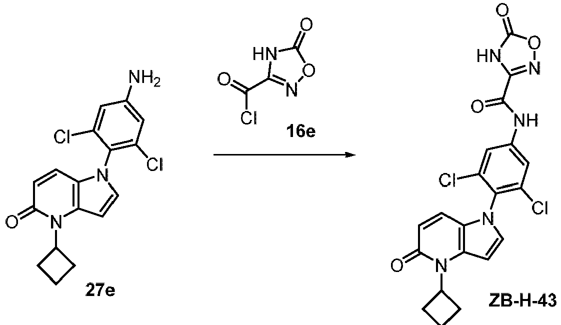

The target **ZB-H-43** was prepared by using the synthetic route of Example 19, except that the raw material **15e** was replaced by **27e.** LC-MS [M+H]⁺: 460.

### Example 42: preparation of ZB-H-45

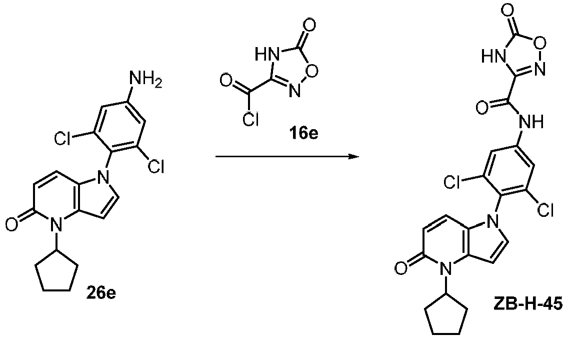

The target **ZB-H-45** was prepared by using the synthetic route of Example 19, except that **15e** was replaced by **26e.** LC-MS [M+H]⁺: 474. ¹H NMR (400 MHz, DMSO-*d*₆) δ 13.35 (s, 1H), 7.89 (s, 2H), 7.50 (d, *J=* 3.2 Hz, 1H), 7.27 (d, *J=* 9.4 Hz, 1H), 6.62 (d, *J* = 3.2 Hz, 1H), 6.12 (d, *J =* 9.4 Hz, 1H), 5.45 - 5.04 (m, 1H), 2.27 - 2.11 (m, 2H), 2.04 - 1.82 (m, 4H), 1.75 - 1.60 (m, 2H).

### Example 43: preparation of ZB-H-47

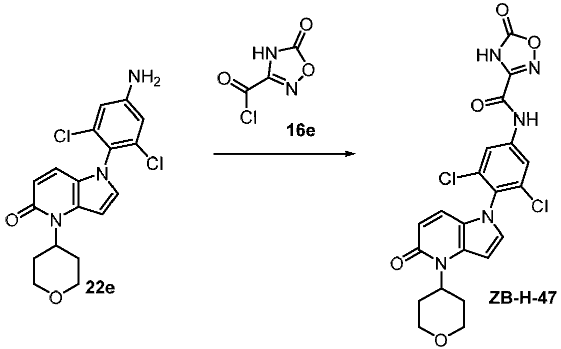

The target **ZB-H-47** was prepared by using the synthetic route of Example 19, except that **15e** was replaced by **22e.** LC-MS [M+H]⁺: 490. ¹H NMR (400 MHz, DMSO-*d*₆) δ 13.34 (s, 1H), 7.89 (s, 2H), 7.50 (d, *J=* 3.2 Hz, 1H), 7.28 (d, *J=* 9.4 Hz, 1H), 6.76 (d, *J=* 3.2 Hz, 1H), 6.14 (d, *J =* 9.4 Hz, 1H), 5.22 - 4.90 (m, 1H), 4.11 - 3.88 (m, 2H), 3.56 - 3.51 (m, 2H), 2.76 - 2.57 (m, 2H), 1.65 - 1.54 (m, 2H).

### Example 44: preparation of ZB-H-49

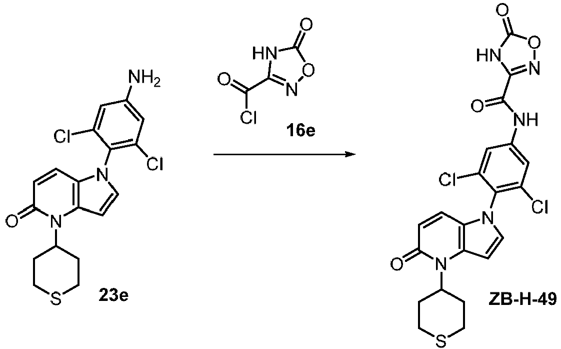

The target **ZB-H-49** was prepared by using the synthetic route of Example 19, except that **15e** was replaced by **23e.** LC-MS [M+H]⁺: 506.

### Example 45: preparation of ZB-H-51

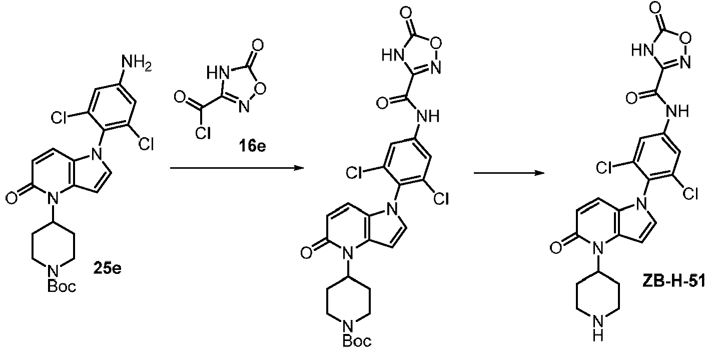

The target **ZB-H-51** was prepared by using the synthetic route of Example 19, except that **15e** was replaced by **25e,** and the Boc protecting group was further removed. LC-MS [M+H]⁺: 489.

### Example 46: preparation of ZB-H-56

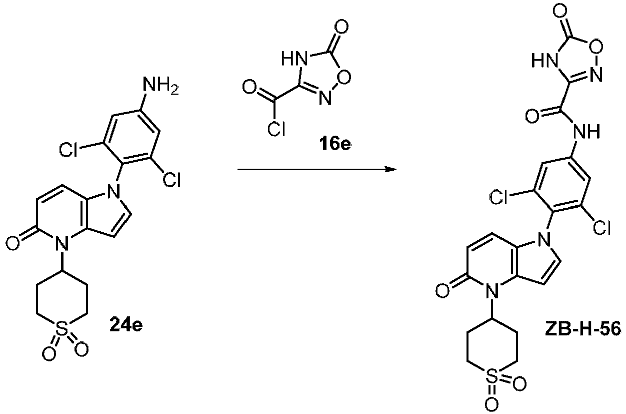

The target **ZB-H-56** was prepared by using the synthetic route of Example 19, except that **15e** was replaced by **24e.** LC-MS [M+H]⁺: 538.

### Example 47: preparation of 47b

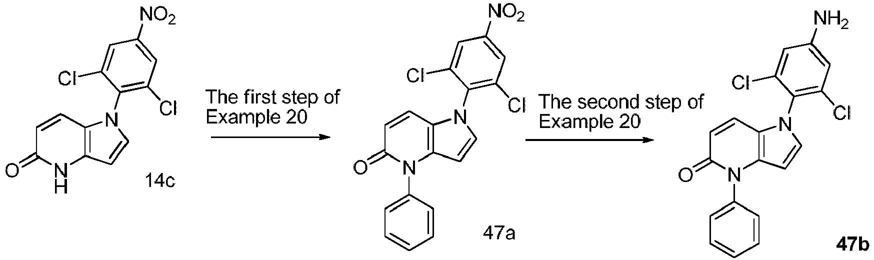

The target **47b** was prepared by using the synthetic route of Example 20, wherein the cyclopropylboronic acid in the first step was replaced by phenylboronic acid, and the remaining steps and conditions were carried out as described in Example 20. LC-MS: [M+H]⁺: 371.

### Example 48: preparation of ZB-H-57

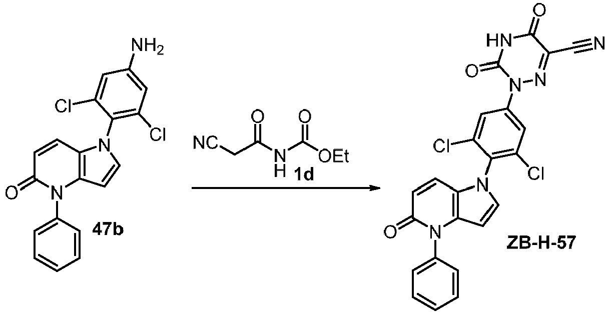

The target **ZB-H-57** was prepared by using the synthetic route of the third step and the fourth step in Example 1, except that the raw material **1c** in the third step was replaced by **47b.** LC-MS: [M+H]⁺: 492.

### Example 49: preparation of ZB-H-58

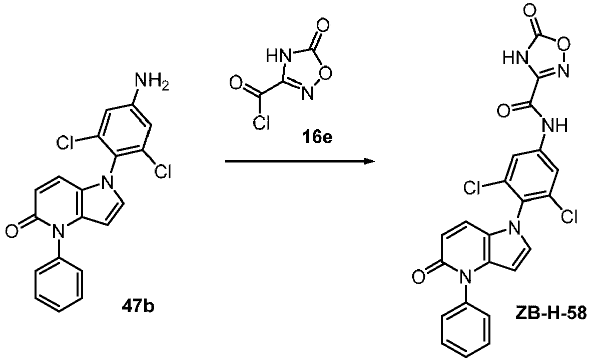

The target **ZB-H-58** was prepared by using the synthetic route of Example 19, except that **15e** was replaced by **47b.** LC-MS [M+H]⁺: 483.

### Example 50: preparation of 50e

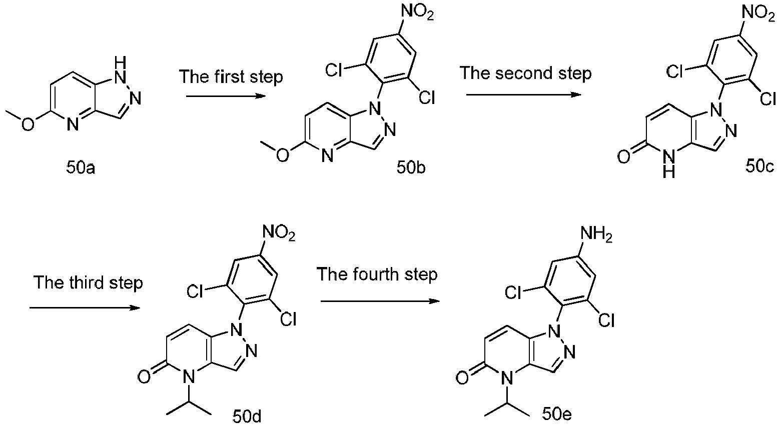

The target **50e** was prepared by using the synthetic route of Example 14, wherein the raw material **11a** in the first step was replaced by **50a,** the raw material iodomethane in the third step was replaced by iodoisopropane, and the remaining steps and conditions were carried out as described in Example 14. LC-MS [M+H]⁺: 338.

### Example 51: preparation of ZB-H-35

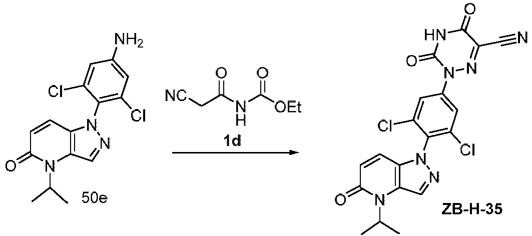

The target **ZB-H-35** was prepared by using the synthetic route of the third step and the fourth step in Example 1, except that the raw material 1c in the third step was replaced by **50e.** ¹H NMR (400 MHz, DMSO-*d*₆) δ 13.38 (s, 1H), 8.42 (s, 1H), 7.93 (s, 2H), 7.64 (d, *J* = 9.6 Hz, 1H), 6.48 (d, *J =* 9.6 Hz, 1H), 5.42 - 5.13 (m, 1H), 1.51 (d, *J =* 7.0 Hz, 6H); LC-MS [M+H]⁺: 459.

### Example 52: preparation of ZB-H-36

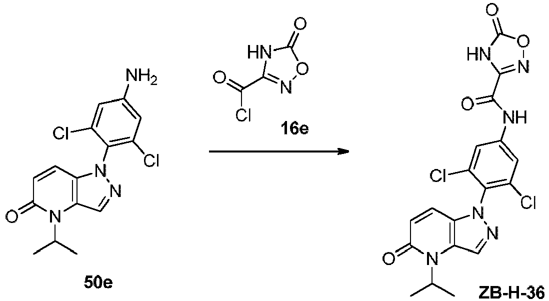

The target **ZB-H-36** was prepared by using the synthetic route of Example 19, except that **15e** was replaced by **50e.** LC-MS [M+H]⁺: 450.

### Example 53: preparation of ZB-H-59

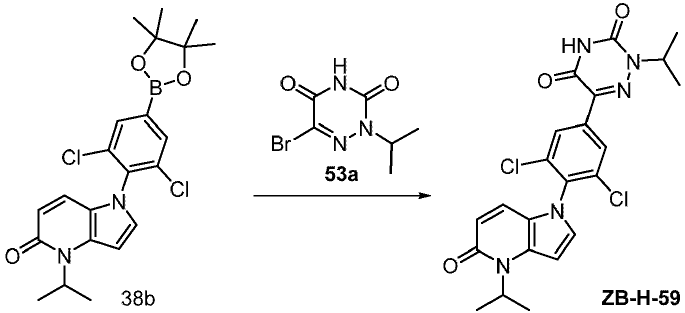

**ZB-H-59** was prepared by using the synthetic route of Example 38, except that the raw material **38c** in the third step was replaced by **53a.** LC-MS [M+H]⁺: 475.

### Example 54: preparation of ZB-H-76

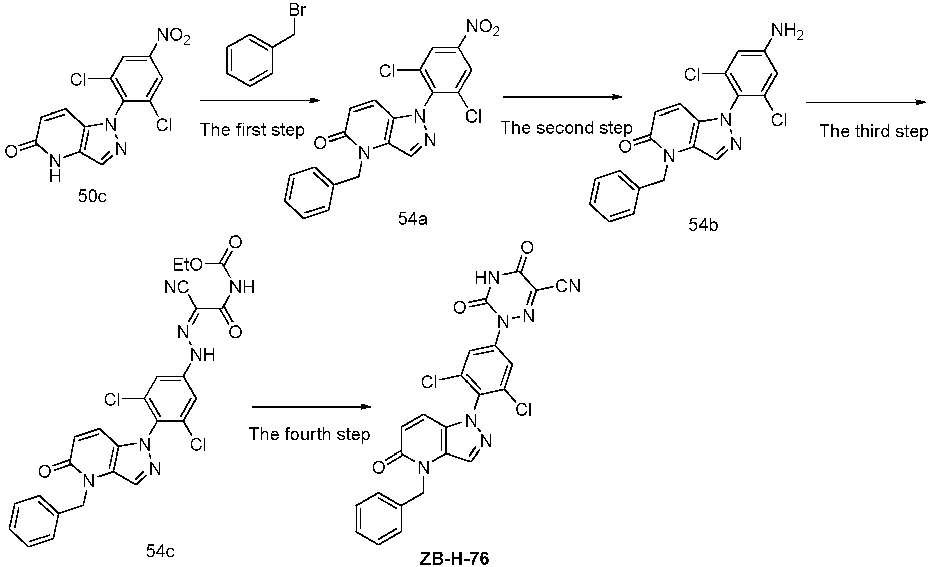

### The first step: preparation of 54a

**50c** (500 mg, 1.54 mmol) was dissolved in 1,4-dioxane (10 mL) and put in a microwave reaction tube, added with benzyl bromide (800 mg, 4.63 mmol) and potassium carbonate (640 mg, 4.63 mmol) respectively, and microwaved at 150°C for 12 hours. After the reaction was finished, the reaction solution was concentrated to dryness under reduced pressure, and purified by column chromatography to obtain **54a** (1.2 g). ¹H NMR (600 MHz, Chloroform-*d*) δ 8.38 (s, 2H), 7.78 (d, *J=* 1.0 Hz, 1H), 7.42 - 7.38 (m, 2H), 7.38 - 7.34 (m, 2H), 7.33 - 7.29 (m, 1H), 7.18 (dd, *J =* 9.6, 0.9 Hz, 1H), 6.73 (d, *J =* 9.6 Hz, 1H), 5.37 (s, 2H). LC-MS: [M+H]⁺:416.

### The second step: preparation of 54b

To a solution of **54a** (1 g, 2.41 mmol) in ethanol (20 mL), stannous chloride dihydrate (2.7 g, 12.0 mmol) was added. After the addition was completed, the reaction solution was raised to 80°C and stirred for 6 hours. After the reaction was stopped, the reaction solution was cooled to room temperature naturally, concentrated to dryness under reduced pressure, and added with ethyl acetate (200 mL) to dissolve. The organic phase was washed with sodium hydroxide aqueous solution (2M) three times, concentrated, and then subjected to column chromatography to obtain **54b,** which was directly used in the next step.

### The third step: preparation of 54c

Tert-butyl nitrite (53 mg, 0.514 mmol) was dissolved in acetic acid (1 ml). This solution was slowly added to a solution of **54b** (180 mg, 0.47 mmol) in acetic acid (10 ml) and acetonitrile (10 mL) at 0°C, stirred at 0°C for 30 minutes, added dropwise with an acetonitrile solution of N-cyanoacetylurethane (80 mg, 0.514 mmol), and stirred for 3 hours after the dropwise addition. After the reaction was complete, the reaction solution was added to saturated aqueous sodium bicarbonate solution (70 ml), and a red solid was filtered out, washed with water and petroleum ether, and dried. The product was directly used in the next step. ¹H NMR (400 MHz, DMSO-*d*₆) δ 12.26 (s, 1H), 11.02 (s, 1H), 8.20 (s, 1H), 8.12 (s, 2H), 7.59 (d, *J* = 9.6 Hz, 1H), 7.43 (d, *J* = 7.2 Hz, 2H), 7.36 (t, *J* = 7.5 Hz, 2H), 7.33 - 7.24 (m, 1H), 6.59 (d, *J =* 9.6 Hz, 1H), 5.32 (s, 2H), 4.21 (q, *J =* 7.1 Hz, 2H), 1.27 (t, *J =* 7.1 Hz, 3H).LC-MS [M+H]⁺: 553.

### The fourth step: preparation of ZB-H-76

To the solid of **54c** (1.0 mmol), N,N-dimethylacetamide (5 mL) and potassium acetate (2.0 mmol) was added, heat to 120°C and stirred for 6 hours. After the reaction was completed, the reaction mixture was directly subjected to preparative chromatography to purify to obtain **ZB-H-76.** ¹H NMR (400 MHz, Methanol-*d*₄) δ 8.07 (d, *J* = 0.9 Hz, 1H), 7.97 (s, 2H), 7.61 (dd, *J* = 9.6, 0.9 Hz, 1H), 7.47 - 7.32 (m, 5H), 6.74 (d, *J* = 9.5 Hz, 1H), 5.47 (s, 2H). LC-MS [M+H]⁺: 507.

### Example 55: preparation of ZB-H-60

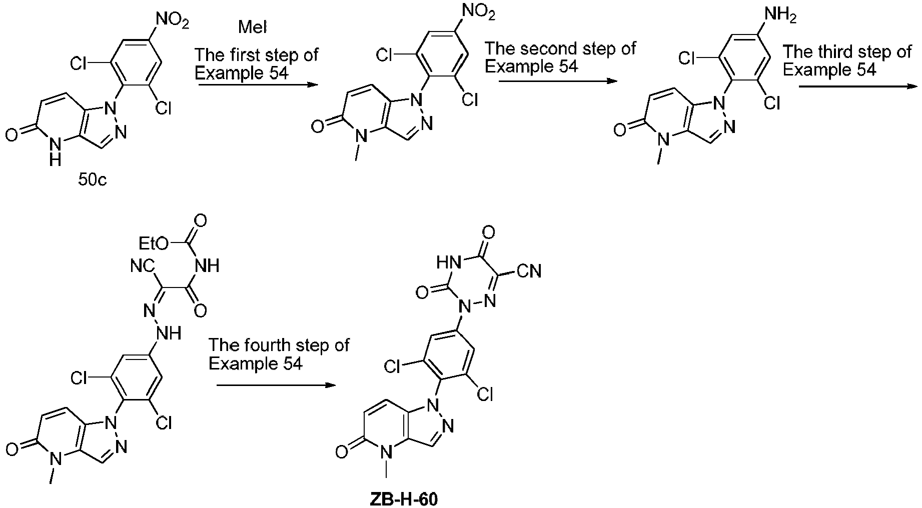

**ZB-H-60** was prepared by referring to the synthetic route of Example 54, except that the benzyl bromide in the first step of Example 54 was replaced with methyl iodide (Mel). LC-MS [M+H]⁺: 431.

### Example 56: preparation of ZB-H-61

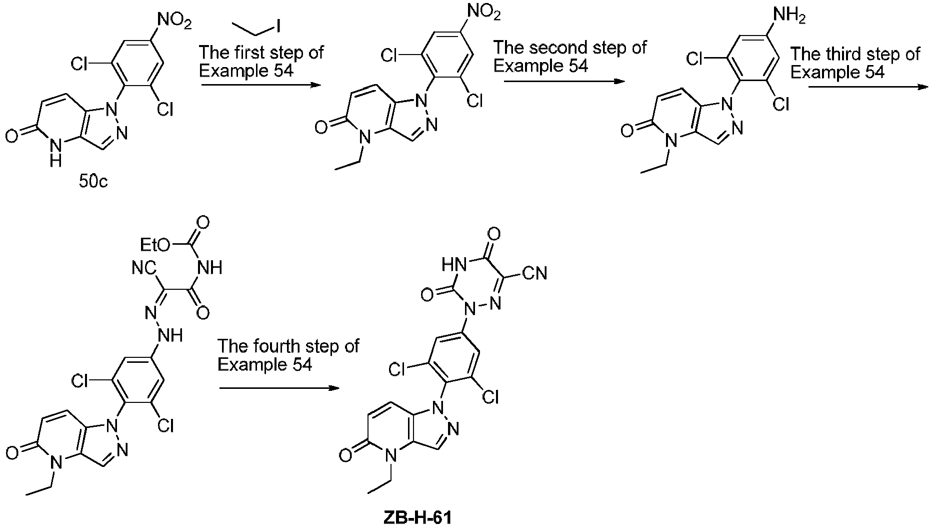

**ZB-H-61** was prepared by referring to the synthetic route of Example 54, except that the benzyl bromide in the first step of Example 54 was replaced with ethyl iodide. LC-MS [M+H]⁺: 445.

### Example 57: preparation of ZB-H-62

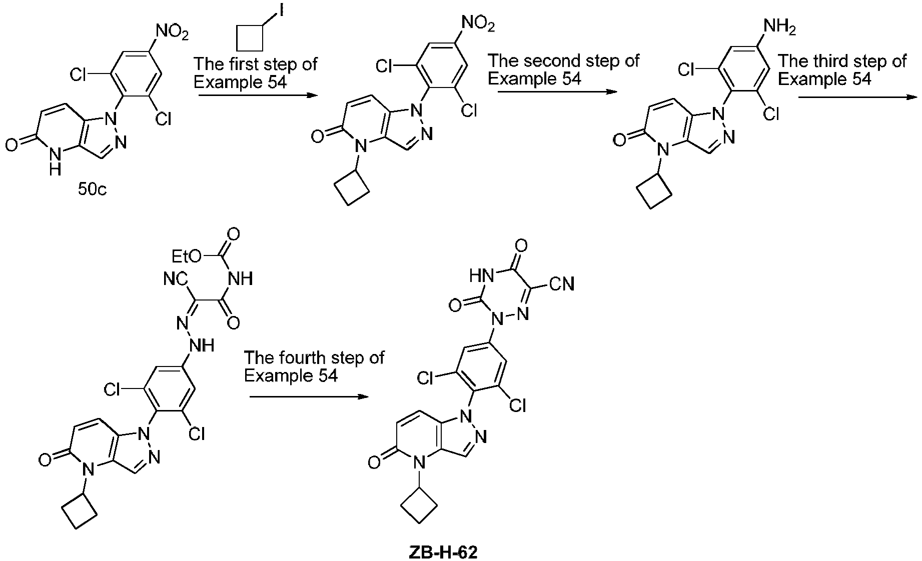

**ZB-H-62** was prepared by referring to the synthetic route of Example 54, except that the benzyl bromide in the first step of Example 54 was replaced with iodocyclobutane. LC-MS [M+H]⁺: 472.

### Example 58: preparation of ZB-H-63

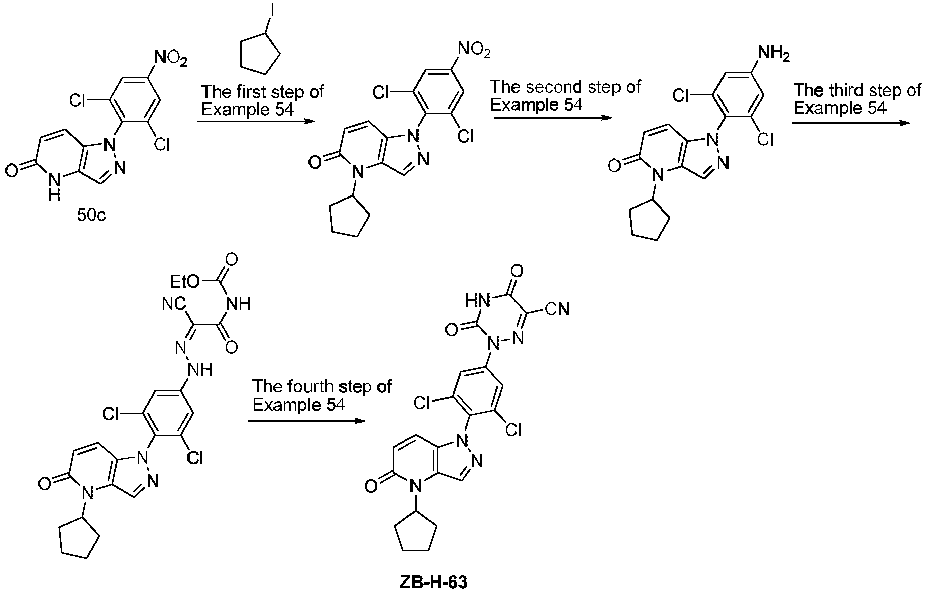

**ZB-H-63** was prepared by referring to the synthetic route of Example 54, except that the benzyl bromide in the first step of Example 54 was replaced with iodocyclopentane. LC-MS [M+H]⁺: 485.

### Example 59: preparation of ZB-H-64

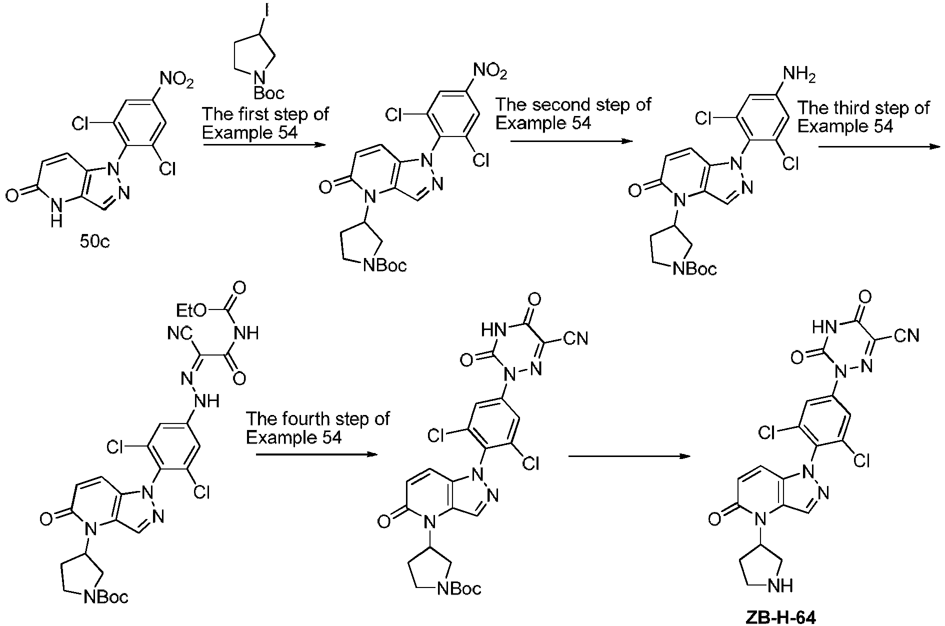

**ZB-H-64** was prepared by referring to the synthetic route of Example 54, except that the benzyl bromide in the first step of Example 54 was replaced with 1-Boc-3-iodopyrrolidine, and the Boc protecting group was further removed. LC-MS [M+H]⁺: 485.

### Example 60: preparation of ZB-H-65

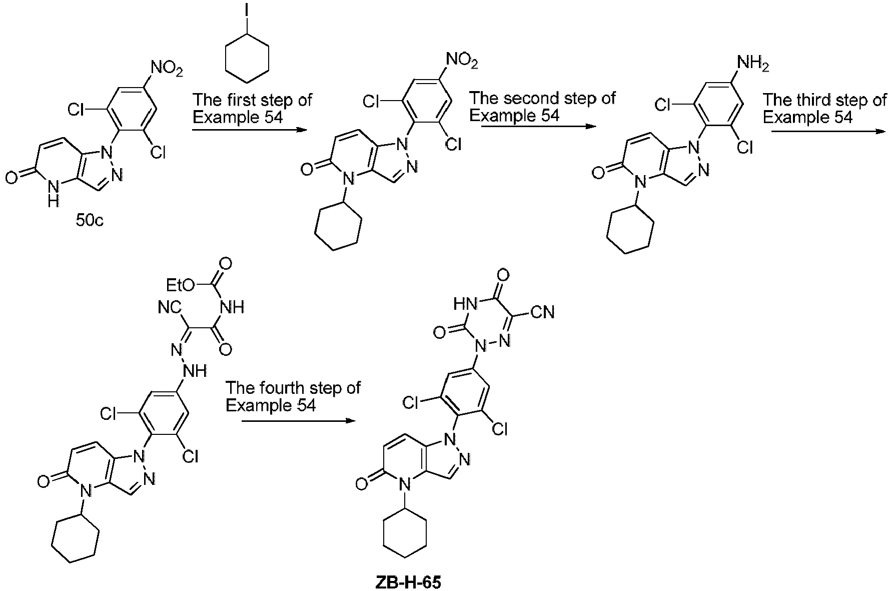

**ZB-H-65** was prepared by referring to the synthetic route of Example 54, except that the benzyl bromide in the first step of Example 54 was replaced with iodocyclohexane. LC-MS [M+H]⁺: 499.

### Example 61: preparation of ZB-H-66

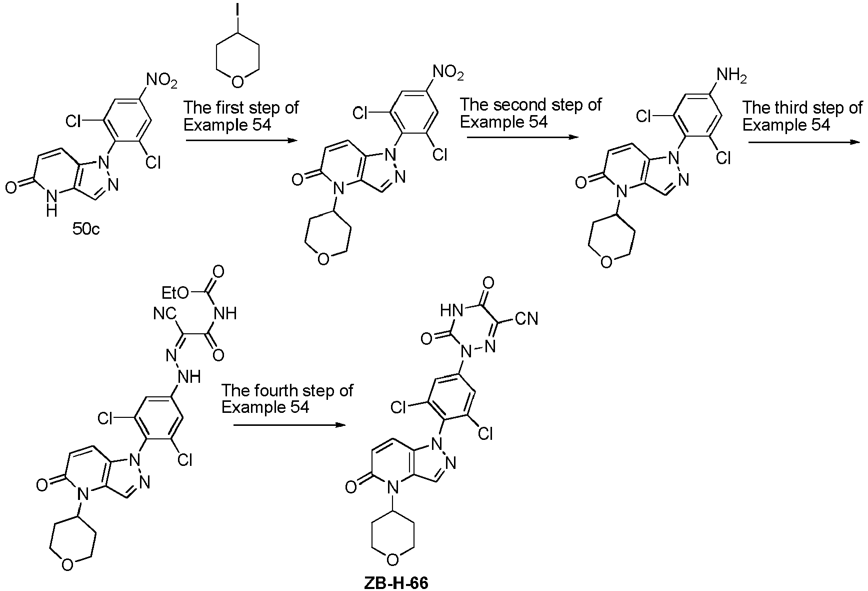

**ZB-H-66** was prepared by referring to the synthetic route of Example 54, except that the benzyl bromide in the first step of Example 54 was replaced with 4-iodotetrahydropyran. LC-MS [M+H]⁺: 501.

### Example 62: preparation of ZB-H-67

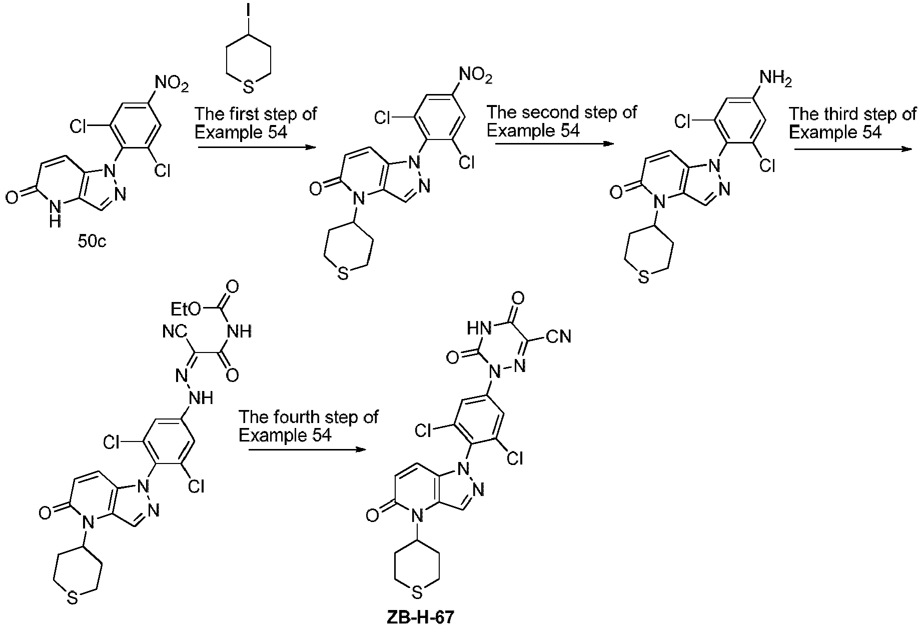

**ZB-H-67** was prepared by referring to the synthetic route of Example 54, except that the benzyl bromide in the first step of Example 54 was replaced with tetrahydro-4-iodo-2H-thiopyran. LC-MS [M+H]⁺: 517.

### Example 63: preparation of ZB-H-68

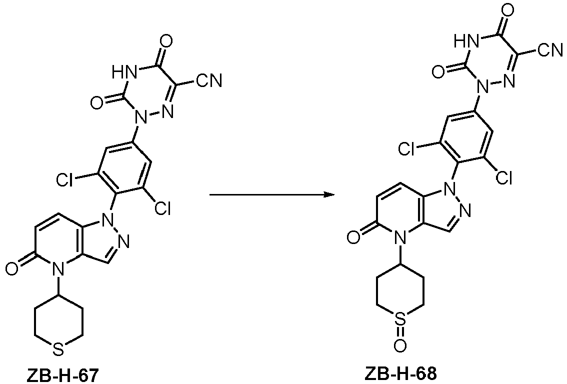

**ZB-H-67** (1.0 mmol) was dissolved in dichloromethane (5 mL), added dropwise with a solution of m-chloroperoxybenzoic acid (0.9 mmol) in dichloromethane (5 mL) under ice bath, reacted under the ice bath for 30 minutes, quenched with saturated sodium bicarbonate solution after the reaction was completed, and extracted with dichloromethane three times. The organic phases were combined, rotarily evaporated to dryness, and purified by column chromatography to obtain **ZB-H-68.** LC-MS [M+H]⁺: 533.

### Example 64: preparation of ZB-H-69

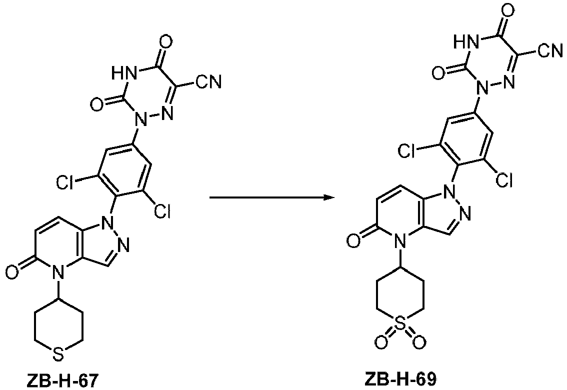

**ZB-H-67** (1.0 mmol) was dissolved in dichloromethane (5 mL), added dropwise with a solution of m-chloroperoxybenzoic acid (2.0 mmol) in dichloromethane (5 mL) under ice bath, reacted under the ice bath for 30 minutes, quenched with saturated sodium bicarbonate solution after the reaction was completed, and extracted with dichloromethane three times. The organic phases were combined, rotarily evaporated to dryness, and purified by column chromatography to obtain **ZB-H-69.** LC-MS [M+H]⁺: 549.

### Example 65: preparation of ZB-H-70

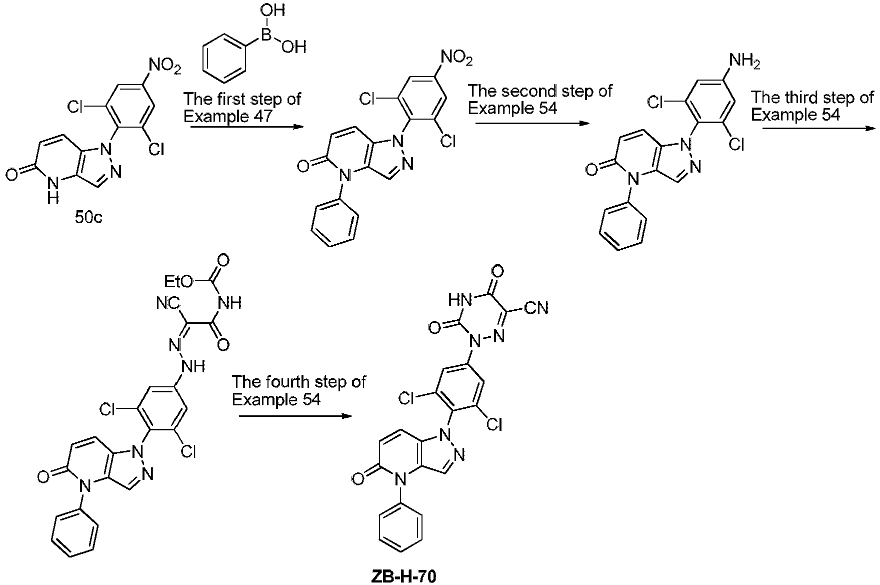

**ZB-H-70** was prepared by referring to the synthetic route of Example 47 and Example 54. LC-MS [M+H]⁺: 593.

### Example 66: preparation of ZB-H-71

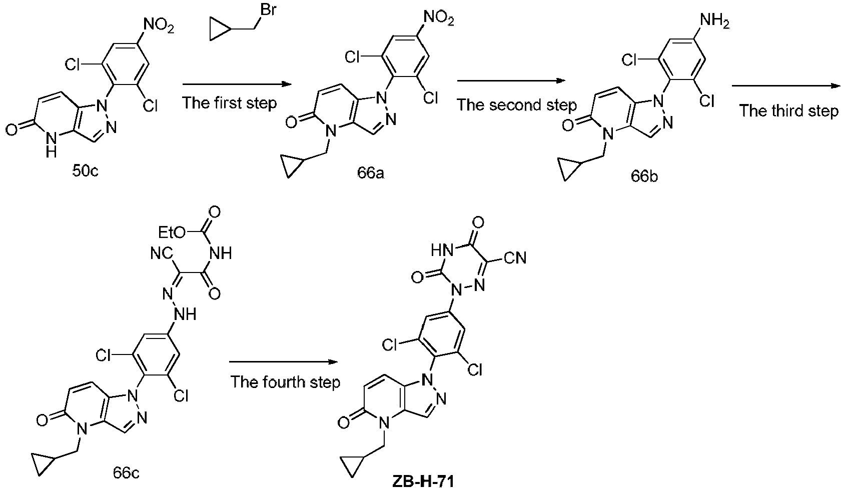

### The first step: preparation of 66a

**50c** (200 mg, 0.615 mmol) was dissolved in 1,4-dioxane (10 mL) and put in a microwave reaction tube, added with bromomethylcyclopropane (250 mg, 1.845 mmol) and potassium carbonate (170 mg, 1.231 mmol) respectively, and microwaved at 150°C for 12 hours. After the reaction was finished, the reaction solution was concentrated to dryness under reduced pressure, and purified by column chromatography to obtain **66a.** ¹H NMR (400 MHz, Chloroform-*d*) δ 8.37 (s, 2H), 7.88 (s, 1H), 7.16 (d, *J=* 9.6 Hz, 1H), 6.67 (d, *J=* 9.6 Hz, 1H), 4.04 (d, *J=* 7.1 Hz, 2H), 2.02 (m, 1H), 1.30 - 1.15 (m, 4H). LC-MS: [M+H]⁺: 379.

### The second step: preparation of 66b

To a solution of **66a** (80 mg, 0.212 mmol) in ethanol (10 mL), stannous chloride dihydrate (238 mg, 1.058 mmol) was added. After the addition was completed, the reaction solution was raised to 80°C and stirred for 6 hours. After the reaction was stopped, the reaction solution was cooled to room temperature naturally, concentrated to dryness under reduced pressure, and added with ethyl acetate (10 mL) to dissolve. The organic phase was washed with sodium hydroxide aqueous solution (2M, 10 mL) three times and concentrated, and then subjected to column chromatography to obtain **66b,** which was directly used in the next step. ¹H NMR (400 MHz, Methanol-*d*₄) δ 8.01 (s, 1H), 7.39 (d, *J* = 9.5 Hz, 1H), 6.74 (s, 2H), 6.58 (d, *J* = 9.5 Hz, 1H), 4.07 (d, *J* = 7.1 Hz, 2H), 1.95 (m, 1H), 0.57 - 0.46 (m, 4H). LC-MS [M+H]⁺: 350.

### The third step: preparation of 66c

Tert-butyl nitrite (16.2 mg, 0.1573 mmol) was dissolved in acetic acid (2 mL). This solution was slowly added to a solution of **66b** (50 mg, 0.143 mmol) in acetic acid (2 ml) and acetonitrile (2 mL) at 0°C, stirred at 0°C for 30 minutes, added dropwise with a solution of N-cyanoacetylurethane (25 mg, 01573 mmol) in acetonitrile (2 mL), and stirred for 3 hours after the dropwise addition. After the reaction was complete, the reaction solution was added to a saturated aqueous sodium bicarbonate solution (20 mL), and a red solid was filtered out, washed with water (10 mL) and petroleum ether (10 mL), and dried. The product was directly used in the next step. ¹H NMR (400 MHz, DMSO-*d*₆) δ 12.28 (s, 1H), 11.02 (s, 1H), 8.25 (d, *J* = 0.9 Hz, 1H), 8.13 (s, 2H), 7.54 (dd, *J* = 9.6, 0.9 Hz, 1H), 6.51 (d, *J* = 9.6 Hz, 1H), 4.21 (q, *J=* 7.1 Hz, 2H), 3.99 (d, *J=* 7.1 Hz, 2H), 1.27 (t, *J* = 7.1 Hz, 3H), 1.23 (m, 1H), 0.52 - 0.44 (m, 4H). LC-MS [M+H]⁺: 517.

### The fourth step: preparation of ZB-H-71

To the solid of **66c** (81 mg, 0.1571 mmol), N,N-dimethylacetamide (5 mL) and potassium acetate (17 mg, 0.1716 mmol) was added, heat to 120°C and stirred for 6 hours. After the reaction was completed, the reaction mixture was directly subjected to preparative chromatography to purify to obtain the **ZB-H-71.** ¹H NMR (400 MHz, Methanol-d4) δ 8.19 (d, J = 0.9 Hz, 1H), 7.96 (s, 2H), 7.56 (dd, J = 9.6, 0.9 Hz, 1H), 6.66 (d, J = 9.5 Hz, 1H), 4.14 (d, J = 7.1 Hz, 2H), 1.48 - 1.36 (m, 1H), 0.64 - 0.49 (m, 4H). LC-MS [M+H]⁺: 471.

### Example 67: preparation of ZB-H-72

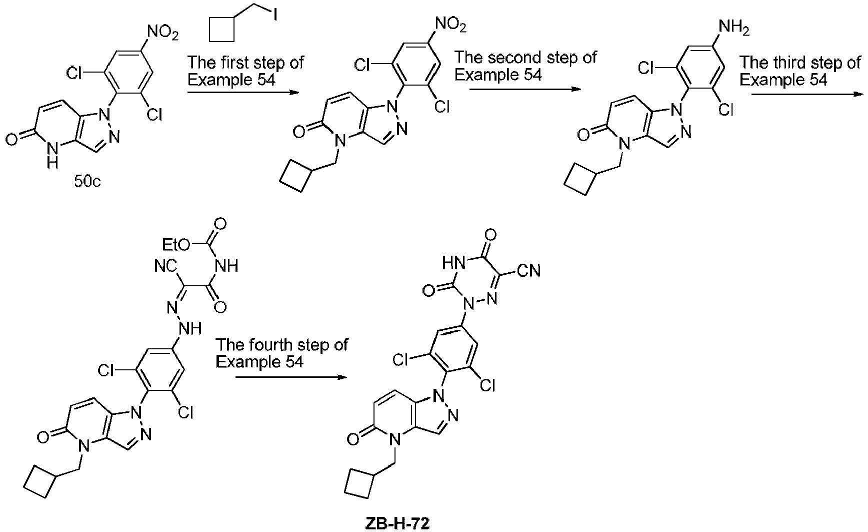

**ZB-H-72** was prepared by referring to the synthetic route of Example 54, except that the benzyl bromide in the first step of Example 54 was replaced with (iodomethyl)cyclobutane. LC-MS [M+H]⁺: 485.

### Example 68: preparation of ZB-H-73

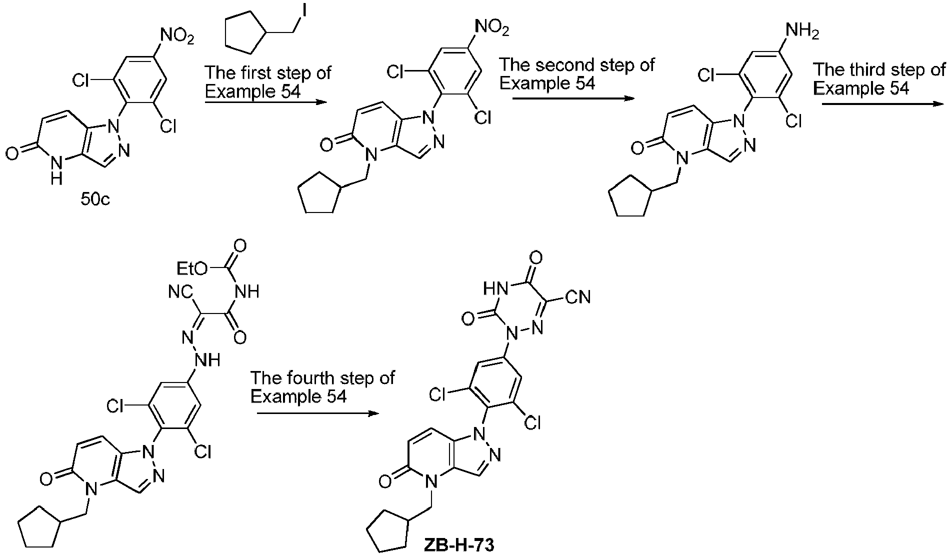

**ZB-H-73** was prepared by referring to the synthetic route of Example 54, except that the benzyl bromide in the first step of Example 54 was replaced with (iodomethyl)cyclopentane. LC-MS [M+H]⁺: 489.

### Example 69: preparation of ZB-H-74

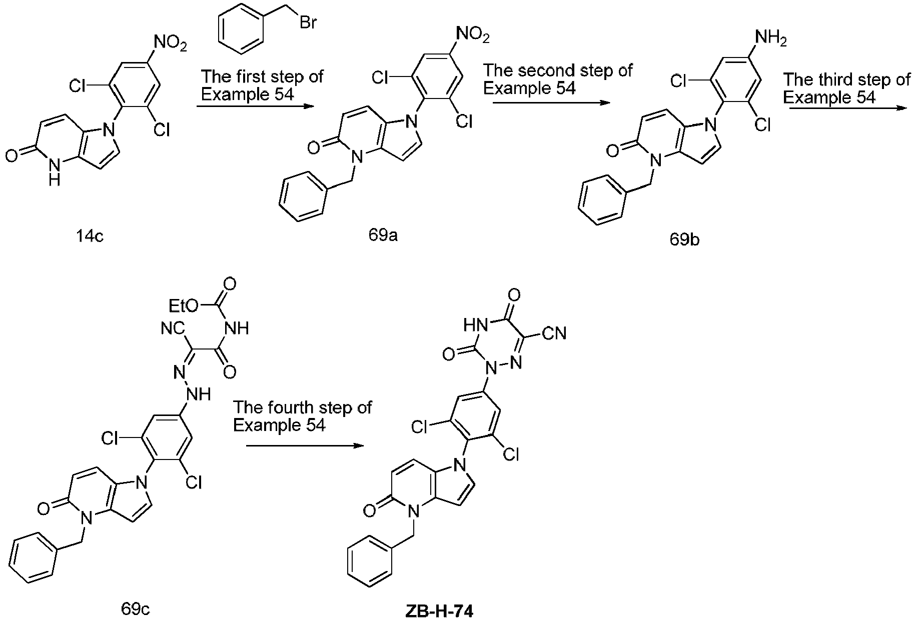

### The first step: synthesis of 69a

**69a** was prepared by referring to the first step of the synthetic route of Example 54, except that **50c** was replaced with **14c.** ¹H NMR (400 MHz, Chloroform-*d*) δ 8.38 (s, 2H), 7.40 - 7.27 (m, 5H), 7.03 (dd, *J* = 9.6, 0.8 Hz, 1H), 6.92 (d, *J* = 3.3 Hz, 1H), 6.49 (d, *J* = 9.5 Hz, 1H), 6.37 (dd, *J=* 3.3, 0.8 Hz, 1H), 5.41 (s, 2H).LC-MS [M+H]⁺: 415.

### The second step: synthesis of 69b

**69b** was obtained by referring to the second step of the synthetic route of Example 54. ¹H NMR (400 MHz, DMSO-*d*₆) δ 7.42 - 7.37 (m, 2H), 7.33 (t, *J* = 7.4 Hz, 2H), 7.28 - 7.22 (m, 2H), 7.18 (d, *J* = 9.4 Hz, 1H), 6.75 (s, 2H), 6.46 (d, *J* = 3.2 Hz, 1H), 6.18 (d, *J* = 9.4 Hz, 1H), 6.08 (s, 2H), 5.28 (s, 2H).LC-MS [M+H]⁺: 385.

### The third step: synthesis of 69c

**69c** was obtained by referring to the third step of the synthetic route of Example 54. LC-MS [M+H]⁺: 552.

### The fourth step: synthesis of ZB-H-74

**ZB-H-74** was obtained by referring to the fourth step of the synthetic route of Example 54. ¹H NMR (400 MHz, Methanol-*d*₄) δ 7.92 (s, 2H), 7.40 - 7.28 (m, 7H), 6.56 (dd, *J* = 3.3, 0.8 Hz, 1H), 6.45 (d, *J=* 9.4 Hz, 1H), 5.48 (s, 2H)..LC-MS [M+H]⁺:506.

### Example 70: preparation of ZB-H-75

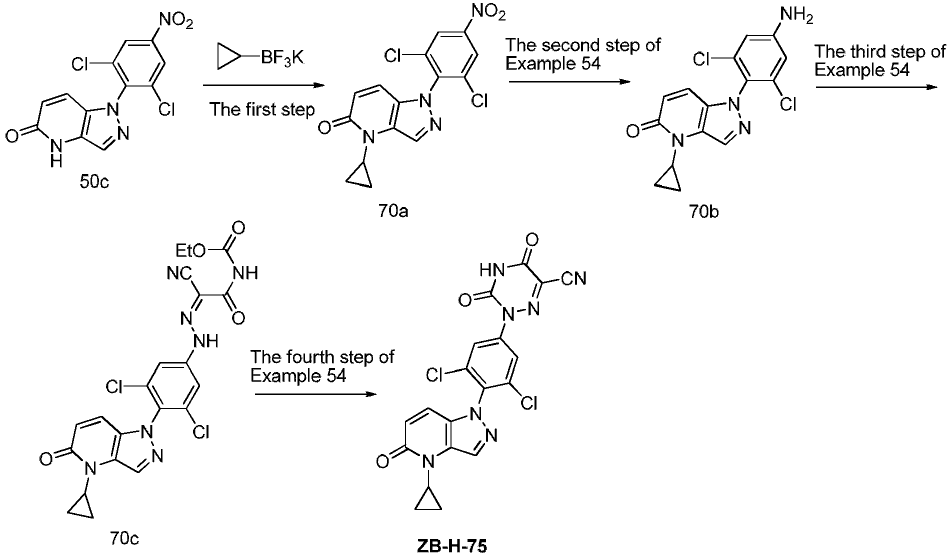

### The first step: synthesis of 70a

**50c** (500 mg, 1.54 mmol) was dissolved in toluene (20 mL), added with potassium cyclopropyltrifluoroborate (4.62 mmol), copper acetate (0.385 mmol), potassium carbonate (3.08 mmol), 1,10-phenanthroline (0.1925 mmol) and water (2 mL) respectively, purged with oxygen three times, and reacted at 80°C overnight. After the reaction was completed, the reaction solution was concentrated to dryness under reduced pressure, and purified by column chromatography to obtain **70a.** ¹H NMR (400 MHz, Chloroform-*d*) δ 8.42 (s, 2H), 8.07 (d, *J* = 0.9 Hz, 1H), 7.14 (dd, *J* = 9.7, 0.9 Hz, 1H), 6.62 (d, *J* = 9.6 Hz, 1H), 3.20 (tt, *J* = 7.3, 4.0 Hz, 1H), 1.37 - 1.30 (m, 2H), 1.14 - 1.08 (m, 2H).LC-MS [M+H]⁺: 366.

### The second step: synthesis of 70b

**70b** was obtained by referring to the second step of the synthetic route of Example 54. ¹H NMR (600 MHz, DMSO-*d*₆) δ 8.00 (d, *J =* 0.9 Hz, 1H), 7.38 (dd, *J =* 9.5, 0.9 Hz, 1H), 6.76 (s, 2H), 6.38 (d, *J =* 9.5 Hz, 1H), 6.27 - 6.03 (br.s, 2H), 3.10 (tt, *J* = 7.3, 4.0 Hz, 1H), 1.16 (td, *J* = 7.4, 5.6 Hz, 2H), 0.93 - 0.87 (m, 2H).LC-MS [M+H]⁺: 336.

### The third step: synthesis of 70c

**70c** was obtained by referring to the third step of the synthetic route of Example 54. ¹H NMR (400 MHz, DMSO-*d*₆) δ 12.27 (s, 1H), 11.00 (s, 1H), 8.12 (s, *J* = 1.9 Hz, 2H), 8.10 (m, 1H), 7.49 (d, *J=* 9.6 Hz, 1H), 6.43 (d, *J* = 9.6 Hz, 1H), 4.21 (q, *J* = 7.1 Hz, 2H), 3.12 (tt, *J* = 7.2, 3.9 Hz, 1H), 1.27 (t, *J =* 7.1 Hz, 3H), 1.23 - 1.13 (m, 2H), 0.94 - 0.86 (m, 2H).LC-MS [M+H]⁺: 503.

### The fourth step: synthesis of ZB-H-75

**ZB-H-75** was obtained by referring to the fourth step of the synthetic route of Example 54. ¹H NMR (500 MHz, DMSO-*d*₆) δ 8.18 (d, *J* = 0.9 Hz, 1H), 7.93 (s, 2H), 7.63 (d, *J* = 9.6 Hz, 1H), 6.44 (d, *J* = 9.6 Hz, 1H), 3.13 (td, *J =* 7.1, 3.7 Hz, 1H), 1.18 (td, *J* = 7.4, 5.6 Hz, 2H), 0.95 - 0.88 (m, 2H).LC-MS [M+H]⁺: 457.

### Example 71: preparation of ZB-H-77

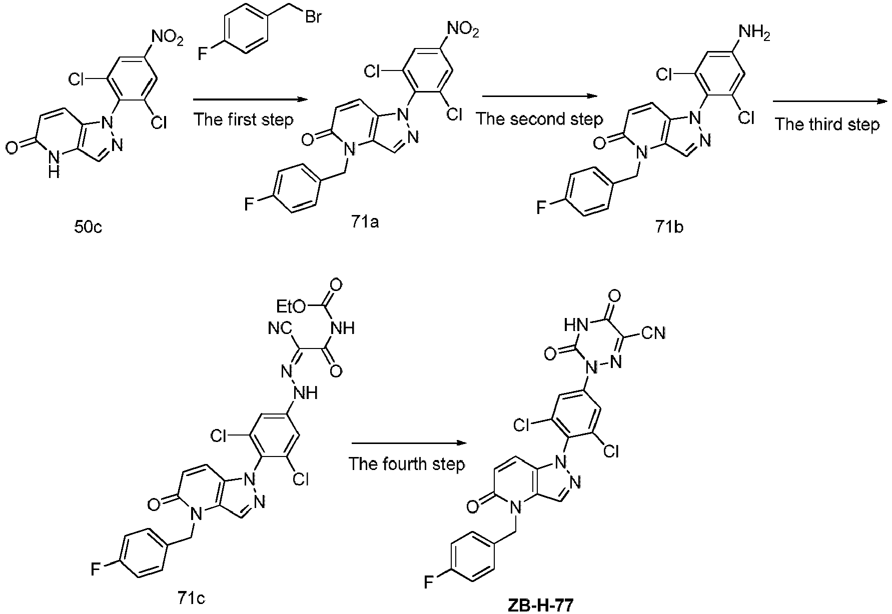

### The first step: preparation of 71a

**50c** (200 mg, 0.615 mmol) was dissolved in 1,4-dioxane (10 mL) and put in a microwave reaction tube, added with 4-fluorobenzyl bromide (233 mg, 1.231 mmol) and potassium carbonate (170 mg, 1.231 mmol) respectively, and microwaved at 150°C for 12 hours. After the reaction was finished, the reaction solution was concentrated to dryness under reduced pressure, and purified by column chromatography to obtain 71a. ¹H NMR (400 MHz, Methanol-*d*₄) δ 6.98 (s, 2H), 6.57 (d, *J* = 0.9 Hz, 1H), 6.04 (dd, *J* = 9.5, 0.9 Hz, 1H), 5.92 - 5.86 (m, 2H), 5.57 - 5.49 (m, 2H), 5.14 (d, *J=* 9.5 Hz, 1H), 3.85 (s, 2H). LC-MS: [M+H]⁺: 434.

### The second step: preparation of 77b

To a solution of **77a** (92 mg, 0.212 mmol) in ethanol (10 mL), stannous chloride dihydrate (238 mg, 1.058 mmol) was added. After the addition was completed, the reaction solution was raised to 80°C and stirred for 6 hours. After the reaction was stopped, the reaction solution was cooled to room temperature naturally, concentrated to dryness under reduced pressure, and added with ethyl acetate (10 mL) to dissolve. The organic phase was washed with sodium hydroxide aqueous solution (2M, 10 mL) three times, concentrated, and then subjected to column chromatography to obtain **71b,** which was directly used in the next step. LC-MS [M+H]⁺: 404.

### The third step: preparation of 71c

Tert-butyl nitrite (16.2 mg, 0.1573 mmol) was dissolved in acetic acid (2 mL). This solution was slowly added to a solution of **71b** (58 mg, 0.143 mmol) in acetic acid (2 ml) and acetonitrile (2 mL) at 0°C, stirred at 0°C for 30 minutes, added dropwise with a solution of N-cyanoacetylurethane (25 mg, 01573 mmol) in acetonitrile (2 mL), and stirred for 3 hours after the dropwise addition. After the reaction was complete, the reaction solution was added to a saturated aqueous sodium bicarbonate solution (20 mL), and a red solid was filtered out, washed with water (10 mL) and petroleum ether (10 mL), and dried. The product was directly used in the next step. ¹H NMR (400 MHz, DMSO-*d*₆) δ 12.27 (s, 1H), 11.01 (s, 1H), 8.25 (d, *J* = 0.9 Hz, 1H), 8.12 (s, 2H), 7.59 (d, *J* = 9.6 Hz, 1H), 7.56 - 7.47 (m, 2H), 7.23 - 7.16 (m, 2H), 6.58 (d, *J=* 9.6 Hz, 1H), 5.30 (s, 2H), 4.21 (q, *J=* 7.1 Hz, 2H), 1.27 (t, *J=* 7.1 Hz, 3H). LC-MS [M+H]⁺: 571.

### The fourth step: preparation of ZB-H-77

To the solid of 71c (89 mg, 0.157 mmol), N,N-dimethylacetamide (5 mL) and potassium acetate (17 mg, 0.1716 mmol) was added, heat to 120°C and stirred for 6 hours. After the reaction was completed, the reaction solution was directly subjected to preparative chromatography to purify to obtain **ZB-H-77.** ¹H NMR (400 MHz, DMSO-d6) δ 13.36 (s, 1H), 8.32 (s, 1H), 7.93 (s, 2H), 7.74 (d, J = 9.6 Hz, 1H), 7.56 - 7.47 (m, 2H), 7.20 (m, 2H), 6.59 (d, J = 9.6 Hz, 1H), 5.31 (s, 2H).LC-MS [M+H]⁺: 525.

### Example 72: preparation of ZB-H-78

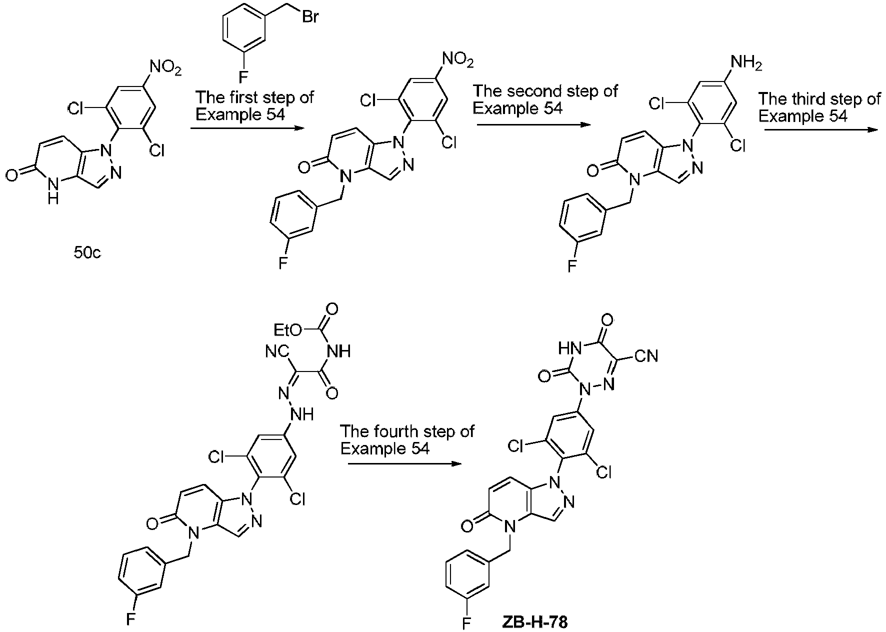

**ZB-H-78** was prepared by referring to the synthetic route of Example 54, except that the benzyl bromide in the first step of Example 54 was replaced with 3-fluorobenzyl bromide. LC-MS [M+H]⁺: 525.

### Example 73: preparation of ZB-H-79

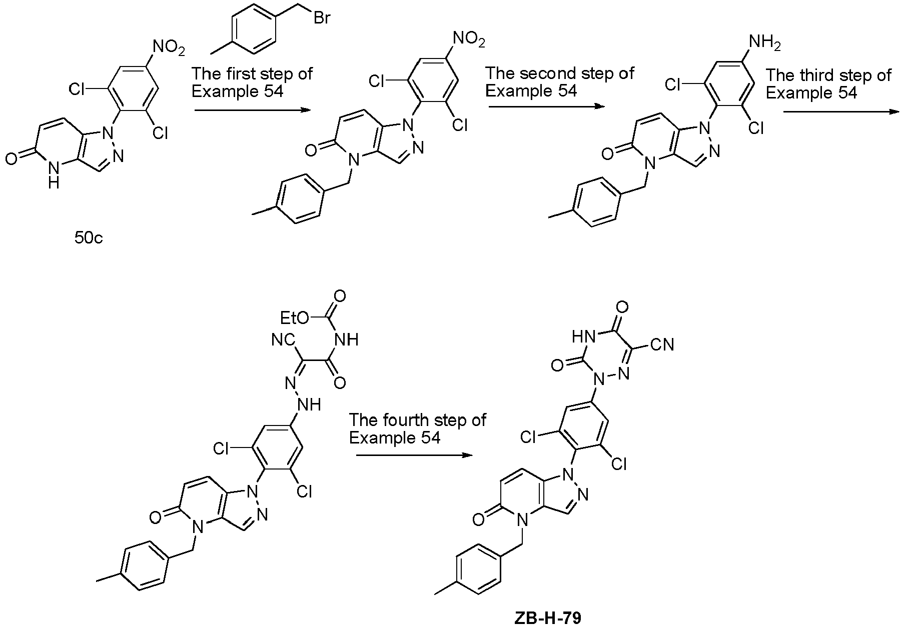

**ZB-H-79** was prepared by referring to the synthetic route of Example 54, except that the benzyl bromide in the first step of Example 54 was replaced with 4-methylbenzyl bromide. LC-MS [M+H]⁺: 521.

### Example 74: preparation of ZB-H-80

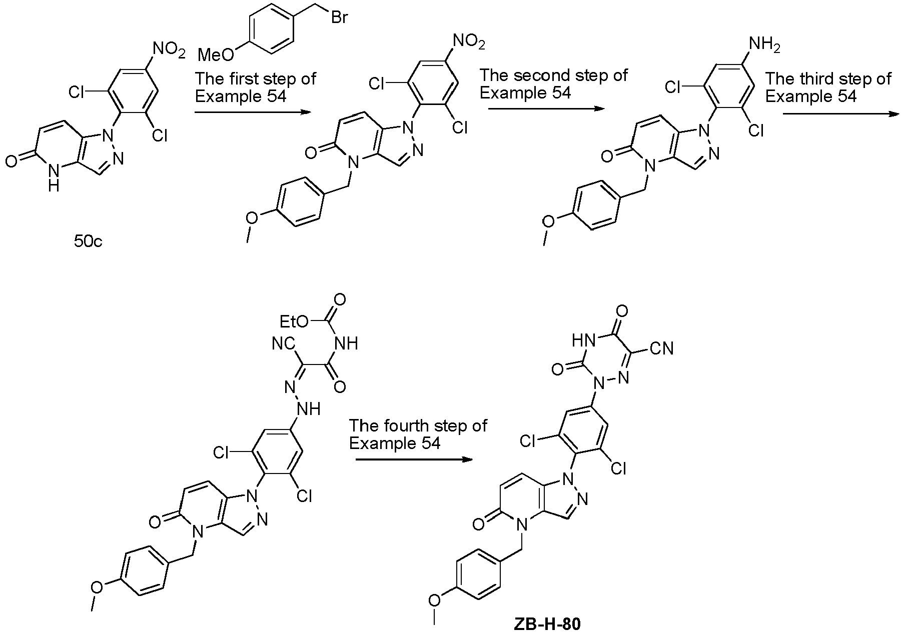

**ZB-H-80e** was prepared by referring to the synthetic route of Example 54, except that the benzyl bromide in the first step of Example 54 was replaced with 4-methoxybenzyl bromide. LC-MS [M+H]⁺: 537.

### Example 75: preparation of ZB-H-81

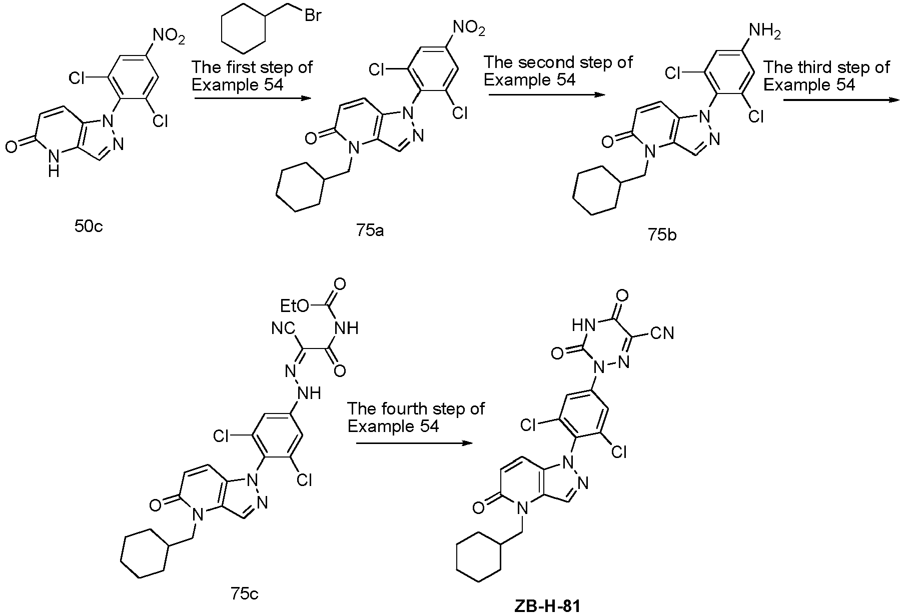

### The first step: synthesis of 75a

**75a** was obtained by referring to the first step of the synthetic route of Example 54, except that the benzyl bromide was replaced with (bromomethyl)cyclohexane. ¹H NMR (600 MHz, Chloroform-*d*) δ 8.36 (s, 2H), 7.82 (s, 1H), 7.13 (d, *J* = 9.6 Hz, 1H), 6.62 (d, *J* = 9.6 Hz, 1H), 3.95 (d, *J* = 7.4 Hz, 2H), 1.97 (dqt, *J* = 10.6, 6.9, 3.2 Hz, 1H), 1.78 - 1.60 (m, 5H), 1.24 - 1.11 (m, 5H).LC-MS [M+H]⁺: 422.

### The second step: synthesis of 75b

**75b** was obtained by referring to the second step of the synthetic route of Example 54. ¹H NMR (400 MHz, Methanol-d₄) δ 7.88 (s, 1H), 7.36 (d, J = 9.6 Hz, 1H), 6.78 (s, 2H), 6.63 (d, J = 9.6 Hz, 1H), 4.03 (d, J = 7.3 Hz, 2H), 2.00 (m, 1H), 1.73 (m, 5H), 1.24 (m, 5H). LC-MS [M+H]⁺: 392.

### The third step: synthesis of 75c

**75c** was obtained by referring to the third step of the synthetic route of Example 54. ¹H NMR (600 MHz, DMSO-*d*₆) δ 12.26 (s, 1H), 11.01 (s, 1H), 8.21 (s, 1H), 8.13 (s, 2H), 7.52 (d, *J* = 9.6 Hz, 1H), 6.49 (d, *J* = 9.6 Hz, 1H), 4.21 (q, *J* = 7.1 Hz, 2H), 3.93 (d, *J* = 7.3 Hz, 2H), 1.90 (m, 1H), 1.71 - 1.57 (m, 5H), 1.27 (t, *J* = 7.1 Hz, 3H), 1.14 (m, 5H). LC-MS [M+H]⁺: 559.

### The fourth step: synthesis of ZB-H-81

**ZB-H-81** was obtained by referring to the fourth step of the synthetic route of Example 54. ¹H NMR (600 MHz, DMSO-*d*₆) δ 13.35 (s, 1H), 8.28 (s, 1H), 7.93 (s, 2H), 7.67 (d, *J* = 9.5 Hz, 1H), 6.51 (d, *J* = 9.5 Hz, 1H), 3.94 (d, *J* = 7.3 Hz, 2H), 1.90 (dtt, *J* = 12.9, 8.9, 4.6 Hz, 1H), 1.71 - 1.58 (m, 5H), 1.19 - 1.07 (m, 5H).LC-MS [M+H]⁺:513.

### Example 76: preparation of ZB-H-82

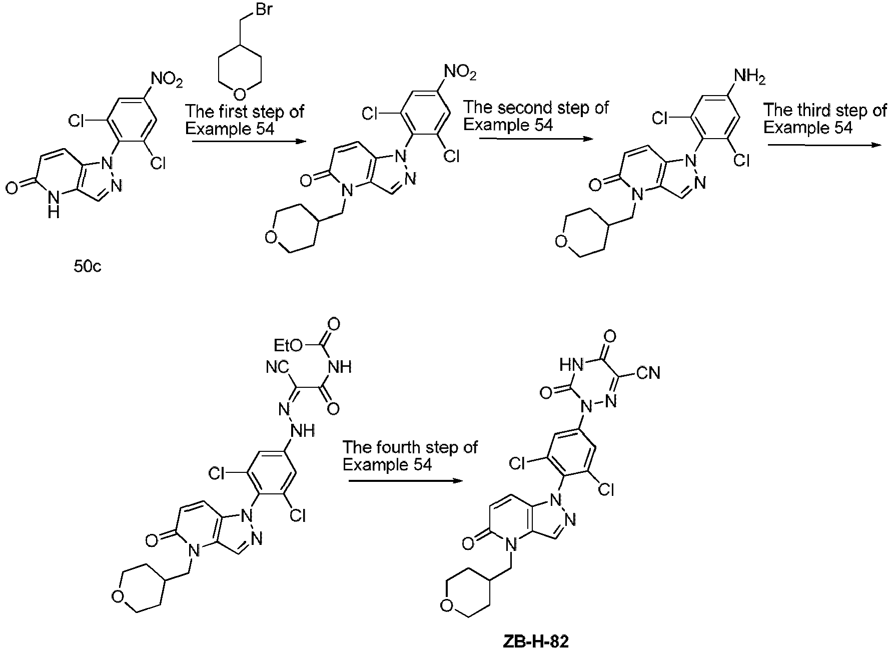

**ZB-H-82** was prepared by referring to the synthetic route of Example 54, except that the benzyl bromide in the first step of Example 54 was replaced with 4-bromomethyltetrahydropyran. LC-MS [M+H]⁺: 515.

### Example 77: preparation of ZB-H-83

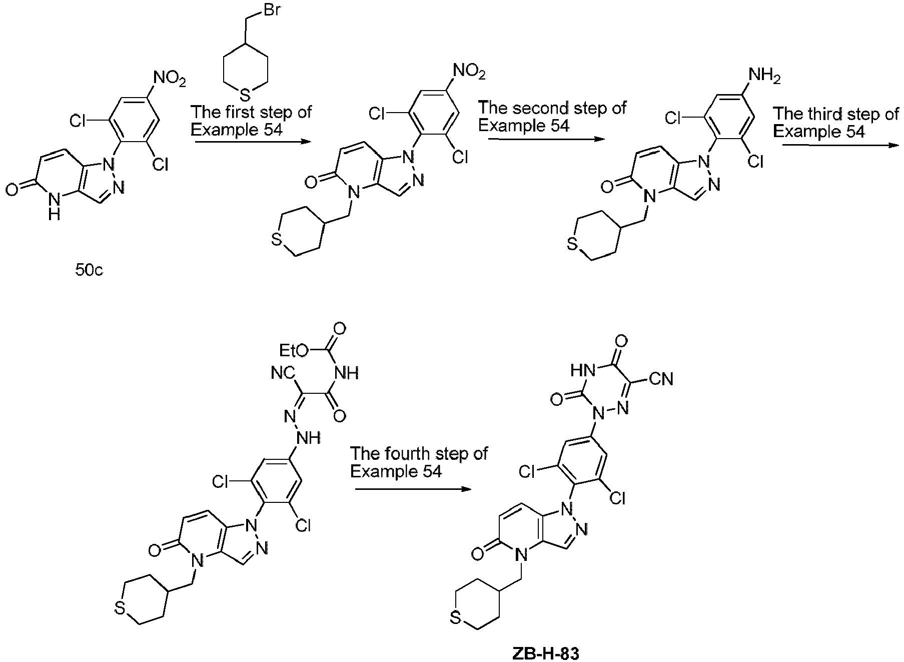

**ZB-H-83** was prepared by referring to the synthetic route of Example 54, except that the benzyl bromide in the first step of Example 54 was replaced with 4-(bromomethyl)-tetrahydro-2H-thiopyran. LC-MS [M+H]⁺: 531.

### Example 78: preparation of ZB-H-84

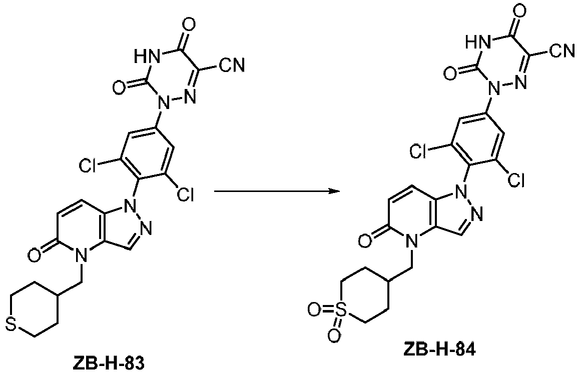

**ZB-H-83** (1.0 mmol) was dissolved in dichloromethane (5 mL), added dropwise with a solution of m-chloroperoxybenzoic acid (2.0 mmol) in dichloromethane (5 mL) under an ice bath, reacted in the ice bath for 30 minutes, quenched with saturated sodium bicarbonate solution after the reaction was completed, and extracted with dichloromethane three times. The organic phases were combined, rotarily evaporated to dryness, and purified by column chromatography to obtain **ZB-H-84.** LC-MS [M+H]⁺: 563.

### Example 79: preparation of ZB-H-85

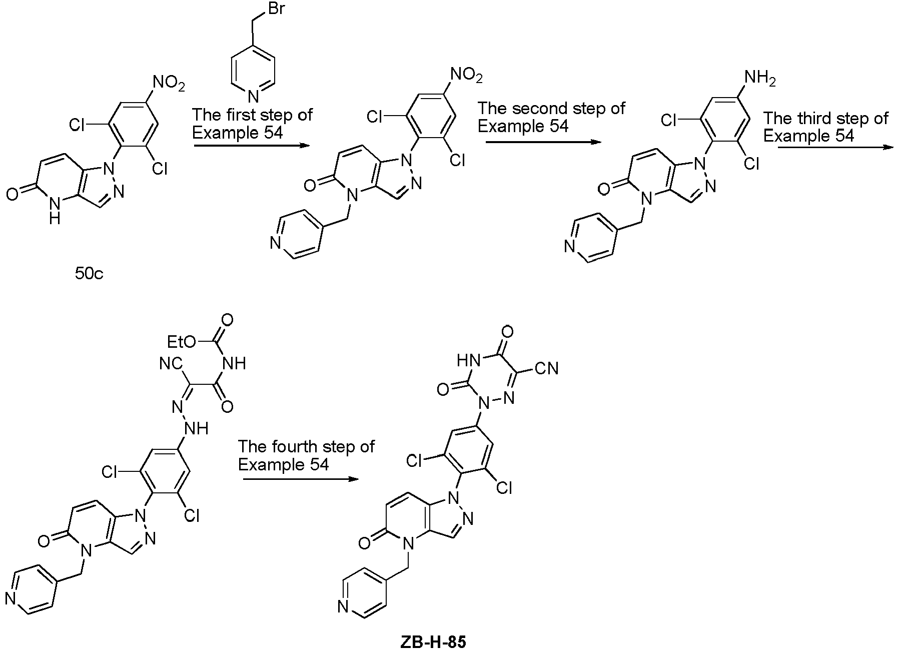

**ZB-H-85** was prepared by referring to the synthetic route of Example 54, except that the benzyl bromide in the first step of Example 54 was replaced with 4-(bromomethyl)-pyridine. LC-MS [M+H]⁺: 508.

### Example 80: preparation of ZB-H-86

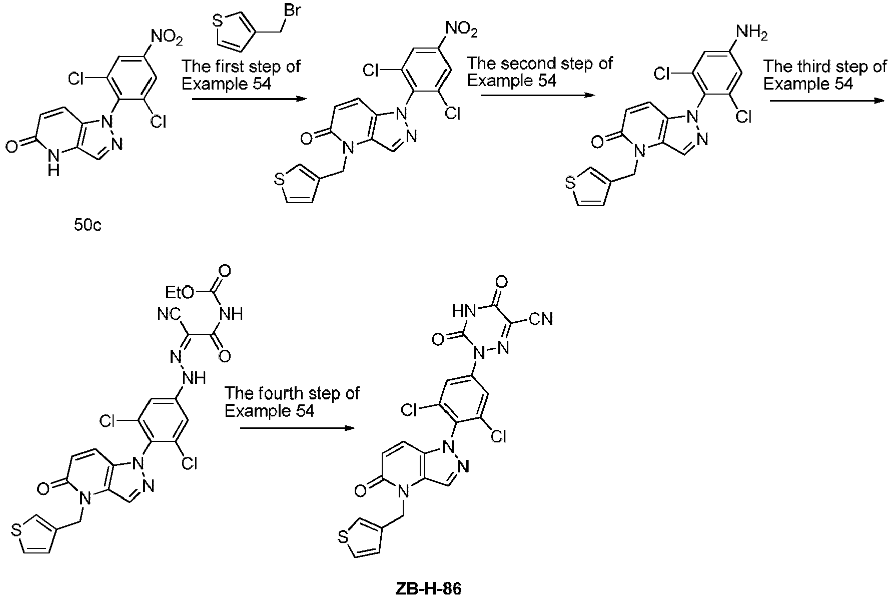

**ZB-H-86** was prepared by referring to the synthetic route of Example 54, except that the benzyl bromide in the first step of Example 54 was replaced with 3-bromomethylthiophene. LC-MS [M+H]⁺: 513.

### Example 81: preparation of ZB-H-87

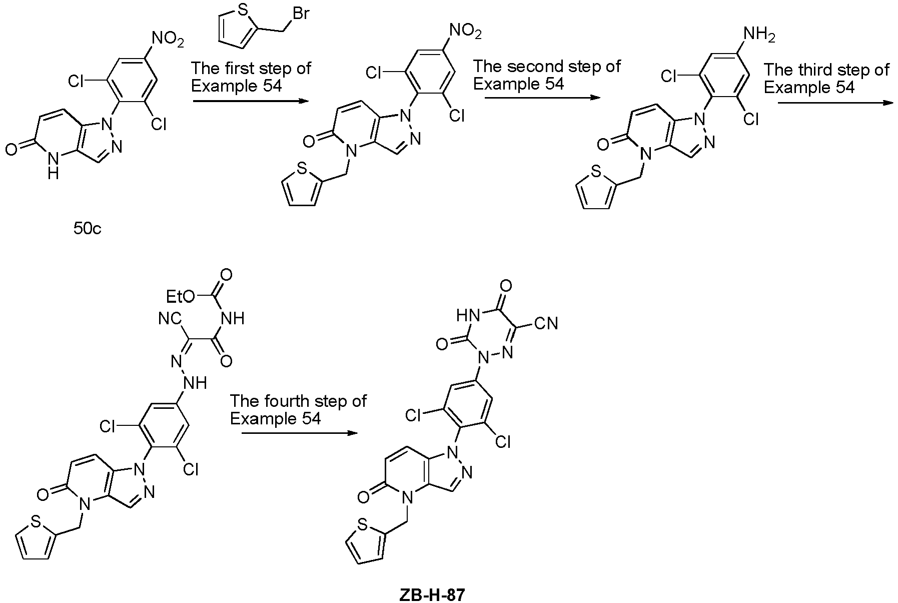

**ZB-H-87** was prepared by referring to the synthetic route of Example 54, except that the benzyl bromide in the first step of Example 54 was replaced with 2-bromomethylthiophene. LC-MS [M+H]⁺: 513.

### Example 82: preparation of ZB-H-88

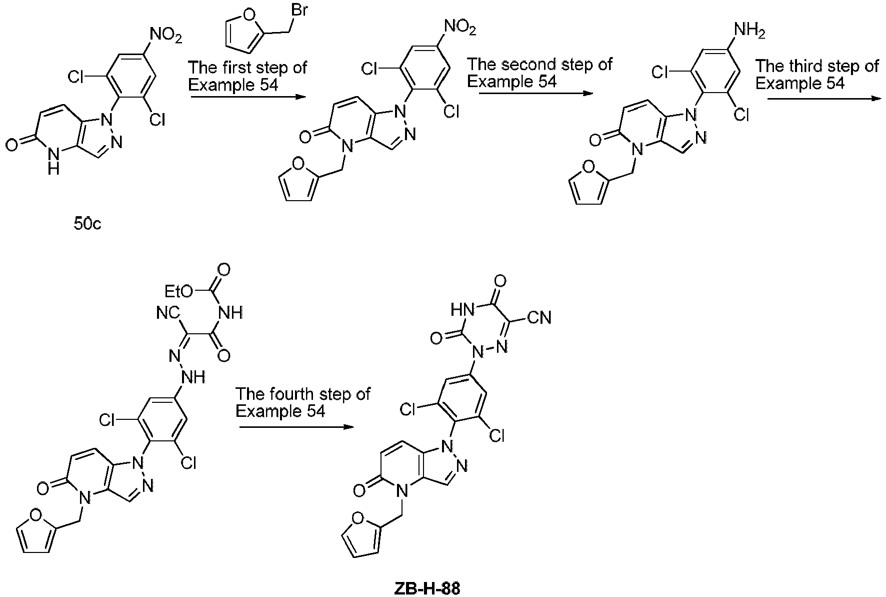

**ZB-H-88** was prepared by referring to the synthetic route of Example 54, except that the benzyl bromide in the first step of Example 54 was replaced with 2-bromomethylfuran. LC-MS [M+H]⁺: 497.

### Example 83: preparation of ZB-H-89

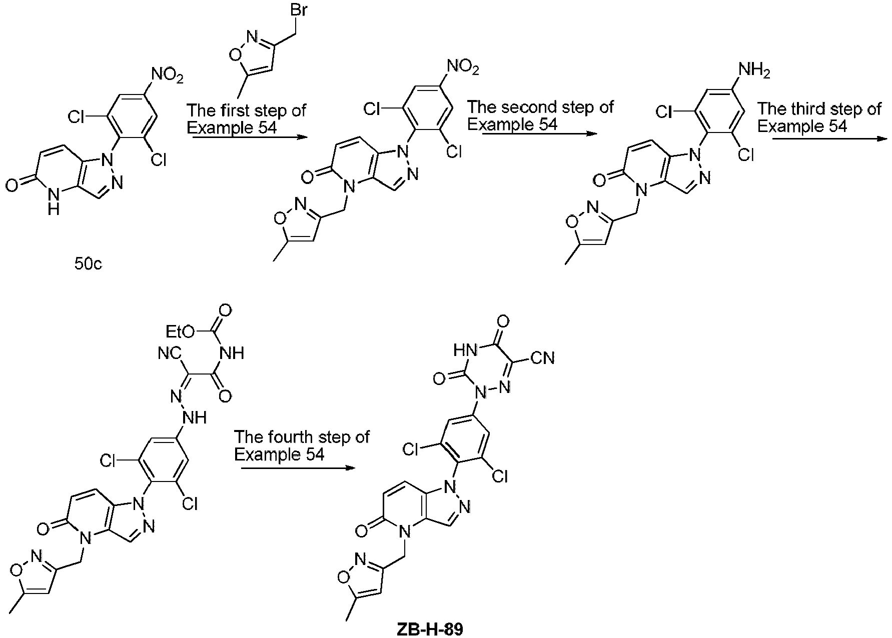

**ZB-H-89** was prepared by referring to the synthetic route of Example 54, except that the benzyl bromide in the first step of Example 54 was replaced with 3-(bromomethyl)-5-methylisoxazole. LC-MS [M+H]⁺: 512.

### Example 84: preparation of ZB-H-90

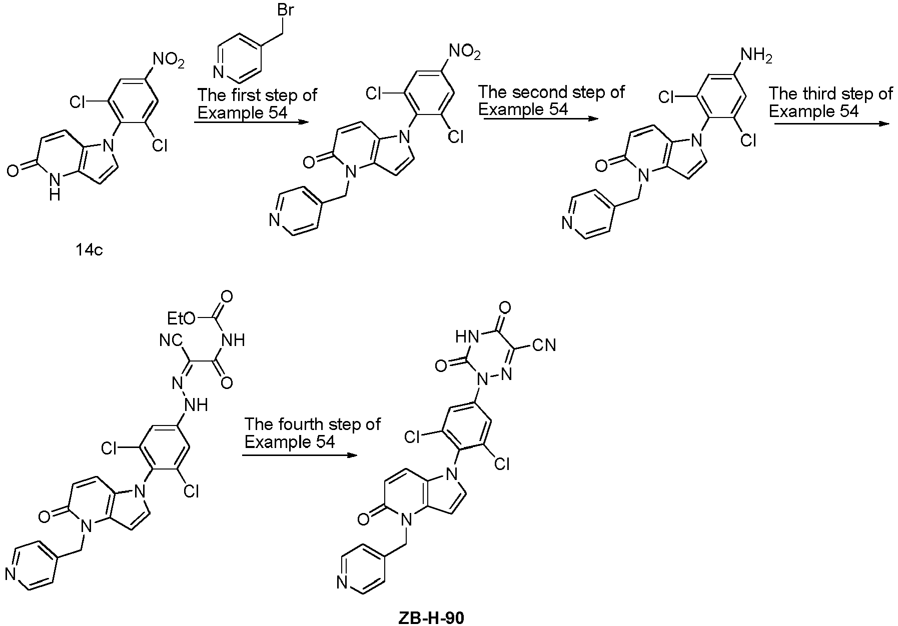

**ZB-H-90** was prepared by referring to the synthetic route of Example 54, except that the benzyl bromide in the first step of Example 54 was replaced with 4-(bromomethyl)-pyridine, and 50c was replaced with **14c.** LC-MS [M+H]⁺: 507.

### Example 85: preparation of ZB-H-91

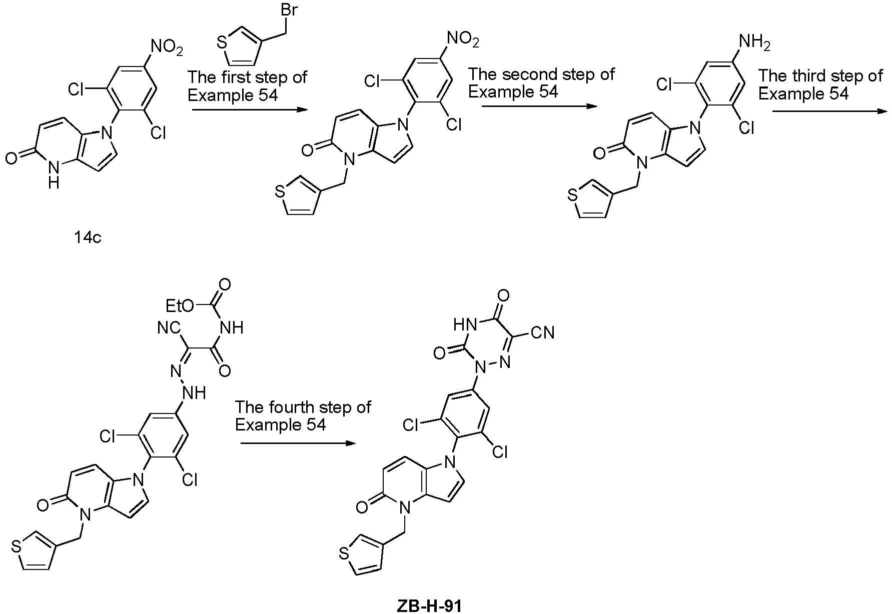

**ZB-H-91** was prepared by referring to the synthetic route of Example 54, except that the benzyl bromide in the first step of Example 54 was replaced with 3-bromomethylthiophene, and **50c** was replaced with **14c.** LC-MS [M+H]⁺: 512.

### Example 86: preparation of ZB-H-92

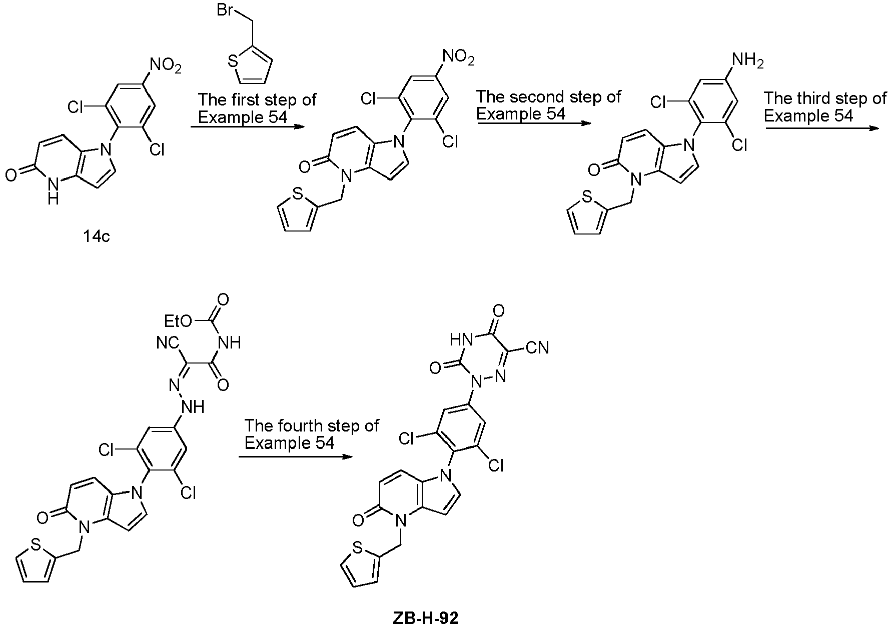

**ZB-H-92** was prepared by referring to the synthetic route of Example 54, except that the benzyl bromide in the first step of Example 54 was replaced with 2-bromomethylthiophene, and **50c** was replaced with **14c.** LC-MS [M+H]⁺: 512.

### Example 87: preparation of ZB-H-93

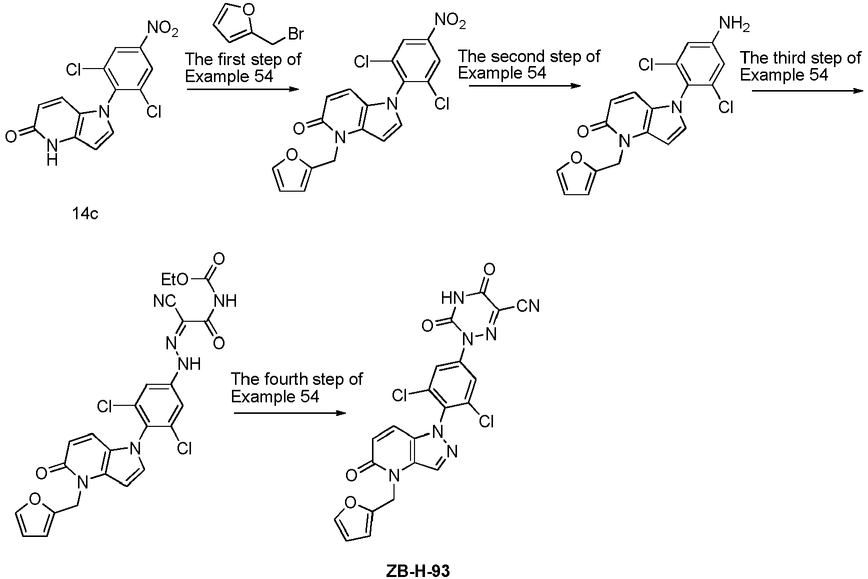

**ZB-H-93** was prepared by referring to the synthetic route of Example 54, except that the benzyl bromide in the first step of Example 54 was replaced with 2-bromomethylfuran, and 50c was replaced with **14c.** LC-MS [M+H]⁺: 496.

### Example 88: preparation of ZB-H-94

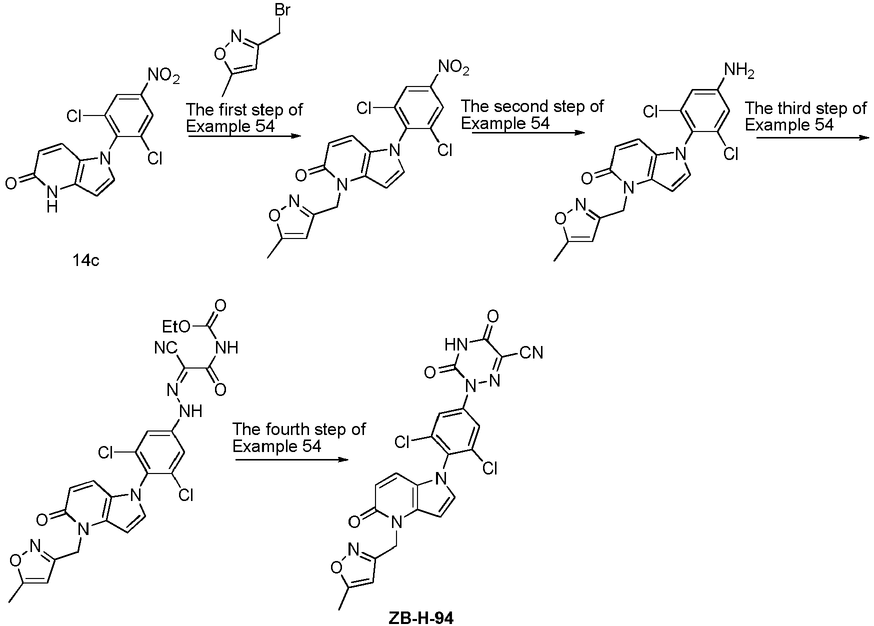

**ZB-H-94** was prepared by referring to the synthetic route of Example 54, except that the benzyl bromide in the first step of Example 54 was replaced with 3-(bromomethyl)-5-methylisoxazole, and 50c was replaced with 14c. LC-MS [M+H]⁺: 511.

### Example 89: preparation of ZB-H-95

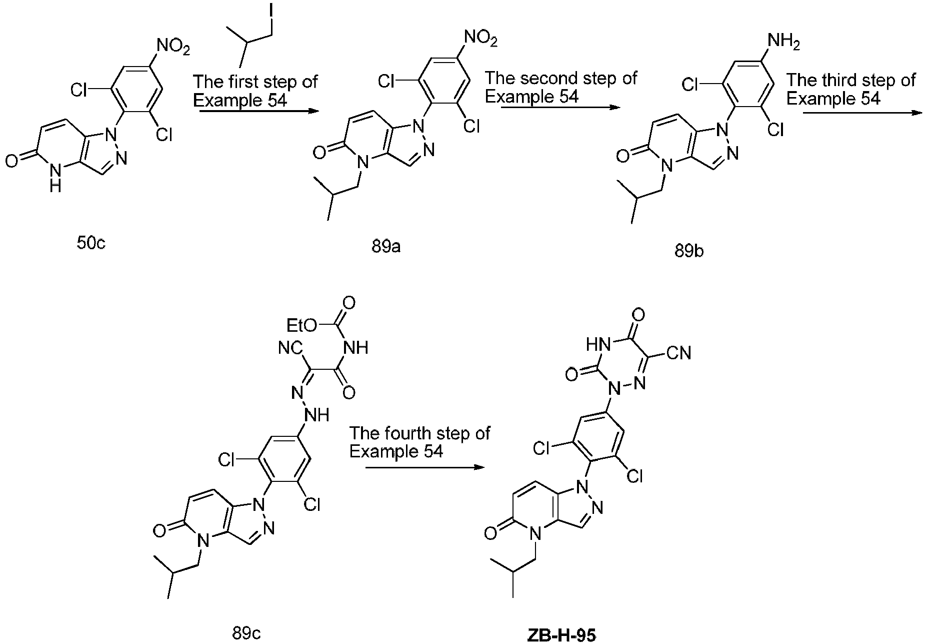

### The first step: synthesis of 89a

**89a** was obtained by referring to the first step of the synthetic route of Example 54, except that the benzyl bromide was replaced with 1-iodo-2-methylpropane. ¹H NMR (600 MHz, Chloroform-*d*) δ 8.37 (s, 2H), 7.82 (s, 1H), 7.14 (d, *J* = 9.6 Hz, 1H), 6.63 (d, *J* = 9.6 Hz, 1H), 3.95 (d, *J* = 7.6 Hz, 2H), 1.28 - 1.15 (m, 1H), 1.00 (d, *J* = 6.7 Hz, 6H).LC-MS [M+H]⁺: 382.

### The second step: synthesis of 89b

**89b** was obtained by referring to the second step of the synthetic route of Example 54. ¹H NMR (400 MHz, Methanol-*d*₄) δ 8.02 (s, 1H), 7.42 (d, *J* = 9.5, 1H), 6.78 (s, 2H), 6.61 (d, *J* = 9.5 Hz, 1H), 4.03 (d, *J* = 7.6 Hz, 2H), 2.31 (m, 1H), 0.99 (d, *J* = 6.7 Hz, 6H).LC-MS [M+H]⁺: 352.

### The third step: synthesis of 89c

**89c** was obtained by referring to the third step of the synthetic route of Example 54. ¹H NMR (600 MHz, DMSO-d6) δ 8.22 (s, 1H), 8.12 (s, 2H), 7.53 (d, J = 9.6 Hz, 1H), 6.50 (d, J = 9.6 Hz, 1H), 4.21 (q, J = 7.1 Hz, 2H), 3.92 (d, J = 7.5 Hz, 2H), 2.28 - 2.16 (m, 1H), 1.27 (t, J = 7.1 Hz, 3H), 0.92 (d, J = 6.7 Hz, 6H).LC-MS [M+H]⁺: 519.

### The fourth step: synthesis of ZB-H-95

**ZB-H-95** was obtained by referring to the fourth step of the synthetic route of Example 54. ¹H NMR (600 MHz, DMSO-*d*₆) δ 13.35 (s, 1H), 8.29 (d, *J* = 1.0 Hz, 1H), 7.93 (s, 2H), 7.68 (d, *J* = 9.6 Hz, 1H), 6.51 (d, *J* = 9.6 Hz, 1H), 3.93 (d, *J* = 7.5 Hz, 2H), 2.21 (m, 1H), 0.93 (d, *J* = 6.7 Hz, 6H).LC-MS [M+H]⁺:473.

### Example 90: preparation of ZB-H-96

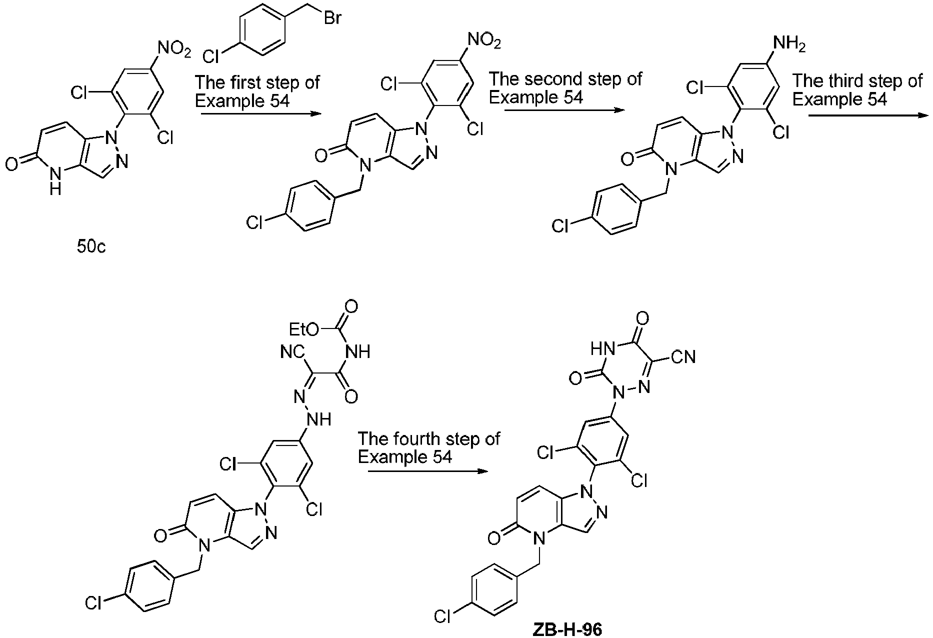

**ZB-H-96** was prepared by referring to the synthetic route of Example 54, except that the benzyl bromide in the first step of Example 54 was replaced with 4-chlorobenzyl bromide. LC-MS [M+H]⁺: 541.

### Example 91: preparation of ZB-H-97

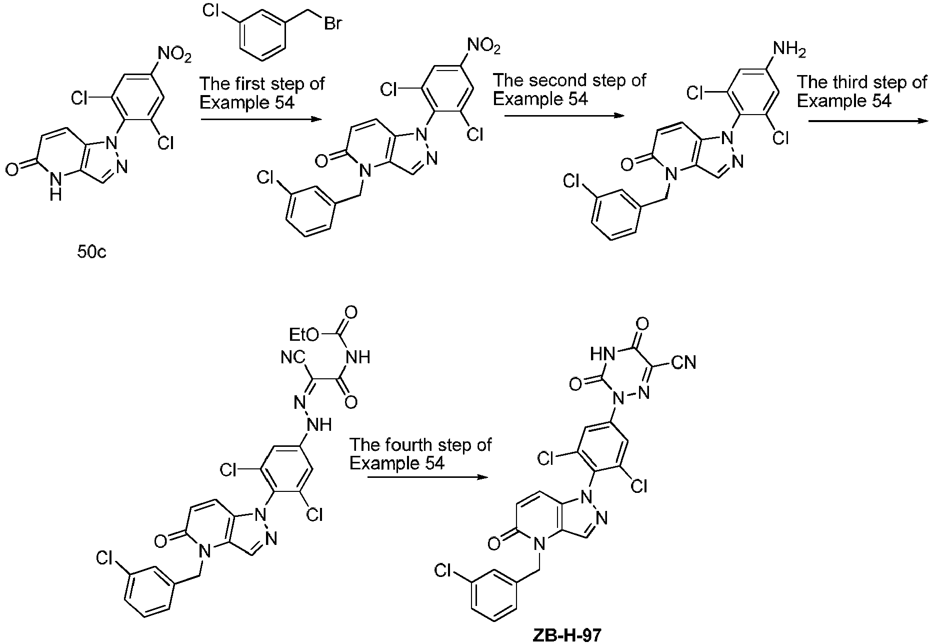

**ZB-H-97** was prepared by referring to the synthetic route of Example 54, except that the benzyl bromide in the first step of Example 54 was replaced with 3-chlorobenzyl bromide. LC-MS [M+H]⁺: 541.

### Example 92: preparation of ZB-H-98

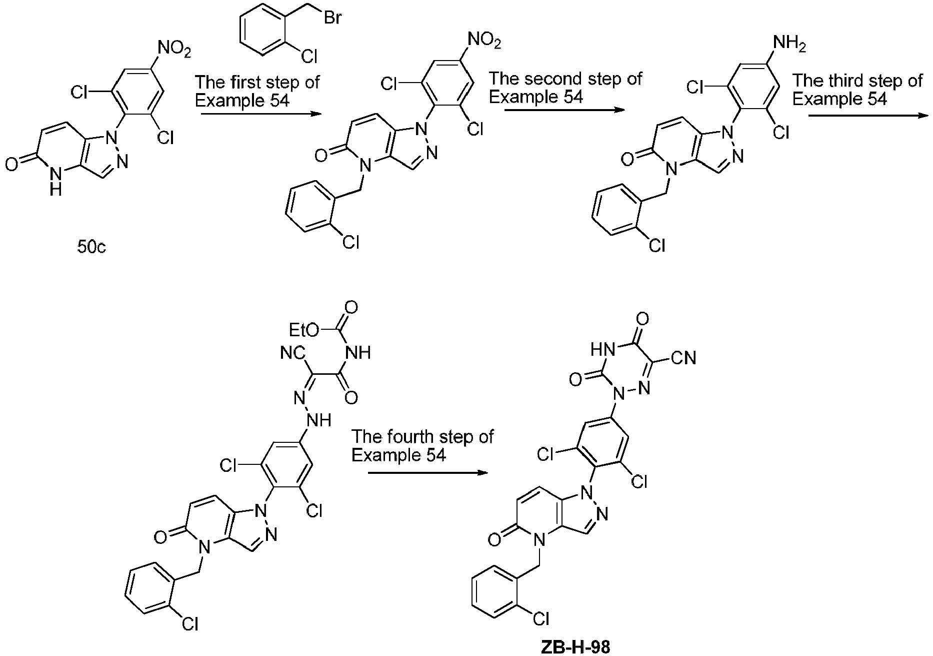

**ZB-H-98** was prepared by referring to the synthetic route of Example 54, except that the benzyl bromide in the first step of Example 54 was replaced with 2-chlorobenzyl bromide. LC-MS [M+H]⁺: 541.

### Example 93: preparation of ZB-H-99

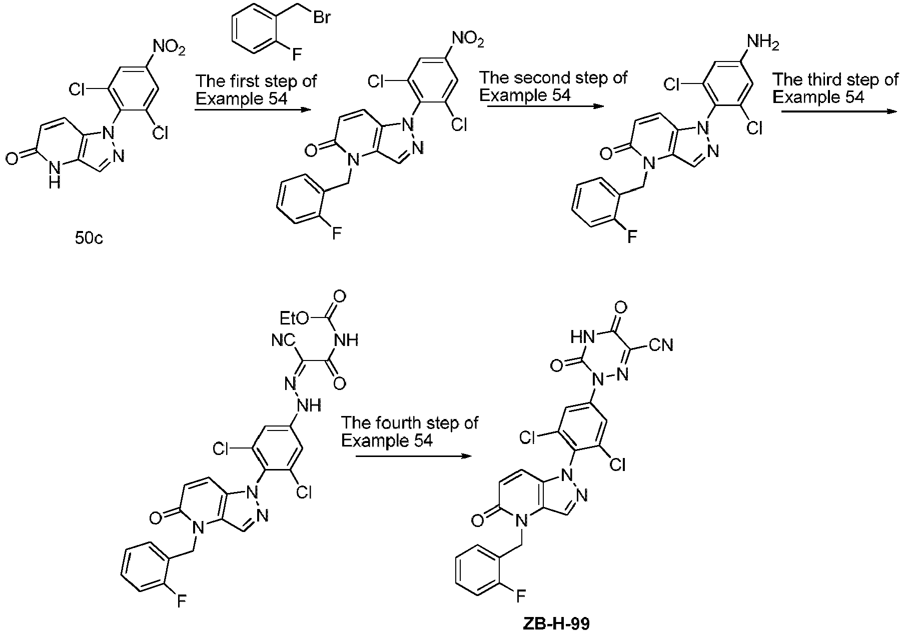

**ZB-H-99** was prepared by referring to the synthetic route of Example 54, except that the benzyl bromide in the first step of Example 54 was replaced with 2-fluorobenzyl bromide. LC-MS [M+H]⁺: 525.

### Example 94: preparation of ZB-H-100

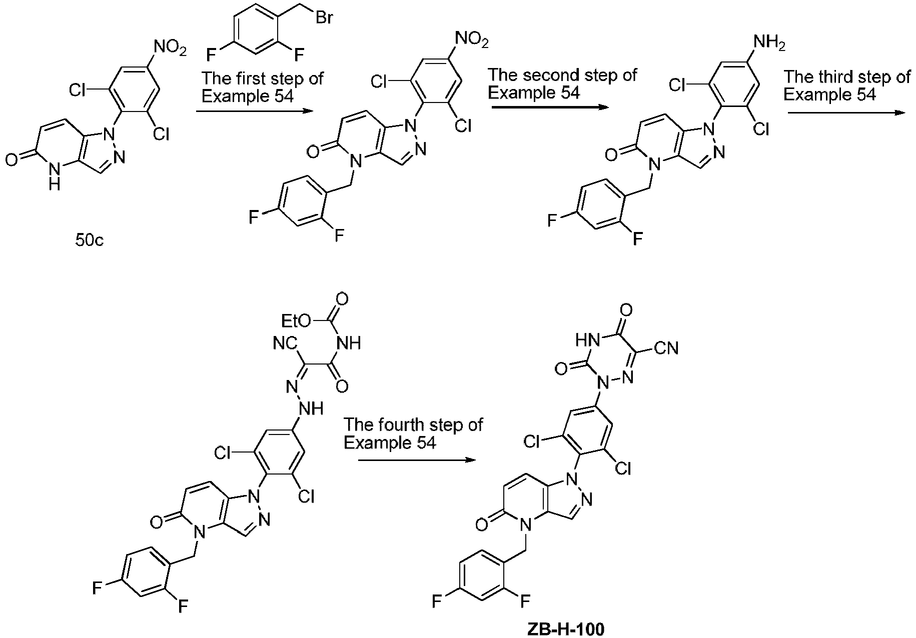

**ZB-H-100** was prepared by referring to the synthetic route of Example 54, except that the benzyl bromide in the first step of Example 54 was replaced with 2,4-difluorobenzyl bromide. LC-MS [M+H]⁺: 543.

### Example 95: preparation of ZB-H-101

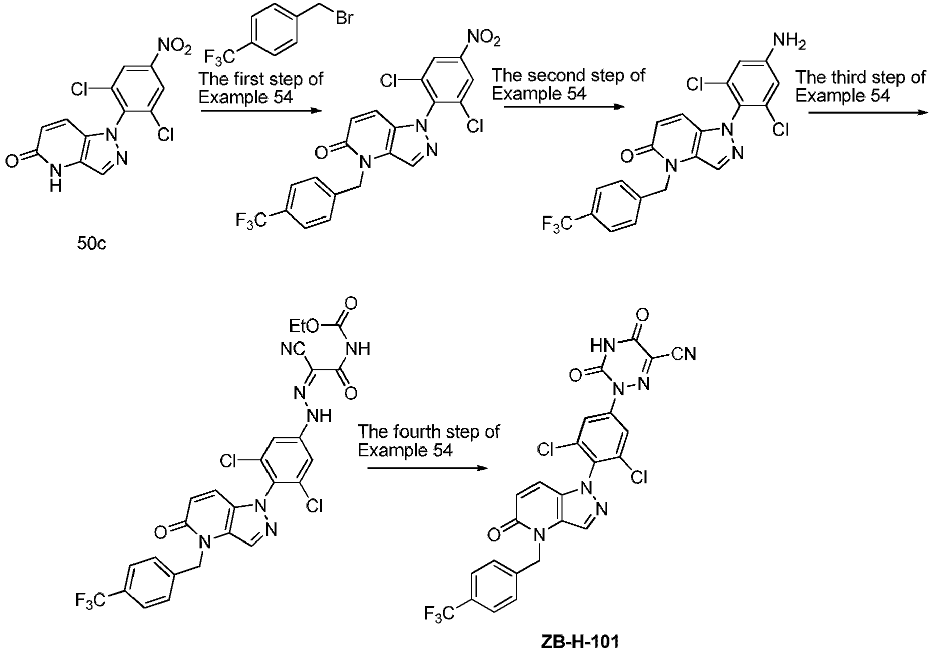

**ZB-H-101** was prepared by referring to the synthetic route of Example 54, except that the benzyl bromide in the first step of Example 54 was replaced with 4-(trifluoromethyl)benzyl bromide. LC-MS [M+H]⁺: 575.

Example 96: Detection of the agonistic activity of compounds on THRα and THRβ by THR reporter gene method

Huh7 cells were cultured in a DMEM medium supplemented with 10% FBS. The cells were inoculated into a 10 cm cell culture dish, proliferated to about 90% confluency, and co-transfected by using a liposome (Lipofectamine 2000) with human THRα eukaryotic expression plasmid or human THRβ eukaryotic expression plasmid, as well as reporter gene plasmid PGL4.26-DR4-Luc containing THR response sequence driver. The operation steps were carried out according to the instructions of Lipofectamine 2000. The next day after transfection, the cells were inoculated with a phenol red-free DMEM (supplemented with 5% activated carbon-treated FBS) into a 96-well cell culture plate at a seeding density of 20,000 cells per well and a volume of 135 µL per well. The cells adhered to wall 6 hours after the inoculation. Compounds dissolved in DMSO were diluted in phenol red-free DMEM (supplemented with 5% activated carbon-treated FBS) to be 20 to 10 times of the final concentration, and added to the cell wells at 15 µL per well, that is, the compounds were diluted 10 times again to reach the final concentration. Triiodothyronine T3 (100 nM) was set as a positive control, and 0.5% DMSO was set as a blank control. After the compounds were added, the cells were cultured at 37°C in a 5% CO₂ incubator overnight (16 hours). After incubation, the culture medium was discarded, and each well was added with 35 µL of serum-free and phenol red-free DMEM and 35 µL of Steady-Glo, shaked for 10 minutes in the dark, and the chemiluminescence value of the sample was detected.

The agonistic activity of the compound was calculated as follows: % effect = (compound - blank control) / (positive control - blank control) × 100%. The EC₅₀ of the compound was obtained by fitting the agonistic activity of the compound and the logarithmic value of the compound concentration with GraphPad Prism. The lower the EC₅₀ value, the better the activity.

The EC₅₀ values of agonistic activities of compounds on THRα and THRβ were firstly corrected by the EC₅₀ values of agonistic activities of T3 on THRα and THRβ in the same experiment, and then the multiple, that is, receptor selectivity were calculated based on the obtained values. The specific calculation method is as follows: Selectivity = (EC₅₀ of compound on THRα/EC₅₀ of T3 on THRα)/(EC₅₀ of compound on THRβ/EC₅₀ of T3 on THRβ). The higher the value, the higher the selectivity of the compound on the THRβ receptor.

The positive control is Resmetirom (MGL-3196) , which is a highly selective THR-β agonist. (Reference: J Med Chem. 2014 , 57(10): 3912-3923).

| **Compound** | **THR-α EC₅₀ [µM]** | **THR-β EC₅₀ [µM]** | **β selectivity** |
|---|---|---|---|
| **MGL-3196** | 7.9 | 2.5 | 6.8 |
| ZB-H-01 | > 33 | > 33 | N/A |
| ZB-H-02 | 1.547 | 0.61 | 6.0 |
| ZB-H-07 | 21.1 | 2.1 | 17.7 |
| ZB-H-08 | 16 | 3.87 | 8.0 |
| ZB-H-09 | > 100 | > 100 | N/A |
| ZB-H-11 | 7.39 | 2.45 | 8.2 |
| ZB-H-12 | 4.17 | 5.04 | 1.6 |
| ZB-H-13 | 3.07 | 1.75 | 3.0 |
| ZB-H-15 | >11 | >4 | N/A |
| ZB-H-16 | > 33 | > 100 | N/A |

| | | | |
|---|---|---|---|
| ZB-H-17 | 0.04 | 0.02 | 3.8 |
| ZB-H-18 | 0.06 | 0.01 | 8.4 |
| ZB-H-19 | 0.3 | 0.1 | 3.2 |
| ZB-H-20 | 3.7 | 2.5 | 2.8 |
| ZB-H-21 | 0.7 | 0.6 | 2.1 |
| ZB-H-22 | >11 | >3.7 | N/A |
| ZB-H-23 | > 33 | > 33 | N/A |
| ZB-H-24 | 0.8 | 0.4 | 3.5 |
| ZB-H-25 | 0.7 | 0.1 | 7.1 |
| ZB-H-26 | 0.23 | 0.03 | 15.9 |
| ZB-H-27 | 1.1 | 0.5 | 3.5 |
| ZB-H-31 | 3.1 | 1.1 | 4.7 |
| ZB-H-32 | 1.3 | 0.2 | 10.6 |
| ZB-H-33 | 0.2 | 0.04 | 10.4 |
| ZB-H-34 | 1.3 | 0.3 | 7.2 |
| ZB-H-35 | 1.9 | 0.42 | 10.9 |
| ZB-H-36 | >11 | 6.5 | N/A |
| ZB-H-38 | 3.6 | 0.2 | 33.5 |
| ZB-H-47 | > 10 | 1.2 | 17.4 |
| ZB-H-45 | 0.5 | 0.05 | 8.7 |
| ZB-H-53 | 0.088 | 0.035 | 2.2 |
| ZB-H-54 | 0.013 | 0.0055 | 2.1 |
| ZB-H-74 | 1.905 | 0.158 | 21.2 |
| ZB-H-75 | > 10 | 2.362 | N/A |
| ZB-H-76 | 2.334 | 0.100 | 45.4 |

| | | | |
|---|---|---|---|
| ZB-H-81 | 0.9433 | < 0.1372 | N/A |
| ZB-H-95 | 6.303 | 0.3006 | 43.1 |

Experimental results showed that some compounds of the present disclosure (such as ZB-H-02, ZB-H-17, ZB-H-18, ZB-H-25, ZB-H-26, ZB-H-33, ZB-H-38, ZB-H-74, ZB-H-75, ZB-H-76, ZB-H-81, ZB-H-95, etc.) have significantly higher activity or selectivity than the positive control, and most other compounds have activity or selectivity comparable to the positive control (MGL-3196).

Example 97: determination of lowering effect on serum cholesterol level after single administration of compounds to ICR mice

ICR mice were used as test animals to test the changes in serum cholesterol level after intragastric administration of the compound to the mice. ICR mice (male, 7-8 weeks old) were randomly divided into groups according to body weight. The solvent control group was given 0.25% CMC-Na by intragastric administration, and the compound group was given the corresponding compounds to be tested with single administration, respectively. Mice were fasted overnight on the day of administration. 24 h after administration, blood was taken and serum was collected. Cholesterol level in mouse serum was measured with a total cholesterol assay kit (Zhejiang Dongou Diagnostic Products Co., Ltd.). The percentage of reduction in serum cholesterol level of mice in each group was calculated with the solvent control group as 100%.

| **Compound** | **Dose** | **Percentage of reduction in serum cholesterol level** |
|---|---|---|
| ZB-H-02 | 10 mg/kg | 20.0% |
| ZB-H-07 | 30 mg/kg | 13.6% |
| ZB-H-08 | 30 mg/kg | 11.6% |
| ZB-H-17 | 30 mg/kg | 25.4% |
| ZB-H-18 | 30 mg/kg | 15.4% |
| ZB-H-25 | 30 mg/kg | 20.4% |
| ZB-H-26 | 30 mg/kg | 33.5% |
| ZB-H-32 | 30 mg/kg | 35.9% |
| ZB-H-33 | 30 mg/kg | 8.2% |
| ZB-H-34 | 30 mg/kg | 20.8% |
| ZB-H-37 | 30 mg/kg | 20.6% |
| ZB-H-38 | 30 mg/kg | 22.7% |

| | | |
|---|---|---|
| ZB-H-43 | 30 mg/kg | 3.7% |
| ZB-H-45 | 30 mg/kg | 24.2% |
| ZB-H-46 | 30 mg/kg | 19.3% |
| ZB-H-47 | 30 mg/kg | 27.4% |
| ZB-H-48 | 30 mg/kg | 17.1% |
| ZB-H-51 | 3 mg/kg | 28.0% |
| ZB-H-53 | 3 mg/kg | 40.6% |

The experimental results obtained 24 hours after the administration showed that the compounds of the present disclosure have a certain in vivo medicinal effects in animals.

## Claims

1. A compound represented by formula (I), or a pharmaceutically acceptable salt, stereoisomer, enantiomer, diastereomer, atropisomer, racemate, polymorph, solvate or isotopically labeled compound thereof: 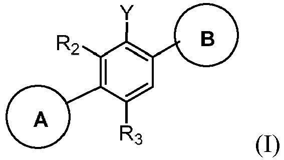 wherein
ring A is selected from the group consisting of: 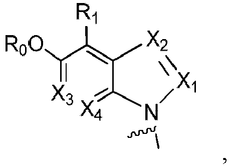 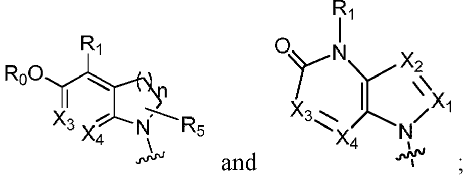
ring B is selected from the group consisting of: 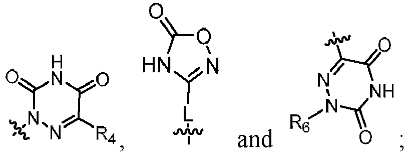
R₀ is selected from the group consisting of hydrogen, and C₁₋₁₀ alkyl;
R₁ is selected from the group consisting of hydrogen, substituted or unsubstituted C₁₋₁₀ alkyl, substituted or unsubstituted C₃₋₁₀ cycloalkyl, substituted or unsubstituted 3-10 membered heterocycloalkyl, substituted or unsubstituted C₆₋₁₀ aryl and substituted or unsubstituted 5-10 membered heteroaryl, wherein the substituents for the "substituted" are selected from the group consisting of halogen, hydroxyl, =O, C₁₋₆ alkoxy, C₁₋₆ alkyl, C₃₋₁₀ cycloalkyl, C₆₋₁₀ aryl, halogenated C₆₋₁₀ aryl, C₁₋₁₀ alkyl C₆₋₁₀ aryl, C₁₋₁₀ alkoxy C₆₋₁₀ aryl, 5-10 membered heteroaryl, C₁₋₁₀ alkyl 5-10 membered heteroaryl, halogenated 5-10 membered heteroaryl, 3-10 membered heterocycloalkyl and -NR¹⁰R¹¹;
R₂, R₃ and Y are each independently selected from the group consisting of hydrogen, halogen, substituted or unsubstituted C₁₋₆ alkyl, substituted or unsubstituted C₃₋₆ cycloalkyl, and substituted or unsubstituted C₁₋₆ alkoxy, wherein the substituents for the "substituted" are selected from the group consisting of halogen, hydroxyl, C₁₋₆ alkyl and C₁₋₆ alkoxy;
R₄ is selected from the group consisting of hydrogen, cyano, -NR¹⁰R¹¹, substituted or unsubstituted C₁₋₆ alkyl, substituted or unsubstituted C₃₋₆ cycloalkyl and substituted or unsubstituted C₂₋₈ alkynyl, wherein the substituents for the "substituted" are selected from the group consisting of halogen, hydroxyl, cyano and C₁₋₆ alkoxy;
R₅ is selected from the group consisting of hydrogen, substituted or unsubstituted C₁₋₆ alkyl and substituted or unsubstituted C₃₋₆ cycloalkyl, wherein the substituents for the "substituted" are selected from the group consisting of halogen, hydroxyl and C₁₋₆ alkoxy;
R₆ is selected from the group consisting of hydrogen and substituted or unsubstituted C₁₋₆ alkyl, wherein the substituents for the "substituted" are selected from the group consisting of halogen, hydroxyl and C₁₋₆ alkoxy;
L is absent, or -NR¹⁰C(O)- or -NR¹⁰CR¹¹R¹¹-;
each R¹⁰ is independently selected from the group consisting of hydrogen and substituted or unsubstituted C₁₋₃ alkyl, wherein the substituents for the "substituted" are selected from the group consisting of halogen, hydroxyl and C₁₋₃ alkoxy;
each R¹¹ is independently selected from the group consisting of hydrogen and substituted or unsubstituted C₁₋₆ alkyl, wherein the substituents for the "substituted" are selected from the group consisting of halogen, hydroxyl and C₁₋₆ alkoxy;
X₁ and X₂ are each independently selected from the group consisting of N and CR¹², wherein R¹² is selected from the group consisting of hydrogen, halogen, cyano, -NR^{b}R^{c}, -C(=O)R^{a}, -C(=O)OR^{b}, -C(=O)NR^{b}R^{c}, substituted or unsubstituted C₁₋₄ alkyl, substituted or unsubstituted C₃₋₆ cycloalkyl, substituted or unsubstituted C₆₋₁₀ aryl and substituted or unsubstituted 5-10 membered heteroaryl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, halogen, cyano, hydroxyl, -OR^{a}, -NR^{b}R^{c}, -C(=O)R^{a}, -C(=O)OR^{b}, -C(=O)NR^{b}R^{c}, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
each R^{a} is independently C₁₋₆ alkyl, C₃₋₁₀ cycloalkyl or 3-10 membered heterocycloalkyl, wherein the alkyl, cycloalkyl and heterocycloalkyl are independently and optionally substituted by one or more substituents selected from the group consisting of halogen, hydroxyl, amino and C₁₋₆ alkyl;
each R^{b} and R^{c} is independently hydrogen, C₁₋₆ alkyl, C₃₋₁₀ cycloalkyl or 3-10 membered heterocycloalkyl; wherein the alkyl, cycloalkyl and heterocycloalkyl are independently and optionally substituted by one or more substituents selected from the group consisting of halogen, hydroxyl, amino and C₁₋₆ alkyl;
or R^{b} and R^{c}, together with the nitrogen atom to which they are connected, form a 3-10 membered heterocycloalkyl, wherein the heterocycloalkyl is optionally substituted by one or more substituents selected from the group consisting of halogen, hydroxyl, amino and C₁₋₆ alkyl;
n is independently 1, 2 or 3 at each occurrence.

2. The compound or the pharmaceutically acceptable salt, stereoisomer, enantiomer, diastereomer, atropisomer, racemate, polymorph, solvate or isotopically labeled compound thereof according to claim 1, wherein, the compound of formula (I) is selected from the group consisting of the compounds represented by formula (Ia) or (Ib): 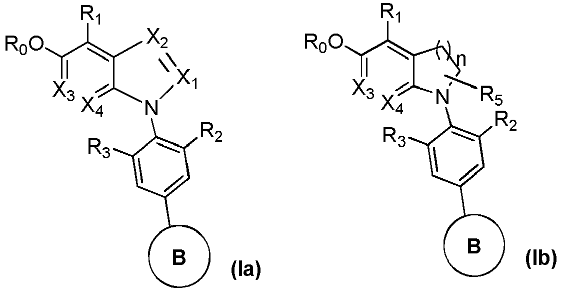 wherein
R₀ is hydrogen;
R₁ is selected from the group consisting of hydrogen, substituted or unsubstituted C₁₋₆ alkyl, substituted or unsubstituted C₃₋₆ cycloalkyl and substituted or unsubstituted C₃₋₈ heterocycloalkyl, wherein the substituents for the "substituted" are selected from the group consisting of halogen, a hydroxyl, =O, C₁₋₆ alkoxy;
R₂ and R₃ are each independently selected from the group consisting of halogen, substituted or unsubstituted C₁₋₆ alkyl and substituted or unsubstituted C₃₋₆ cycloalkyl, wherein the substituents for the "substituted" are selected from the group consisting of halogen, hydroxyl, C₁₋₄ alkyl and C₁₋₄ alkoxy;
n is independently 1 or 2 at each occurrence;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
R₅ and B ring are the same as defined in the formula I of claim 1.

3. The compound or the pharmaceutically acceptable salt, stereoisomer, enantiomer, diastereomer, atropisomer, racemate, polymorph, solvate or isotopically labeled compound thereof according to claim 1, wherein, the compound of formula (I) is selected from the group consisting of the compounds represented by formula (Ic): 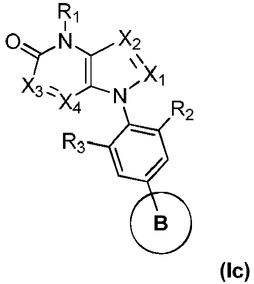 wherein
R₂ and R₃ are each independently selected from the group consisting of halogen, a substituted or unsubstituted C₁₋₆ alkyl and a substituted or unsubstituted C₃₋₆ cycloalkyl, wherein the substituents for the "substituted" are selected from the group consisting of halogen, hydroxyl, C₁₋₄ alkyl and C₁₋₄ alkoxy;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
X₃ and X₄ are each independently selected from the group consisting of N and CR¹³, wherein R¹³ is selected from the group consisting of hydrogen, substituted or unsubstituted C₁₋₄ alkyl and substituted or unsubstituted C₃₋₆ cycloalkyl, wherein the substituents for the "substituted" are selected from the group consisting of halogen, hydroxyl and C₁₋₆ alkoxy;
R₁ and B ring are the same as defined in the formula I of claim 1.

4. The compound or the pharmaceutically acceptable salt, stereoisomer, enantiomer, diastereomer, atropisomer, racemate, polymorph, solvate or isotopically labeled compound thereof according to claim 1, wherein, the compound of formula (I) is selected from the group consisting of the compounds represented by formula (Id): 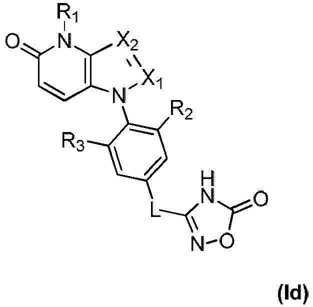 wherein
R₂ and R₃ are each independently selected from the group consisting of halogen;
L is -NHC(O)- or -NHCHR¹¹-; wherein R¹¹ is selected from the group consisting of hydrogen and substituted or unsubstituted C₁₋₆ alkyl, wherein the substituents for the "substituted" are selected from the group consisting of halogen, hydroxyl and C₁₋₆ alkoxy;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ is the same as defined in the formula I of claim 1.

5. The compound or the pharmaceutically acceptable salt, stereoisomer, enantiomer, diastereomer, atropisomer, racemate, polymorph, solvate or isotopically labeled compound thereof according to claim 1, wherein, the compound of formula (I) is selected from the group consisting of the compounds represented by formula (Ie): 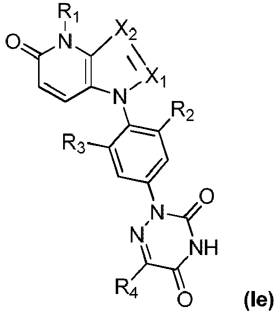 wherein
R₂ and R₃ are each independently selected from the group consisting of halogen;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ and R₄ are the same as defined in the formula I of claim 1.

6. The compound or the pharmaceutically acceptable salt, stereoisomer, enantiomer, diastereomer, atropisomer, racemate, polymorph, solvate or isotopically labeled compound thereof according to claim 1, wherein, the compound of formula (I) is selected from the group consisting of the compounds represented by formula (If): 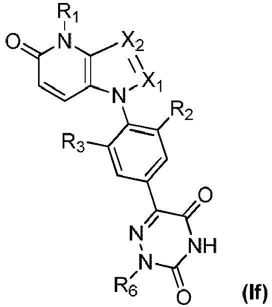 wherein
R₂ and R₃ are each independently selected from the group consisting of halogen;
X₁ and X₂ are each independently selected from the group consisting of N and CR¹², wherein R¹² is selected from the group consisting of hydrogen, halogen, cyano, substituted or unsubstituted C₁₋₄ alkyl, substituted or unsubstituted C₃₋₆ cycloalkyl, wherein the substituents for the "substituted" are selected from the group consisting of halogen, hydroxyl and C₁₋₆ alkoxy;
R₁ and R₆ are the same as defined in the formula I of claim 1.

7. The compound or the pharmaceutically acceptable salt, stereoisomer, enantiomer, diastereomer, atropisomer, racemate, polymorph, solvate or isotopically labeled compound thereof according to claim 1, wherein, the compound of formula I is selected from the group consisting of:

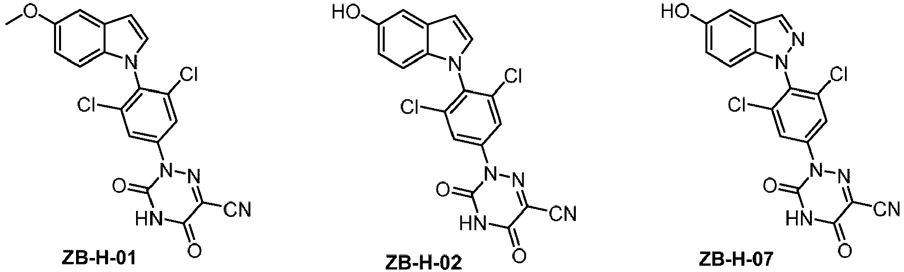

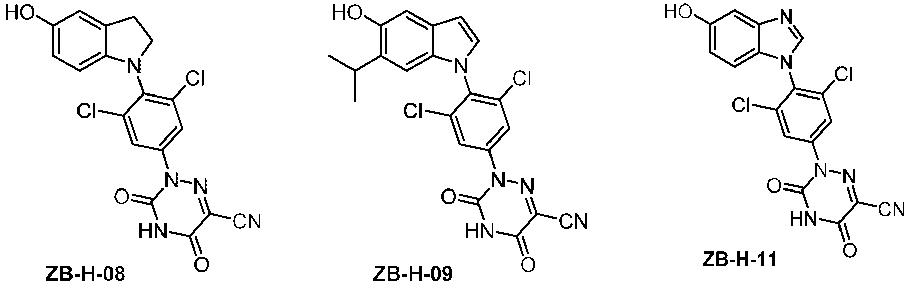

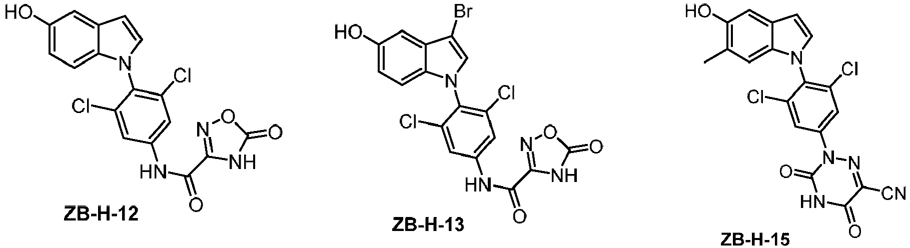

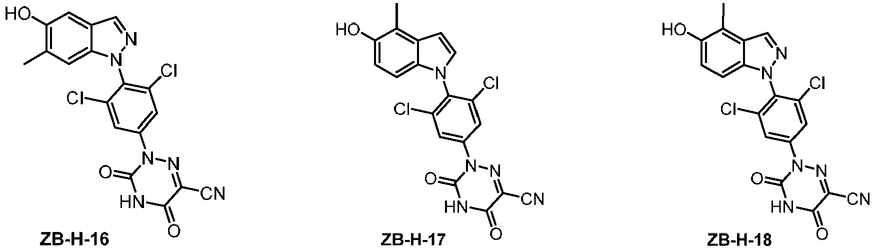

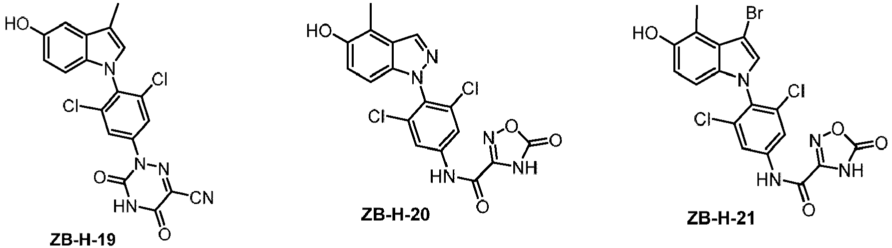

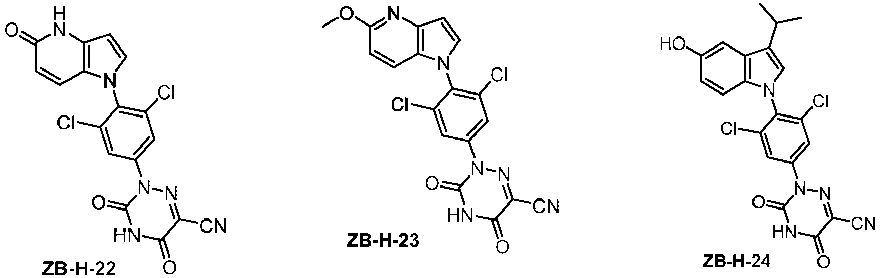

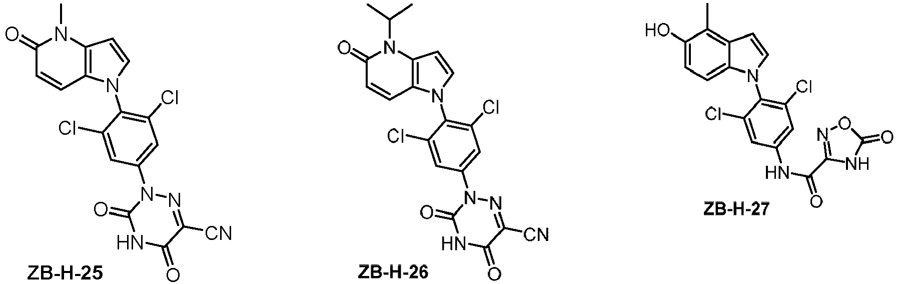

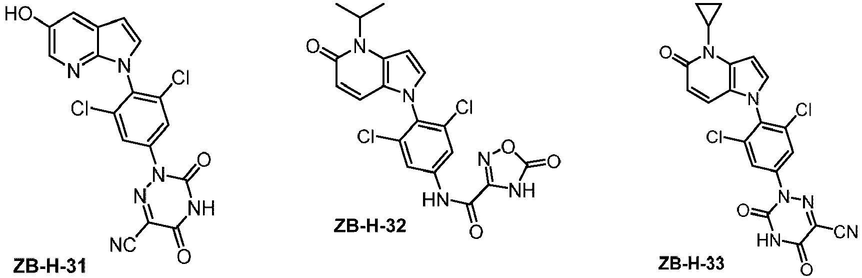

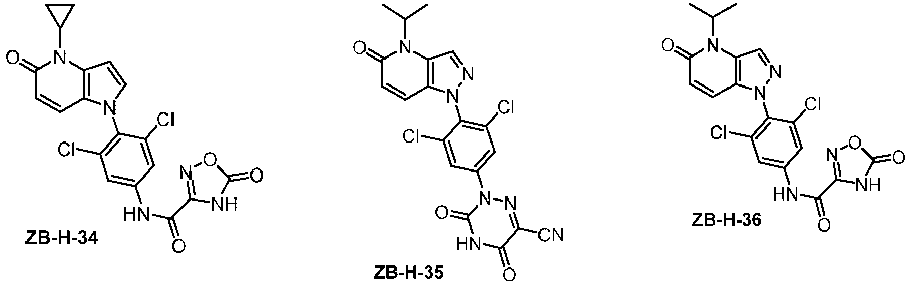

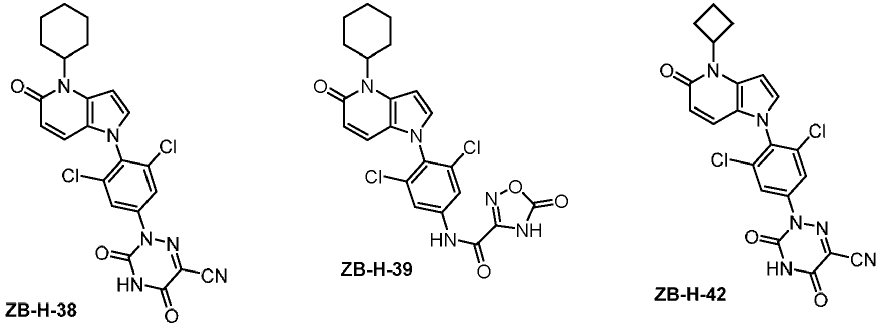

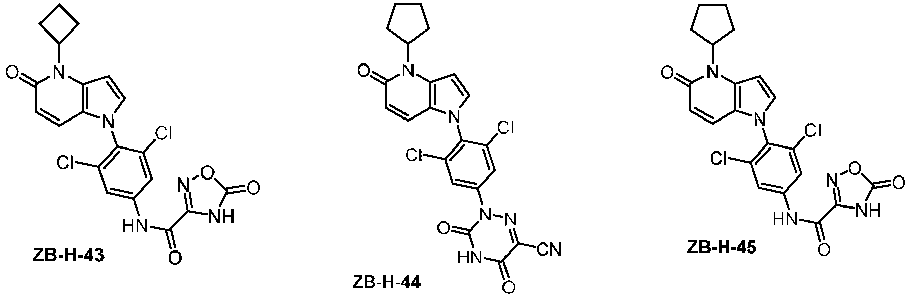

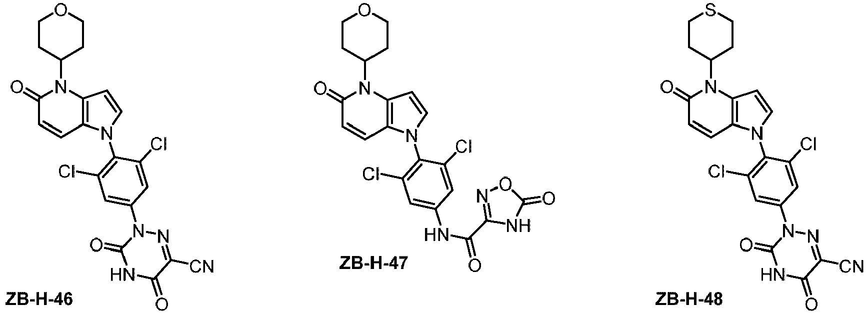

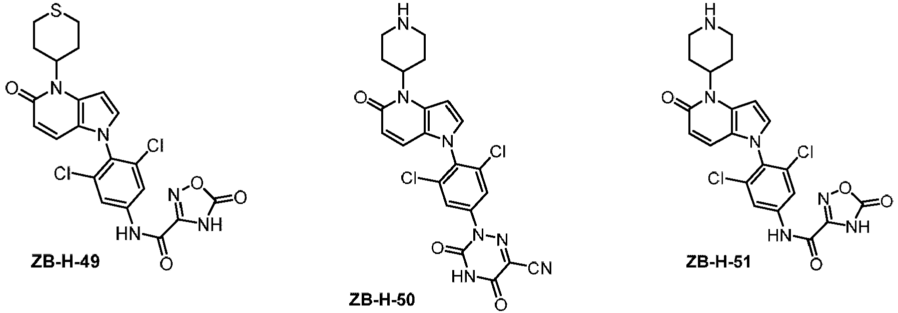

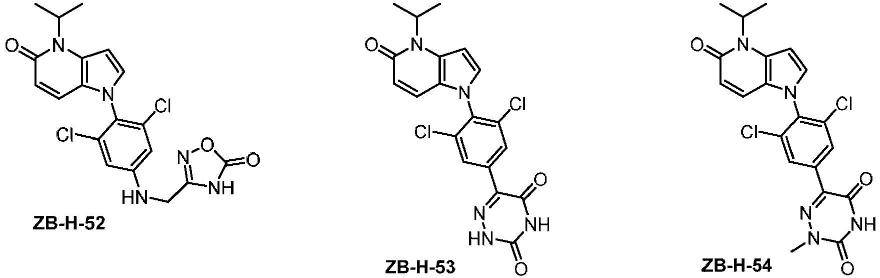

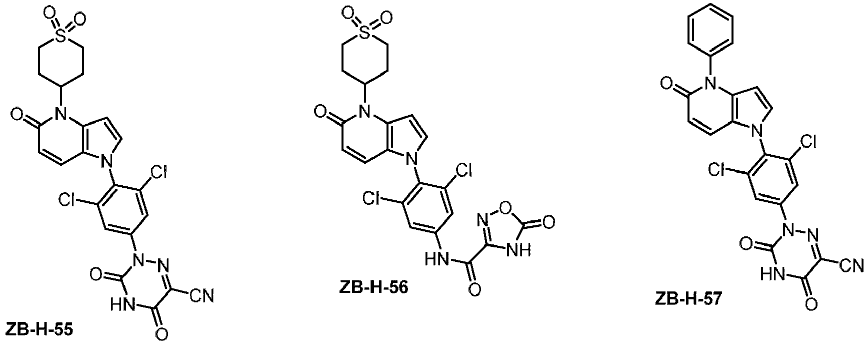

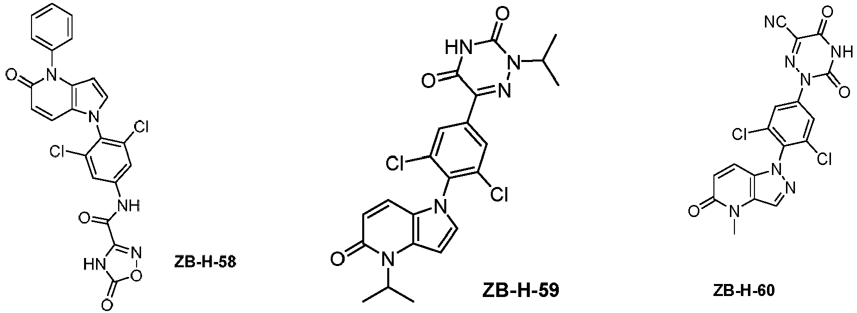

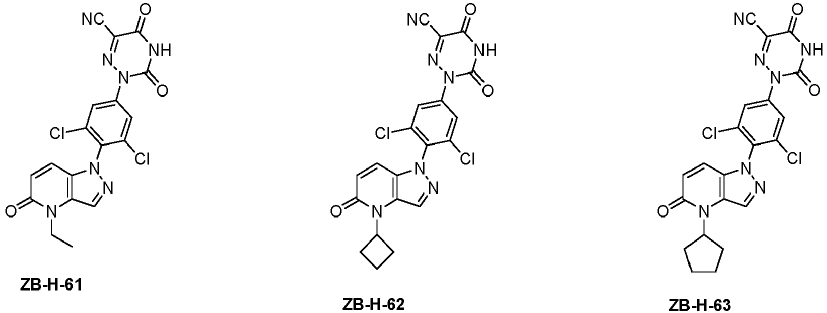

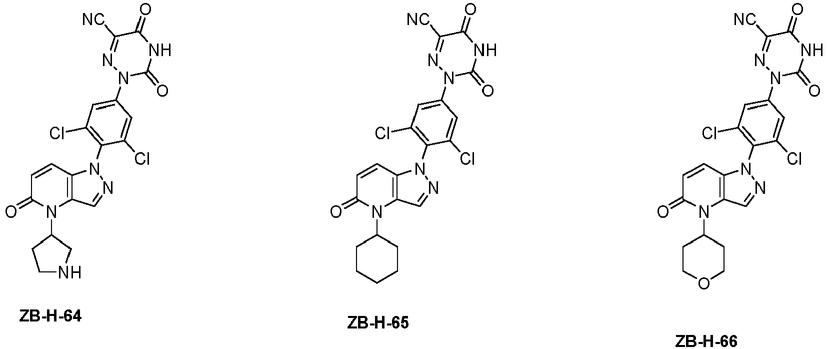

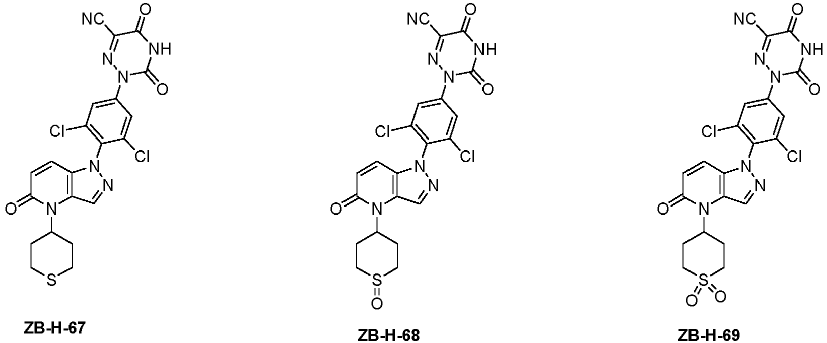

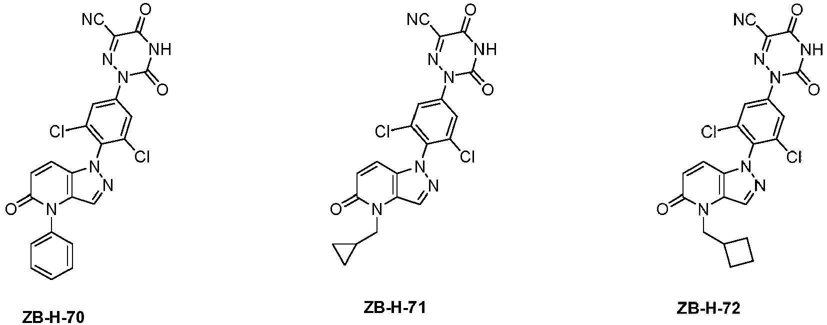

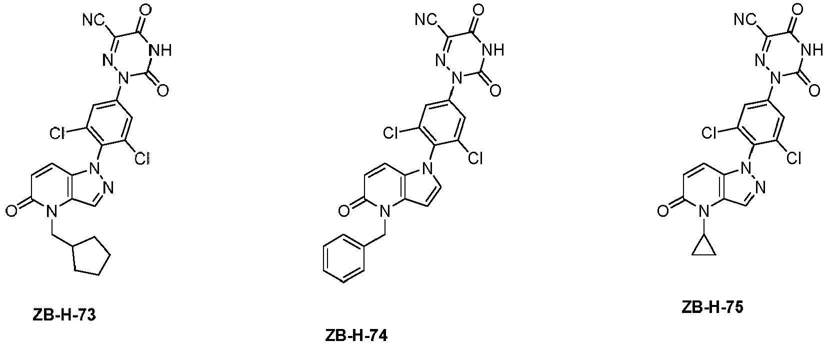

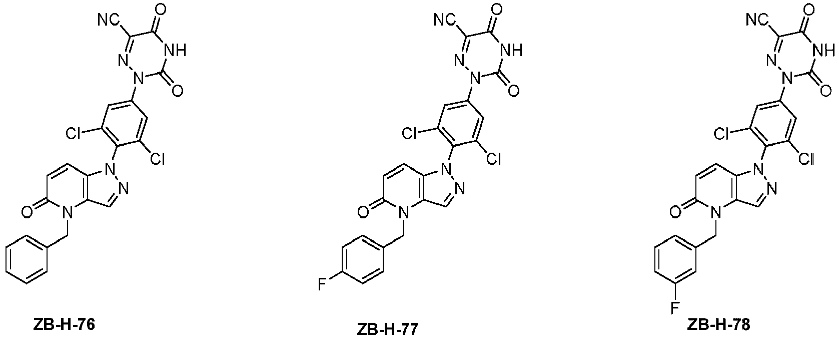

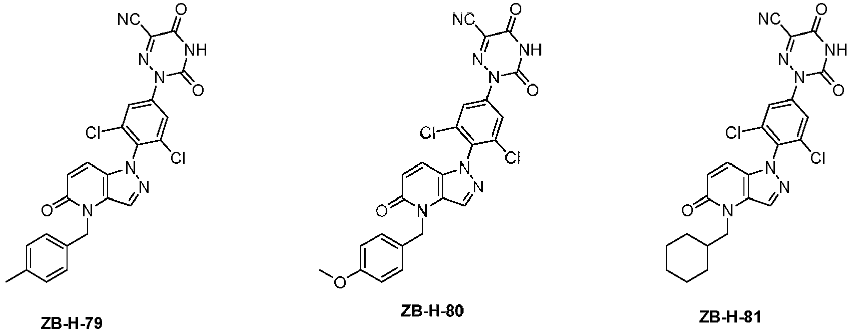

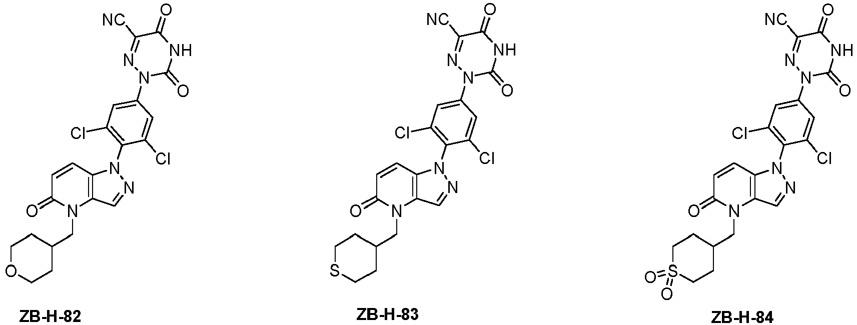

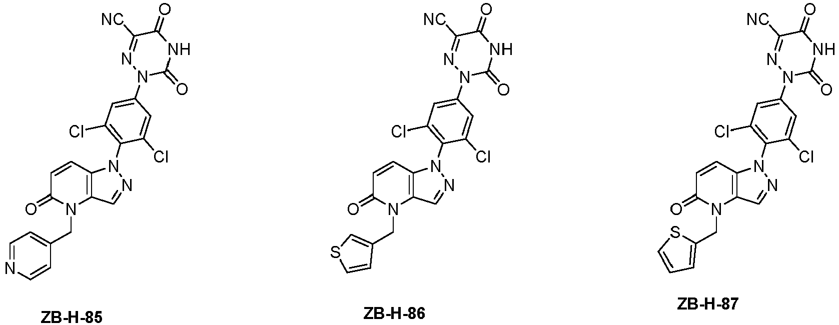

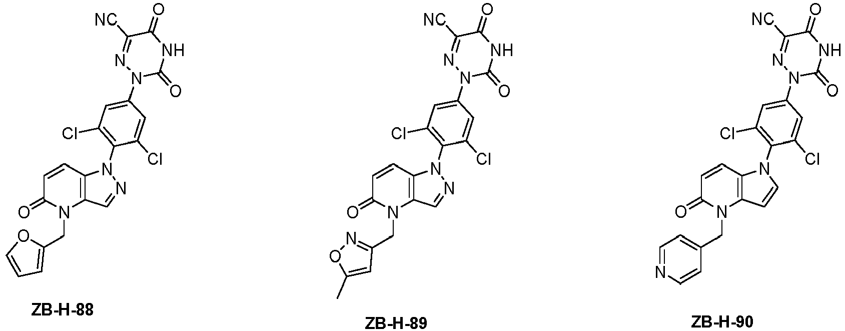

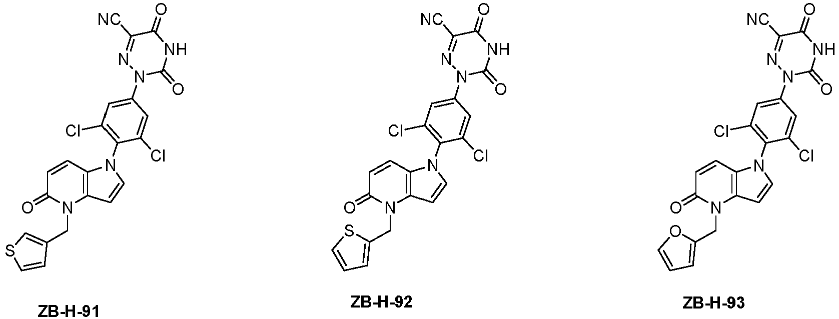

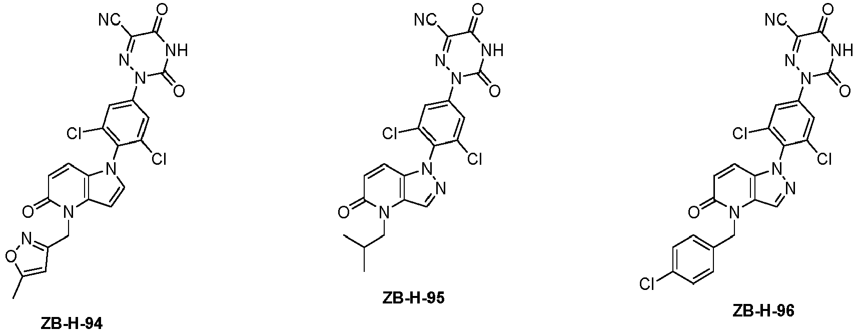

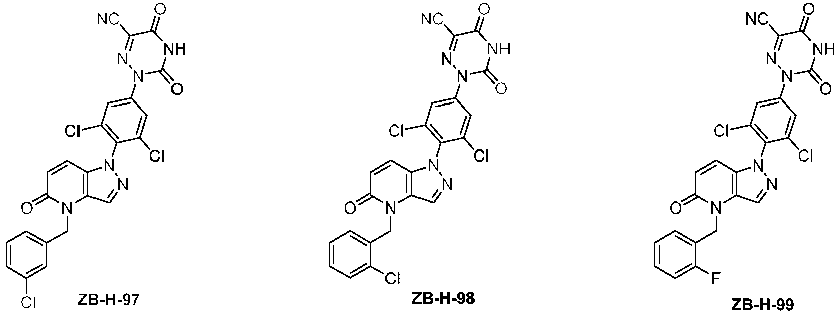

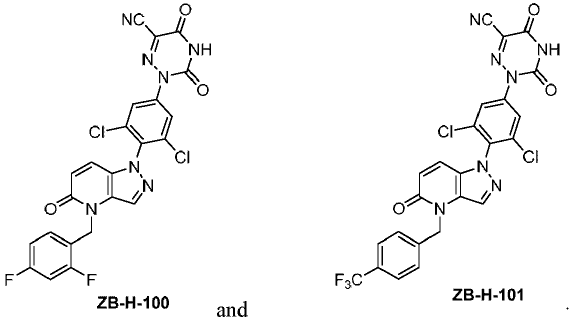

8. A pharmaceutical composition, comprising one or more selected from the group consisting of the compound and the pharmaceutically acceptable salt, stereoisomer, enantiomer, diastereoisomer, atropisomer, racemate, polymorph, solvate and isotopically labeled compound thereof according to any one of claims 1 to 7, and optionally a pharmaceutically acceptable excipient.

9. Use of the compound or the pharmaceutically acceptable salt, stereoisomer, enantiomer, diastereoisomer, atropisomer, racemate, polymorph, solvate or isotopically labeled compound thereof according to any one of claims 1 to 7 or the composition according to claim 8 in preparation of a medicine for treatment of a metabolic-related disease.

10. The use according to claim 9, wherein the metabolic-related disease is selected from the group consisting of obesity, hyperlipidemia, hypercholesterolemia, diabetes, non-alcoholic steatohepatitis, hepatic steatosis, atherosclerosis, thyroid function hypothyroidism and thyroid cancer, in particular, the metabolic-related disease is selected from the group consisting of: non-alcoholic steatohepatitis (NASH), hypothyroidism and thyroid cancer.
